# EUROPÄISCHE PATENTSCHRIFT

(11) **EP 1 572 707 B1**
(45) Veröffentlichungstag und Bekanntmachung des Hinweises auf die Patenterteilung: **29.03.2006**
(21) Anmeldenummer: 03795853.5
(22) Anmeldetag: 28.11.2003
(51) Int. Cl.: C07H 15/203, A61K 31/70, A61P 3/10

(54) **NEUE AROMATISCHE FLUORGLYCOSIDDERIVATE, DIESE VERBINDUNGEN ENTHALTENDE ARZNEIMITTEL UND DEREN VERWENDUNG**
NOVEL AROMATIC FLUOROGLYCOSIDE DERIVATIVES, PHARMACEUTICAL PRODUCTS CONTAINING SAID COMPOUNDS AND THE USE THEREOF
NOUVEAUX DERIVES DE FLUORGLYCOSIDE AROMATIQUES, PRODUITS PHARMACEUTIQUES CONTENANT CES COMPOSES ET LEUR UTILISATION

(30) Priorität: 12.12.2002 DE 10258007
(43) Veröffentlichungstag der Anmeldung: 14.09.2005
(73) Patentinhaber: Sanofi-Aventis Deutschland GmbH, 65929 Frankfurt am Main (DE)
(72) Erfinder: FRICK, Wendelin, 65510 Hünstetten-Beuerbach (DE); GLOMBIK, Heiner, 65719 Hofheim (DE); KRAMER, Werner, 55130 Mainz-Laubenheim (DE); HEUER, Hubert, 55270 Schwabenheim (DE); BRUMMERHOP, Harm, 60316 Frankfurt (DE); PLETTENBURG, Oliver, 65795 Hattersheim (DE)
(86) Internationale Anmeldenummer: PCT/EP2003/013454
(87) Internationale Veröffentlichungsnummer: WO 2004/052902

(56) Entgegenhaltungen:
- EP-A- 0 850 948
- EP-A- 1 213 296
- WO-A-01/27128
- WITHERS, S.G. ET AL.: "2-Deoxy-2-fluoroglucosides: a novel class of mechanism-based glucosidase inhibitors" J. AM. CHEM. SOC., Bd. 109, 1987, Seiten 7530-1, XP002275265

## Beschreibung

Die Erfindung betrifft substituierte aromatische Fluorglycosidderivate, deren physiologisch verträgliche Salze sowie physiologisch funktionelle Derivate.

In der Literatur sind bereits mehrere Substanzklassen mit SGLT-Wirkung bekannt. All diesen Strukturen diente als Leitbild der Naturstoff Phlorizin. Von diesem wurden folgende Klassen abgeleitet, die in den nachfolgenden Schutzrechten beschrieben sind:
- Propiophenonglycoside von Tanabe (WO 0280936, WO 0280935, JP 2000080041 und EP 850948)
- 2-(Glucopyranoslyoxy)-benzylbenzole von Kissei (WO 0244192, WO 0228872 und WO 0168660)
- Glucopyranosyloxy-pyrazole von Kissei und Ajinomoto (WO 0268440, WO 0268439, WO 0236602 und WO 0116147)
- O-Glycosidbenzamide von Bristol-Myers Squibb (WO 0174835 und WO 0174834)
- und C-Arylglycosidewon Bristol-Myers Squibb (WO 0127128 und US 2002137903).

Alle bekannten Strukturen enthalten als sehr wichtiges Strukturelement die Glucose.

Ferner sind aus US 2002/132807 Diarylsulfid-Verbindungen zur Behandlung von Entzündungs- und Immun-Erkrankungen bekannt. In EP 0 953 357 A1 werden allgemein Glycosid-Verbindungen als renale Drug-Carrier und in WO 95/23780 4-Hydroxy-phenoxy-hetero-cycloalkyl-Verbindungen als Hautaufheller beschrieben.

Der Erfindung lag die Aufgabe zugrunde, neue Verbindungen zur Verfügung zu stellen, mit denen eine Prävention und Behandlung von Diabetes Typ 1 und Typ 2 möglich ist. Wir haben nun überraschenderweise gefunden, dass aromatische Fluorglycosidderivate die Wirkung auf SGLT steigern. Diese Verbindungen eignen sich daher besonders zur Prävention und Behandlung von Diabetes Typ 1 und Typ 2.

Die Erfindung betrifft daher Verbindungen der Formel l, 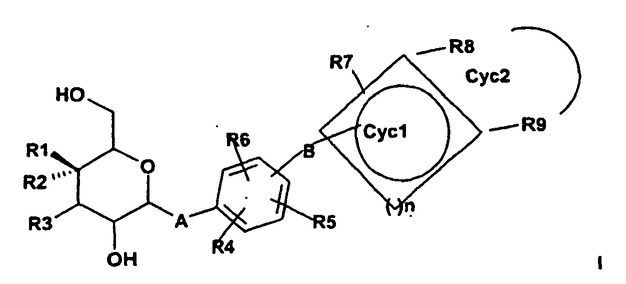 worin bedeuten
- R1, R2: OH, F oder H oder R1 und R2 = F, wobei die drei Kombinationen R1=F, R2=OH und R1=OH, R2=F und R1,R2=OH ausgenommen sind;
- R3: OH oder F, wobei mindestens einer der Reste R1, R2, R3 F sein muss;
- A: O, NH, CH₂, S oder eine Bindung;
- R4,R5,R6: Wasserstoff, F, Cl, Br, J, OH, NO₂, CN, COOH, CO(C₁-C₆)-Alkyl, COO(C₁-C₆)-Alkyl, CONH₂, CONH(C₁-C₆)-Alkyl, CON[(C₁-C₆)-Alkyl]2, (C₁₋C₆)-Alkyl, (C₂-C₆)-Alkenyl, (C₂-C₆)-Alkinyl, (C₁-C₆)-Alkoxy, HO-(C₁-C₆)-Alkyl, (C₁-C₆)-Alkyl-O-(C₁-C₆)-alkyl, Phenyl, Benzyl, wobei in den Alkyl-, Alkoxy-, Alkenyl- und Alkinylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂, S-(C₁-C₆)-Alkyl, S-(CH₂)ₒ-Phenyl, SO-(C₁-C₆)-Alkyl, SO-(CH₂)ₒ-Phenyl, SO₂-(C₁-C₆)-Alkyl, SO₂-(CH₂)ₒ-Phenyl, wobei o = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₒ-Phenyl, wobei o = 0 - 6 sein kann, wobei der Phenylring ein bis 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂ CH₃, COOH, COO-(C₁-C₆)-Alkyl, CONH₂;
- B: (C₀-C₁₅)-Alkandiyl, wobei ein oder mehrere C-Atome des Alkandiyl-Rests unabhängig voneinander durch -O-, -(C=O)-. -CH=CH-, -C≡C-, -S-, -CH(OH)-, -CHF-, -CF₂-, -(S=O)-, -(SO₂)-, -N((C₁-C₆)-Alkyl)-, -N((C₁-C₆)-Alkyl-Phenyl)- oder -NH- ersetzt sein können;
- n: eine Zahl von 0 bis 4;
- Cyc1: ein 3 bis 7 gliedriger gesättigter, teilweise gesättigter oder ungesättigter Ring, wobei 1 C-Atom durch O, N oder S ersetzt sein kann;
- R7, R8, R9: Wasserstoff, F, Cl, Br, J, OH, CF₃, NO₂, CN, COOH, COO(C₁-C₆)-Alkyl, CO(C₁-C₄)-Alkyl, CONH₂, CONH(C₁-C₆)-Alkyl, CON[(C₁-C₆)-Alkyl]₂,(C₁₋C₆)-Alkyl, (C₂-C₆)-Alkenyl, (C₂-C₆)-Alkinyl, (C₁-C₈)-Alkoxy, (C₁-C₆)-Alkyl-OH, (C₁-C₆)-Alkyl-O-(C₁-C₆)-alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂ , S-(C₁-C₆)-Alkyl, S-(CH₂)ₒ-Phenyl, SO-(C₁-C₆)-Alkyl, SO-(CH₂)ₒ-Phenyl, SO₂-(C₁-C₆)-Alkyl, SO₂-(CH₂)ₒ-Phenyl, wobei o = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₒ-Phenyl, wobei o = 0 - 6 sein kann, wobei der Phenylring ein bis 3-fach substituiert sein kann mit F, Cl, Br, J, OH, CF₃, NO₂, CN, OCF₃, (C₁- C₈)-Alkoxy, (C₁-C₆)-Alkyl, NH₂, NH(C₁-C₆)-Alkyl, N((C₁-C₆)-Alkyl)₂, SO₂₋CH₃, COOH, COO-(C₁-C₆)-Alkyl, CONH₂;
oder
- R8 und R9: gemeinsam mit den sie tragenden C-Atomen ein 5 bis 7 gliedrigen, gesättigter, teilweise oder vollständig ungesättigter Ring Cyc2, wobei 1 oder 2 C-Atom(e) des Ringes auch durch N, O oder S ersetzt sein können und Cyc2 gegebenenfalls durch (C₁-C₆)-Alkyl, (C₂-C₅)-Alkenyl, (C₂-C₅)-Alkinyl, wobei jeweils eine CH₂-Gruppe durch O ersetzt sein kann, oder durch H, F, Cl, OH, CF₃, NO₂, CN, COO(C₁-C₄)-Alkyl, CONH₂, CONH(C₁₋C₄)-Alkyl, OCF₃ substituiert sein kann;
sowie deren pharmazeutisch verträglichen Salze.

Die Verknüpfungspunkte von R4, R5, R6 und B an den Phenylring sind frei wählbar. Alle resultierenden Verbindungen der Formel I gehören zur vorliegenden Erfindung. Bevorzugt sind Verbindungen der Formel I, in denen der B-Substituent am Phenylring in ortho-Position. (Nachbarstellung) zu dem A-Substituenten angeordnet ist.

Bevorzugt sind Verbindungen der Fomnel I, worin bedeuten
- R1, R2: OH, F oder H oder R1 und R2 = F, wobei einer der Reste R1 oder R2 F sein muss und die Kombinationen R1=F, R2=OH und R1=OH, R2=F und R1,R2=OH ausgenommen sind;
- R3: OH;
- A: O oder NH;
- R4,R5,R6: Wasserstoff, F, Cl, Br, J, OH, CF₃, NO₂, CN, COOH, CO(C₁-C₆)-Alkyl, COO(C₁-C₆)-Alkyl, CONH₂, CONH(C₁-C₆)-Alkyl, CON[(C₁-C₆)-Alkyl]₂, (C₁₋C₆)-Alkyl, (C₂-C₆)-Alkenyl, (C₂-C₆)-Alkinyl, (C₁-C₆)-Alkoxy, HO-(C₁-C₆)-Alkyl, (C₁-C₆)-Alkyl-O-(C₁-C₆)-alkyl, Phenyl, Benzyl, SO-(C₁-C₆)-Alkyl, wobei in den Alkyl-, Alkoxy-, Alkenyl- und Alkinylresten ein, mehrere, oder alle Wasserstoff(e) durch Fluor ersetzt sein können;
- B: (C₀-C₁₅)-Alkandiyl, wobei ein oder mehrere C-Atom(e) des Alkandiyl-Rests unabhängig voneinander durch -O-, -(C=O)-, -CH=CH-, -C=C-, -S-, -CH(OH)-, -CHF-, -CF₂-, -(S=O)-, -(SO₂)-, -N((C₁-C₆)-Alkyl)-, -N((C₁-C₆)-Alkyl-Phenyl)- oder -NH- ersetzt sein können;
- n: eine Zahl 0 bis 4;
- Cyc1: ein 3 bis 7 gliedriger gesättigter, teilweise gesättigter oder ungesättigter Ring, wobei 1 C-Atom durch O, N oder S ersetzt sein kann;
- R7, R8, R9: Wasserstoff, F, Cl, Br, J, OH, CF₃, NO₂, CN, COOH, COO(C₁-C₆)-Alkyl, CO(C₁-C₄)-Alkyl, CONH₂, CONH(C₁-C₆)-Alkyl, CON[(C₁-C₆)-Alkyl]₂, (C₁₋C₆)-Alkyl, (C₂-C₆)-Alkenyl, (C₂-C₆)-Alkinyl, (C₁-C₈)-Alkoxy, (C₁-C₆)-Alkyl-OH, (C₁-C₆)-Alkyl-O-(C₁-C₆)-alkyl, SO-(C₁-C₆)-Alkyl, wobei in den Alkylresten ein, mehrere, oder alle Wasserstoff(e) durch Fluor ersetzt sein können;
oder
- R8 und R9: gemeinsam mit den sie tragenden C-Atomen ein 5 bis 7 gliedrigen, gesättigter, teilweise oder vollständig ungesättigter Ring Cyc2, wobei 1 oder 2 C-Atom(e) des Ringes auch durch N, O oder S ersetzt sein können und Cyc2 gegebenenfalls durch (C₁-C₆)-Alkyl, (C₂-C₅)-Alkenyl, (C₂-C₅)-Alkinyl, wobei jeweils eine CH₂-Gruppe durch O ersetzt sein kann, oder durch H, F, Cl, OH, CF₃, NO₂, CN, COO(C₁-C₄)-Alkyl, CONH₂, CONH(C₁₋C₄)-Alkyl, OCF₃ substituiert sein kann.

Bevorzugt sind ferner Verbindungen der Formel I, in denen die Zucker-Reste beta(β)-verknüpft sind und die Stereochemie in 2-,3- und 5-Position des Zuckerrestes D-gluco-konfiguriert ist.

Besonders bevorzugt sind Verbindungen der Formel I, worin
- R1, R2: OH, F oder H oder R1 und R2 = F, wobei einer der Reste R1 oder R2 F sein muss und die Kombinationen R1=F, R2=OH und R1=OH, R2=F und R1,R2=OH ausgenommen sind ;
- R3: OH;
- A: O;
- R4, R5, R6: Wasserstoff, OH, (C₁-C₆)-Alkyl, (C₁-C₄)-Alkoxy, HO-(C₁-C₄)-Alkyl, (C₁₋C₄)-Alkyl-O-(C₁-C₄)-alkyl, F, Cl, Br, J, CF₃, OCF₃ , OCH₂CF₃ (C₁-C₄)-Alkyl-CF₂-, Phenyl, Benzyl, (C₂-C₄)-Alkenyl, (C₂-C₄)-Alkinyl, COO(C₁-C₄)-Alkyl;
- B: (C₁-C₄)-Alkandiyl, wobei eine CH₂-Gruppe auch ersetzt sein kann durch -(C=O)-, -CH(OH)-, -CO-NH-, -CO-N(C₁-C₆)-Alkyl-, -CHF-, -CF₂-, -O-, -NH-;
- n: eine Zahl 2 oder 3;
- Cyc1: ungesättigter 5- oder 6-gliedriger Ring, wobei 1 C-Atom durch O, N oder S ersetzt sein kann;
- R7,R8,R9: Wasserstoff, (C₁-C₆)-Alkyl, (C₁-C₈)-Alkoxy, OCF₃, OCH₂CF₃ , OH , (C₁₋C₄)-Alkyl-OH, (C₁-C₄)-Alkyl-O-(C₁-C₄)-alkyl, F, Cl, Br oder
- R8 und R9: gemeinsam -CH=CH-O-, -CH₂-CH₂-O-,-CH=CH-S-, -CH=CH-CH=CH-, -O-(CH₂)ₚ-O-, mit p = 1 oder 2 und
- R7: Methyl, Ethyl, OMe, F, Cl, Br oder Wasserstoff bedeuten.

Ganz besonders bevorzugt sind auch Verbindungen der Formel I, worin
- R1: F und R2 H oder
- R1: H und R2 F;
- R1: F und R2 F
- R3: OH;
- A: O;
- R4, R5, R6: Wasserstoff, OH, (C₁-C₄)-Alkoxy, CF₃, (C₁-C₄)-Alkyl, F, Cl, Br, J,
- B: -CH₂-, -C₂H₄-, -C₃H₆, -CH(OH)-, -(C=O)-, -CO-NH-CH₂- oder -CO-CH₂₋CH₂-, -O-, -NH-;
- n: eine Zahl 2 oder 3;
- Cyc1: ungesättigter 6-gliedriger Ring, wobei 1 C-Atom durch N ersetzt sein kann oder ungesättigter 5-gliedriger Ring, wobei 1 C-Atom durch S ersetzt sein kann;
- R7,R8,R9: Wasserstoff, OH, (C₁-C₄)-Alkyl, (C₁-C₇)-Alkoxy, OCF₃, Halogen oder
- R8 und R9: gemeinsam -CH=CH-O-, -CH₂-CH₂-O-, -CH=CH-CH=CH- -O-(CH₂)p-O-, mit p = 1 oder 2, und
- R7: Methyl, Ethyl, Methoxy, F, Cl, Br, Wasserstoff bedeuten.

Ferner sind ganz besonders bevorzugt Verbindungen der Formel la 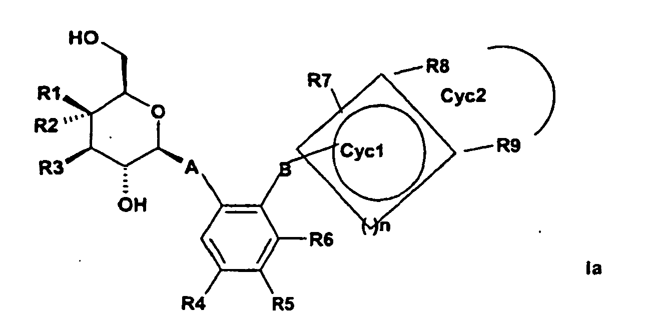 worin
- R1: F und R2 H oder
- R1: H und R2 F oder
- R1: F und R2 F;
- R3: OH;
- A: O;
- R4: Wasserstoff, (C₁-C₄)-Alkyl, (C₁-C₄)-Alkoxy oder OH;
- R5: Wasserstoff, F, Methoxy oder Ethoxy;
- R6: Wasserstoff oder OH;
- B: -CH₂-, -CO-NH-CH₂-; -O- oder -CO-CH₂-CH₂-;
- Cyc1: Phenyl oder Thiophen;
- R7,R8,R9: Wasserstoff, OH, Cl, OCF₃, (C₁-C₄)-Alkyl oder (C₁-C₄)-Alkoxy; oder
- R8 und R9: gemeinsam -CH=CH-O-, -CH=CH-CH=CH- oder -CH₂-CH₂-O- und
- R7: Wasserstoff bedeuten.

Von besonders bevorzugter Bedeutung sind außerdem Verbindungen der Formel Ib 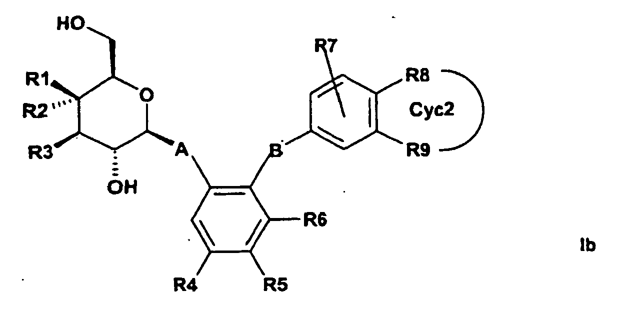 worin
- R1: F und R2 H oder
- R1: H und R2 F oder
- R1: F und R2 F;
- R3: OH;
- A: O;
- R4: Wasserstoff, Methyl, Methoxy oder OH;
- R5: Wasserstoff, F oder Methoxy;
- R6: Wasserstoff oder OH;
- B: -CH₂-, -CO-NH-CH₂-; -O- oder -CO-CH₂-CH₂-;
- Cyc1: Phenyl;
- R7: Wasserstoff;
- R8: Wasserstoff, OH, Ethyl, Cl, OCF₃ oder Methoxy;
- R9: Wasserstoff; oder
- R8 und R9: gemeinsam -CH=CH-O- oder -CH₂-CH₂-O- bedeuten.

Außerdem sind solche Verbindungen der Formel I ganz besonders bevorzugt, in den R1 = H und R2 = F ist.

Die Erfindung bezieht sich auf Verbindungen der Formel l, in Form ihrer Racemate, racemischen Mischungen und reinen Enantiomere sowie auf ihre Diastereomere und Mischungen davon.

Die Alkylreste in den Substituenten R4, R5, R6, R7, R8 und R9 können sowohl geradkettig wie verzweigt sein. Unter Halogen werden F, Cl, Br, J, bevorzugt F und Cl verstanden.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure.

Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel.I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht. Bevorzugt sind Carbonate an der 6-Position des Zuckers (siehe WO 0280936 und WO 0244192), besonders bevorzugt Methyl-, und Ethylcarbonat.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel l" auf Verbindung(en) der Formel l wie vorstehend beschrieben, sowie ihre Salze, Solvate und physiologisch funktionellen Derivate wie hierin beschrieben.

Die Verbindung(en) der Formel (I) können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel l enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35 %, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Gegenstand der Erfindung sind weiterhin Verfahren zu Herstellung der Verbindungen der allgemeinen Formel I, die entsprechend den folgenden Reaktionsschemata der Verfahren A bis F erhalten werden können:

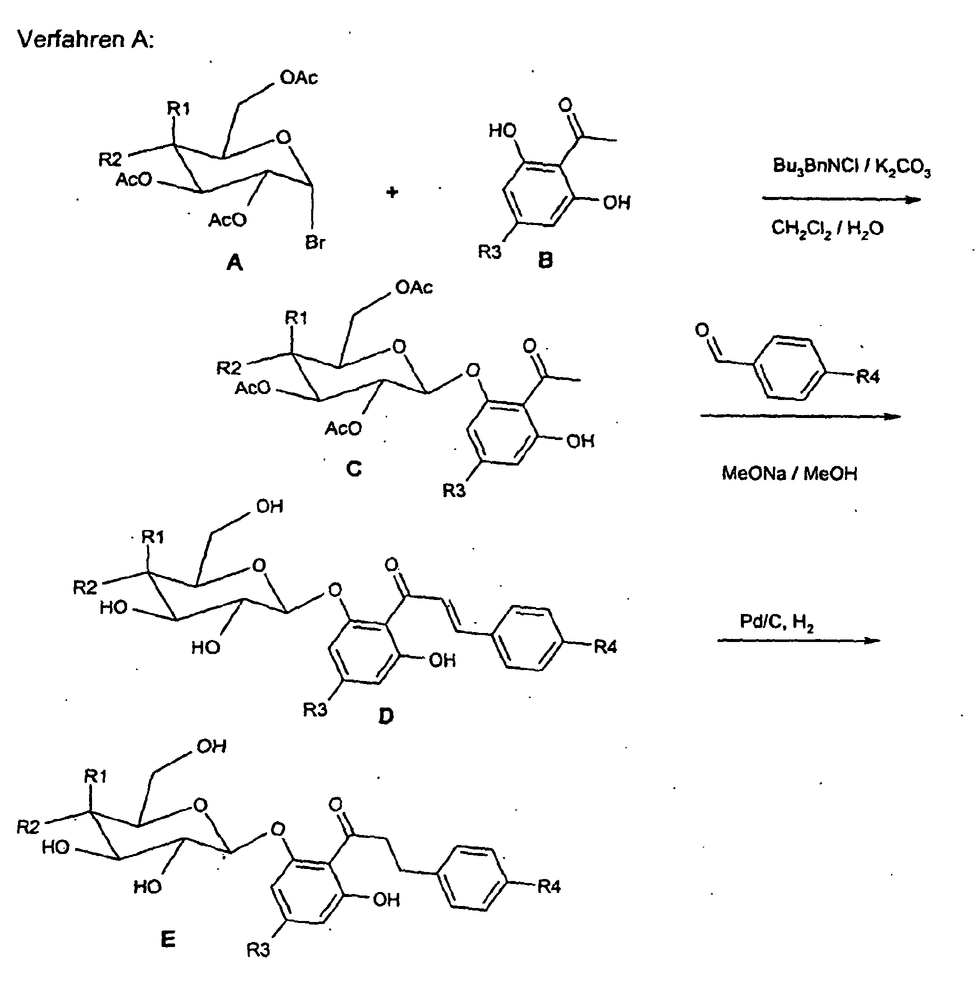

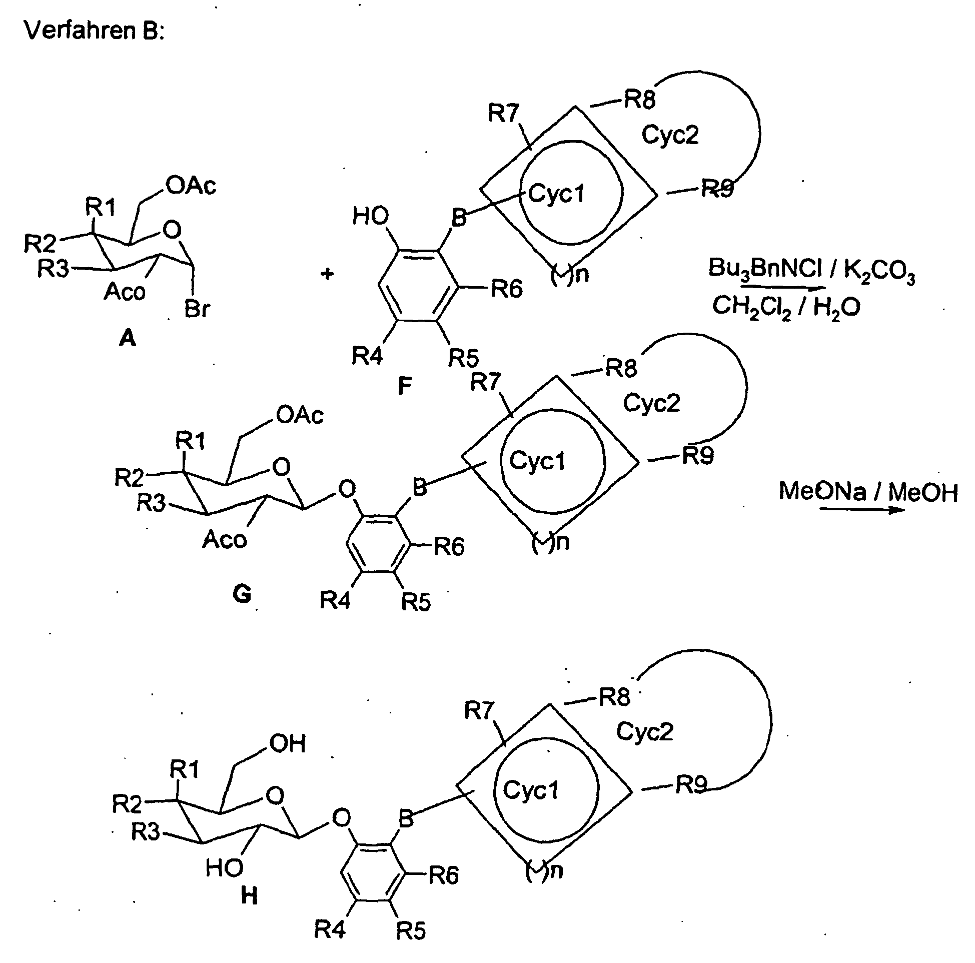

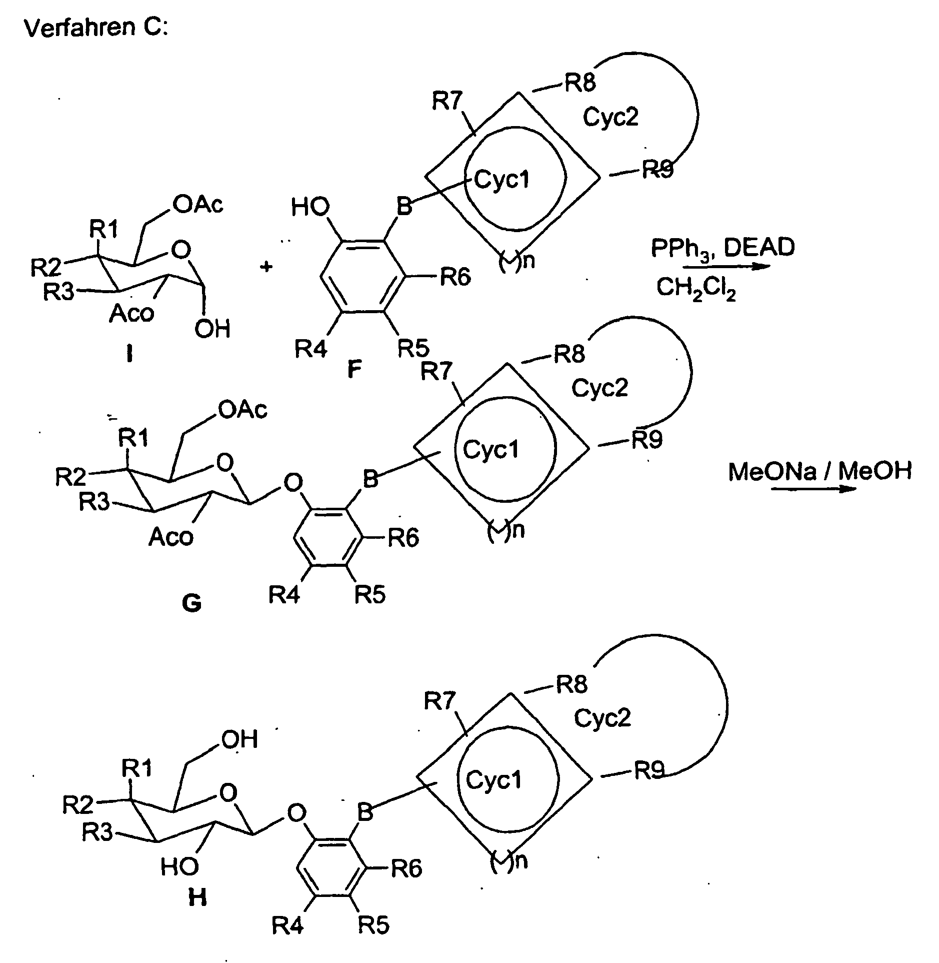

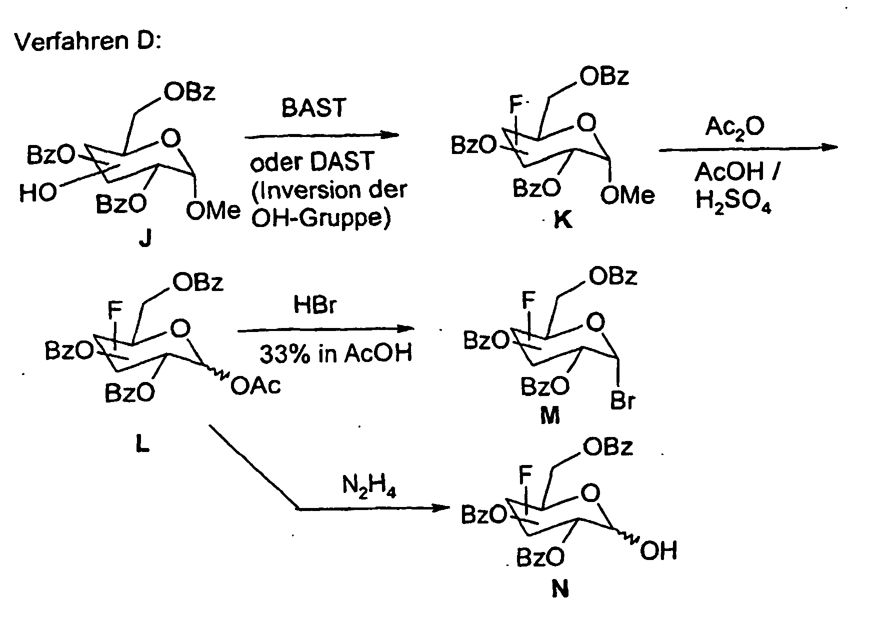

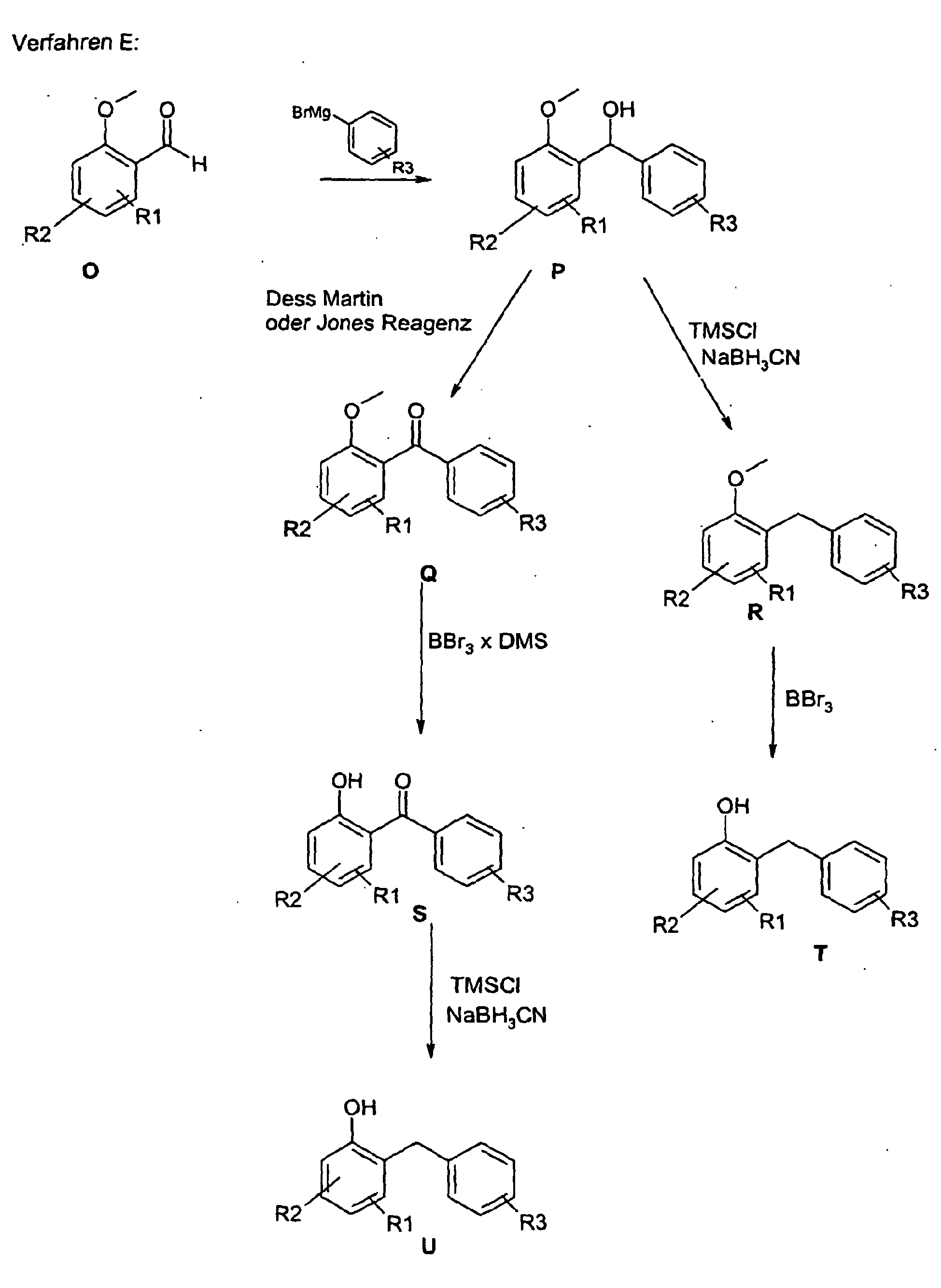

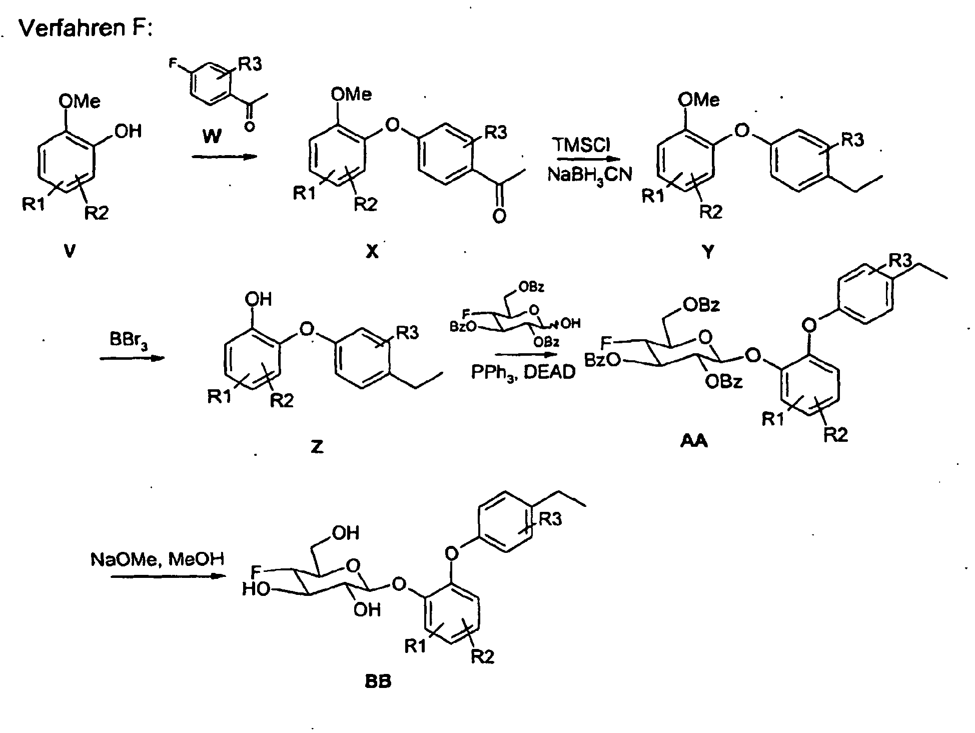

Die dargestellten Schemata der Verfahren A-F sprechen für sich selbst und sind für den Fachmann so ausführbar. Nähere Einzelheiten sind dennoch im Experimentellen Teil angegeben. Gemäß den Verfahren A-F wurden die Verbindungen der Beispiele 1 bis 24 erhalten. Andere Verbindungen der Formel I können entsprechend oder nach bekannten Verfahren erhalten werden.

Die Verbindung(en) der Formel I können auch in Kombination mit weiteren Wirkstoffen verabreicht werden.

Als weitere Wirkstoffe für die Kombinationspräparate sind geeignet:

Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.
Antidiabetika umfassen Insulin und Insulinderivate, wie z.B. Lantus® (siehe www.lantus.com) oder HMR 1964, schnell wirkende Insuline (siehe US 6.221,633), GLP-1-Derivate wie z.B. diejenigen die in WO 98/08871 von Novo Nordisk A/S offenbart wurden, sowie oral wirksame hypoglykämische Wirkstoffe.
Die oral wirksamen hypoglykämischen Wirkstoffe umfassen vorzugsweise Sulphonylfharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glycogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Katiumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, AVE0897 oder wie in WO 00/64888, WO 00/64876, WO 03/20269 beschrieben verabreicht.

Bei einer Ausführungsform der Erfindung werden die Verbindungen der Formel I in Kombination mit einem Fibrat, wie z.B. Fenofibrat. Clofibrat, Bezafibrat, verabreicht.

Bei einer Ausführungsform der Erfindung werden die Verbindungen der Formel I in Kombination mit einem MTP-Inhibitor, wie z.B. lmplitapide , BMS-201038, R-103757, verabreicht.

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel l in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. Cl-1027 oder Nicotinsäure, verabreicht.

Bei einer Ausführungsform der Erfindung werden die Verbindungen der Formel 1 in Kombination mit einem Lipase Inhibitor, wie z.B. Orlistat, verabreicht.

Bei einer Ausführungsform der Erfindung werden die Verbindungen der Formel I in Kombination mit Insulin verabreicht.
Bei einer Ausführungsform werden die Verbindungen der Formel I in Kombination mit einem Sulphonylharnstoff, wie z.B. Tolbutamid, Glibenclamid, Glipizid oder Glimepirid verabreicht.

Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.
Bei einer Ausführungsform werden die Verbindungen der Formel I in Kombination mit einem α-Glukosidase-Inhibitor, wie z.B. Miglitol oder Acarbose, verabreicht.
Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid. Bei einer Ausführungsform werden die Verbindungen der Formel I in Kombination mit mehr als einer der vorstehend genannten Verbindungen, z.B. in Kombination mit einem Sulphonylhamstoff und Metformin, einem Sulphonylhamstoff und Acarbose, Repaglinid und Metformin, Insulin und einem Sulphonylhamstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4.3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)propan-1- on Oxalsäuresalz (WO 00 / 63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2-carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),
DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001). 2(10), 1615-1622.

Bei einer Ausführungsform ist der weitere Wirkstoff Dexamphatamin oder Amphetamin.
Bei einer Ausführungsform ist der weitere Wirkstoff Fenfluramin oder Dexfenfluramin. Bei noch einer Ausführungsform ist der weitere Wirkstoff Sibutramin.
Bei einer Ausführungsform ist der weitere Wirkstoff Orlistat.

Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax® (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GinbH, Industriepark Höchst, 65926 Frankfurt / Main)) verabreicht. Die Kombination mit Caromax® kann in einer Zubereitung erfolgen, oder durch getrennte Gabe von Verbindungen der Formel I und Caromax®. Caromax® kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

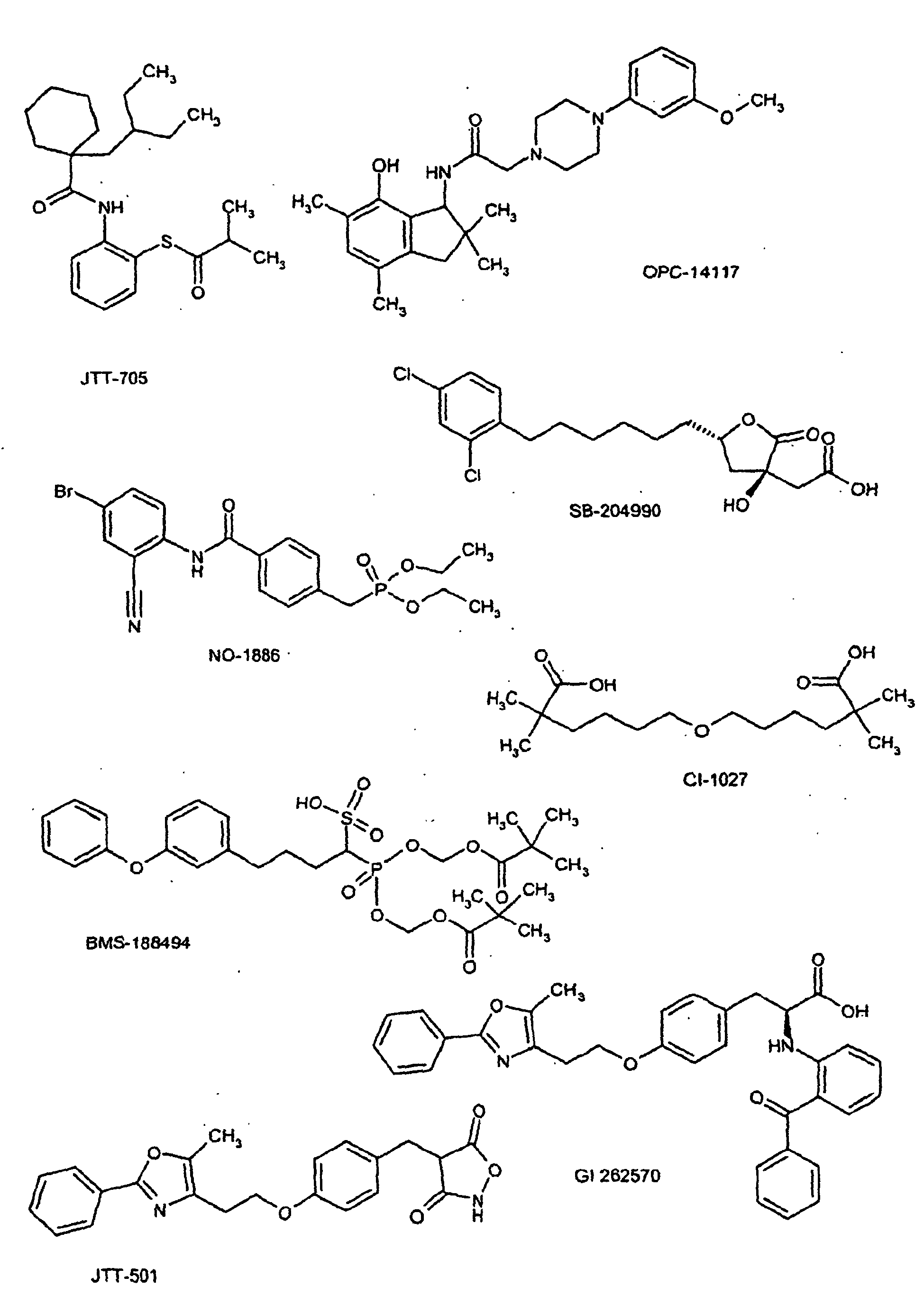

Die nachfolgend aufgeführten Beispiele dienen zur Erläuterung der Erfindung, ohne diese jedoch einzuschränken

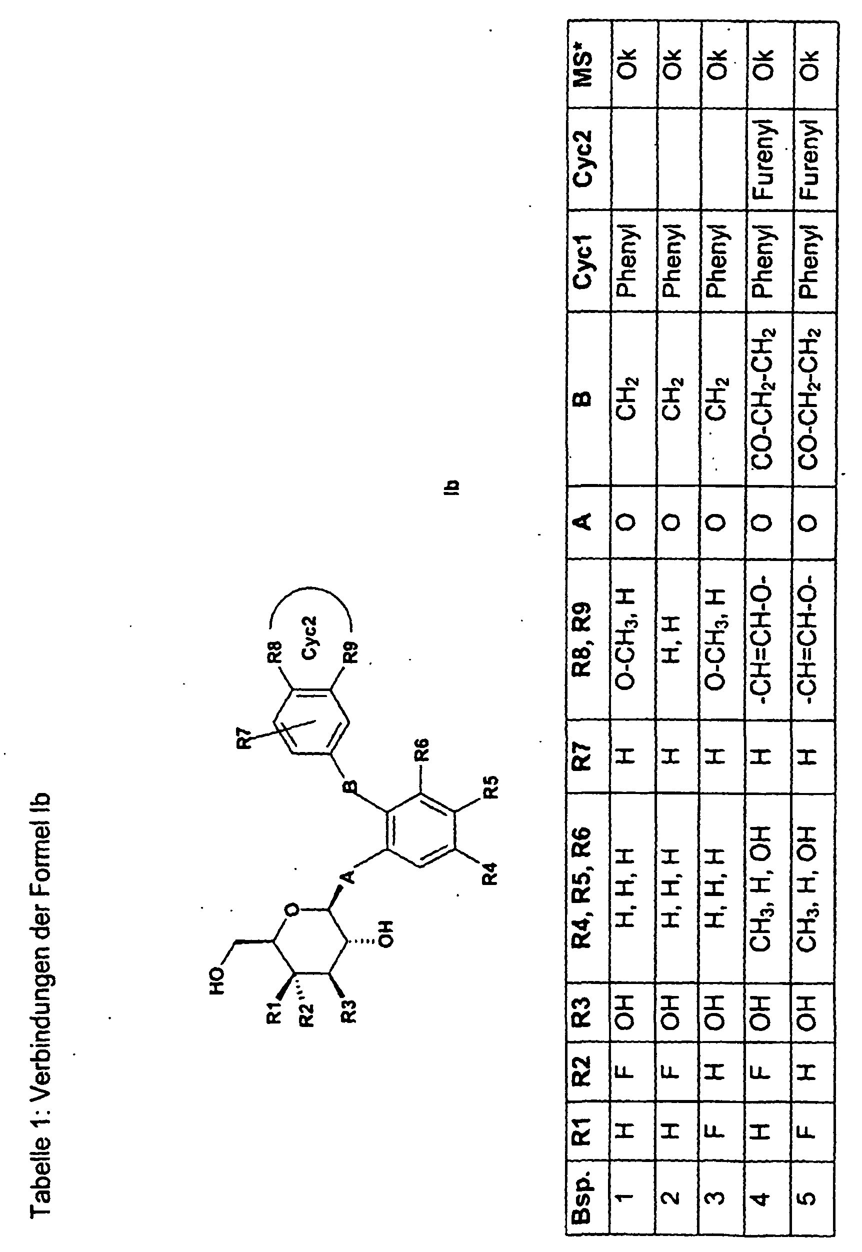

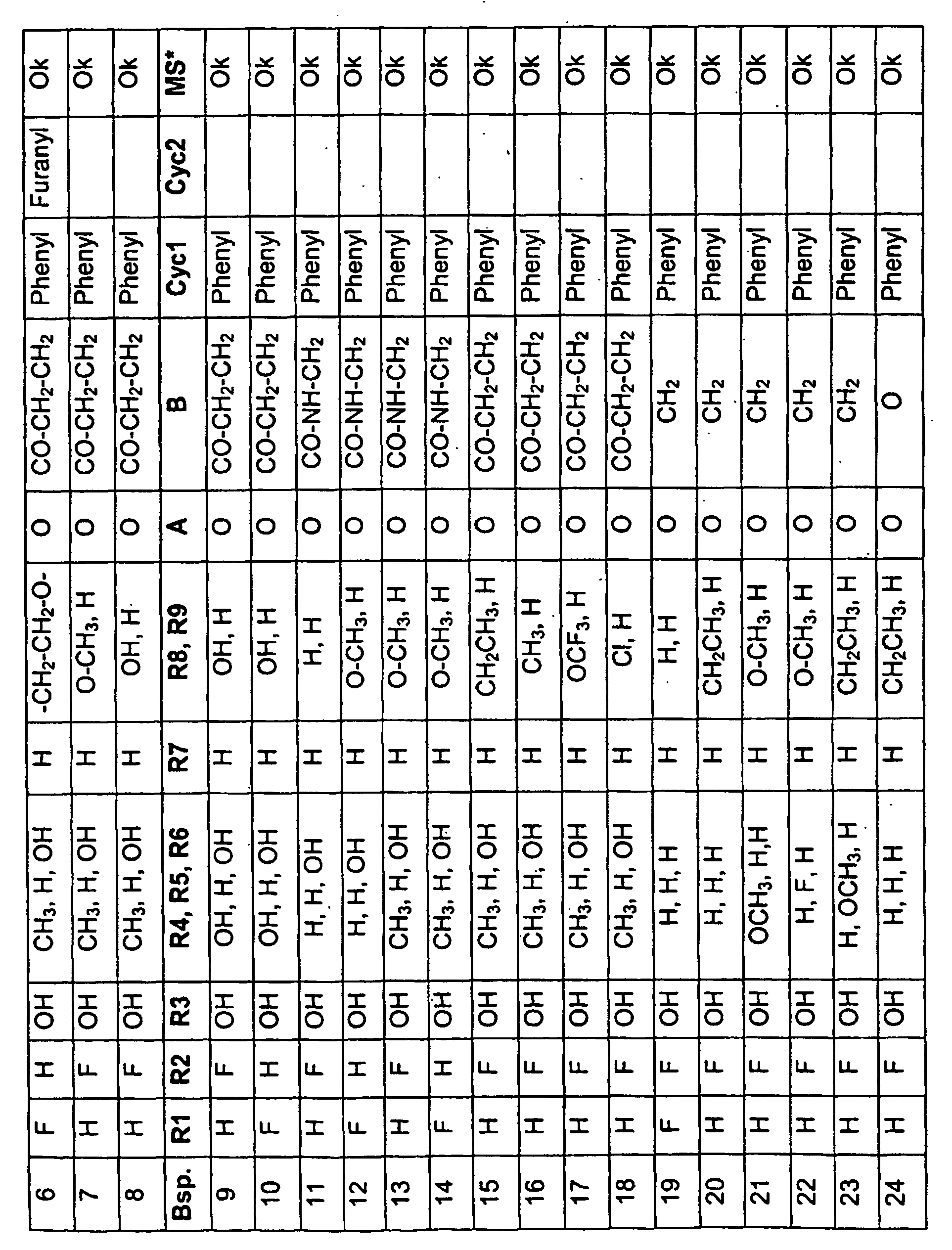

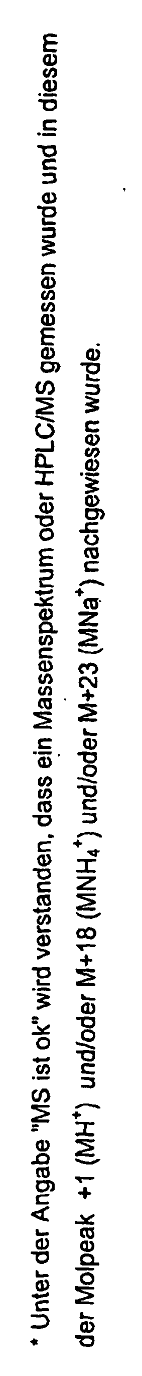

Die Verbindungen der Formel I zeichnen sich durch günstige Wirkungen auf den Glucosestoffwechsel aus, sie senken insbesondere den Blutzuckerspiegel und sind zur Behandlung von Typ 1 und Typ 2 Diabetes geeignet. Die Verbindungen können daher allein oder in Kombination mit weiteren Blutzucker-senkenden Wirkstoffen (Antidiabetika) eingesetzt werden.
Die Verbindungen der Formel I eignen sich weiterhin zur Prävention und Behandlung von Diabetischen Spätschäden, wie z.B. Nephropatie, Retinopathie, Neuropathie sowie Syndrom X, Obesitas, Herzinfarkt, Myocardialem Infarkt, peripheren arteriellen Verschlusskrankheiten, Thrombosen, Arteriosklerose, Entzündungen, lmmunkrankheiten, Autoimmunkrankheiten, wie z.B. AIDS, Asthma, Osteoporose, Krebs, Psoriasis, Alzheimer, Schizophrenie und Infektionskrankheiten, bevorzugt ist die Behandlung von Typ 1 und Typ 2 Diabetes sowie zur Prävention und Behandlung von Diabetischen Spätschäden, Syndrom X und Obesitas.

Die Wirksamkeit der Verbindungen wurde wie folgt getestet:

### Präparation von Bürstensaummembran-Vesikeln aus dem Dünndarm von Kaninchen, Ratte und Schwein

Die Präparation von Bürstensaummembran-Vesikeln aus den Darmzellen des Dünndarms erfolgte mit der sog. Mg²⁺-Präzipitationsmethode. Die Mucosa aus dem Dünndarm wurde abgeschabt und in 60 ml eiskaltem Tris/HCl-Puffer (ph 7,1) 300 mM Mannit, 5 mM EGTA suspendiert. Nach dem Verdünnen auf 300 ml mit eiskaltem destilliertem Wasser wurde mit einem Ultraturrax (18-Stab, IKA Werk Staufen, BRD) 2 x 1 Minute bei 75 % max. Leistung unter Eiskühlung homogenisiert. Nach Zugabe von 3 ml 1M MgCl₂-Lösung (Endkonzentration 10 mM) lässt man exakt 15 Minuten bei 0° C stehen: Durch die Zugabe von Mg²⁺ aggregieren die Zell-membranen und präzipitieren, mit Ausnahme der Bürstensaummembranen. Nach einer 15-minütigen Zentrifugation bei 3 000 x g (5 000 rpm, SS-34-Rotor) wird der Niederschlag verworfen und der Überstand, der die Bürstensaummembranen enthält, 30 Minuten bei 26 700 x g (15 000 rpm, SS-34-Rotor) zentrifugiert. Der Überstand wird verworfen, der Niederschlag in 60 ml 12 mM Tris/HCl-Puffer (ph 7,1) / 60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M MgCl₂-Lösung und 15-minütiger Inkubation bei 0°C wird erneut 15 Minuten bei 3 000 x g zentrifugiert. Der Überstand wird anschließend nochmals 30 Minuten bei 46 000 x g (20 000 rpm, SS-34-Rotor) zentrifugiert. Der Niederschlag wird in 30 ml 20 mM Tris/Hepes-Puffer (pH 7,4) /280 mM Mannit aufgenommen und durch 20 Hübe in einem Potter Elveihem Homogenisator bei 1 000 rpm homogen resuspendiert. Nach 30-minütiger Zentrifugation bei 48 000 xg (20 000 rpm, SS-34-Rotor) wurde der Niederschlag in 0,5 bis 2 ml Tris/Hepes-Puffer (pH 7,4) / 280 mM Mannit (Endkonzentration 20 mg/ml) aufgenommen und mit Hilfe einer Tuberkulinspritze mit einer 27 Gauge-Nadel resuspendiert.
Die Vesikel wurden entweder unmittelbar nach der Präparation für Markierungs- oder Transportuntersuchungen verwendet oder wurden bei - 196°C in 4 mg Portionen in flüssigem Stickstoff aufbewahrt.
Für die Präparation von Bürstensaummembranvesikeln aus Rattendünndarm wurden 6 bis 10 männliche Wistar-Ratten (Tierzucht Kastengrund, Aventis Pharma), durch zervikale Dislokation getötet, die Dünndärme entnommen und mit kalter isotonischer Kochsalzlösung gespült. Die Därme wurden aufgeschnitten und die Mucosa abgeschabt. Die Aufarbeitung zur Isolierung von Bürstensaummembranen erfolgte wie oben beschrieben. Zur Abtrennung von Cytoskelettanteilen wurden die Bürstensaummembranvesikel aus Rattendünndarm mit KSCN als chaotropem Ion behandelt.

Für die Präparation von Bürstensaummembranen aus Kaninchendünndarm wurden Kaninchen durch intravenöse Injektion von 0,5 ml einer wässrigen Lösung von 2,5 mg Tetracain-HCl, 100 mg m-Butramid und 25 mg Mebezoniumjodid getötet. Die Dünndärme wurden entnommen, mit eiskalter physiologischer Kochsalzlösung gespült und in Kunststoffbeutel unter Stickstoff bei - 80 °C eingefroren und 4 bis 12 Wochen gelagert. Zur Präparation der Membranvesikel wurden die eingefrorenen Därme bei 30°C im Wasserbad aufgetaut und anschließend die Mucosa abgeschabt. Die Aufarbeitung zu Membranvesikeln erfolgte wie oben beschrieben.

Zur Präparation von Bürstennsaummembranvesikein aus Schweinedarm wurden Jejunumsegmente eines frisch geschlachteten Schweines mit eiskalter isotonischer Kochsalzlösung gespült und in Plastikbeuteln unter Stickstoff bei - 80°C eingefroren. Die Präparation der Membranvesikel erfolgte wie oben beschrieben.

### Präparation von Bürstensaummembranvesikeln aus dem Nierenkortex der Rattenniere

Die Präparation der Bürstensaummembranvesikel aus dem Kortex der Rattenniere erfolgte nach der Methode von Biber et al. Die Nieren von 6 bis 8 Ratten (200 bis 250 g) wurden entnommen und von jeder Niere wurde der Kortex als ca. 1 mm starke Schicht abgetragen. Die Nieren wurden in 30 ml eiskaltem 12 mM Tris/HC1-Puffer (pH 7,4) / 300mM Mannit aufgenommen und unter Eiskühlung 4 x 30 Sekunden mit einem Ultraturraxstab (Stufe 180 V) homogenisiert. Nach der Zugabe von 42 ml eiskaltem destilliertem Wasser wurden 850 µl einer 1 M MgCl₂-Lösung zugegeben. Nach 15-minütiger Inkubation bei 0°C wurde 15 Minuten bei 4 500 rpm (Sorvall SS-34-Rotor) zentrifugiert. Der Niederschlag wurde verworfen, der Überstand 30 Minuten bei 16 000 rpm zentrifugiert. Nach Resuspendierung des Niederschlags in 60 ml 6 mM Tris/HCl-Puffer (pH 7,4) /150 mM Mannit / 2,5 mM EGTA durch 10 Hübe in einem Potter-Elvejhem Homogenisator (900 rpm) wurde nach Zugabe von 720 µl 1 mM MgCl₂-Lösung 15 Minuten bei 0°C inkubiert. Nach 15-minütiger Zentrifugation bei 4 500 rpm (SS-34-Rotor) wurde der resultierende Überstand 30 Minuten bei 16000 rpm zentrifugiert. Der Überstand wurde durch 10 Hübe in 60 ml 20 mM Tris/Hepes-Puffer (pH 7,4) /280 mM Mannit homogenisiert und die entstehende Suspension anschließend 30 Minuten bei 20 000 rpm zentrifugiert. Der Niederschlag wurde mit Hilfe einer Tuberkulinspritze mit einer 27 Gauge-Nadel in 20 mM Tris/HCl-Puffer (pH 7,4)/280 mM Mannit resuspendiert und auf eine Proteinkonzentration von 20 mg/ml eingestellt.

### Messung der Glukoseaufnahme durch Bürstensaummembranvesikel

Die Aufnahme von [¹⁴C]-markierter Glukose in Bürstensaummembranvesikel wurde mittels der Membranfiltrationsmethode gemessen. 10 µl der Bürstensaummembranvesikelsuspension in 10 mM Tris/Hepes-Puffer (pH 7.4)/300 mM Mannitol wurden bei 30°C zu 90 µl einer Lösung von 10 *µ*M [¹⁴C]D-Glukose und den entsprechenden Konzentrationen der betreffenden Hemmstoffe (5-200 µM) in 10 mM Tris/Hepes-Puffer (pH 7.4)/ 100 mM NaCl/100 mM Mannitol gegeben.
Nach 15 sec. Inkubation wurde der Transportprozess durch Zugabe von 1 ml eiskalter Stopplösung (10 mM Tris/Hepes-Puffer (pH 7.4)/150 mM KCI) angehalten und die Vesikelsuspension wurde sofort bei einem Vakuum von 25 bis 35 mbar über ein Membranfilter aus Cellulosenitrat (0,45 µm, 25 mm Durchmesser, Schleicher & Schüll) abgesaugt. Der Filter wurde mit 5 ml eiskalter Stopplösung nachgewaschen.
Jeder Messpunkt wurde als Doppel- oder Dreifachbestimmung ausgeführt.
Zur Messung der Aufnahme radioaktiv markierter Substrate wurde der Membranfilter in 4 ml eines entsprechenden Szintillators (Quickszint 361, Zinsser Analytik GmbH, Frankfurt am Main) aufgelöst und die Radioaktivität durch Flüssigkeisszintillationsmessung bestimmt. Die gemessenen Werte wurden nach Eichung des Gerätes mit Hilfe von Standardproben und nach Korrektur evtl. vorhandener Chemilumiszenz als dpm (Desintegrations per minute) erhalten.

Der Aktivitätsvergleich der Wirkstoffe wird anhand von IC₅₀ Daten durchgeführt, die im Transport-Assay an Dünndarm- Bürstensaummembranvesikeln des Kaninchens für ausgewählte Substanzen erhalten wurden. (Die Absolutwerte können Spezies- und Versuchs-abhängig sein)

| Beispiel Nr. | IC50 [µM] |
|---|---|
| Phlorizin | 16 |
| 1 | 0.5 |
| 2 | 0.7 |
| 4 | 1.5 |
| 5 | 0.4 |
| 7 | 0.9 |

Nachfolgend wird die Herstellung verschiedener Beispiele detailliert beschrieben, die übrigen Verbindungen der Formel l wurden analog erhalten:

### Experimenteller Teil:

### Reaktionsschema: Synthese von α-Bromglycosiden

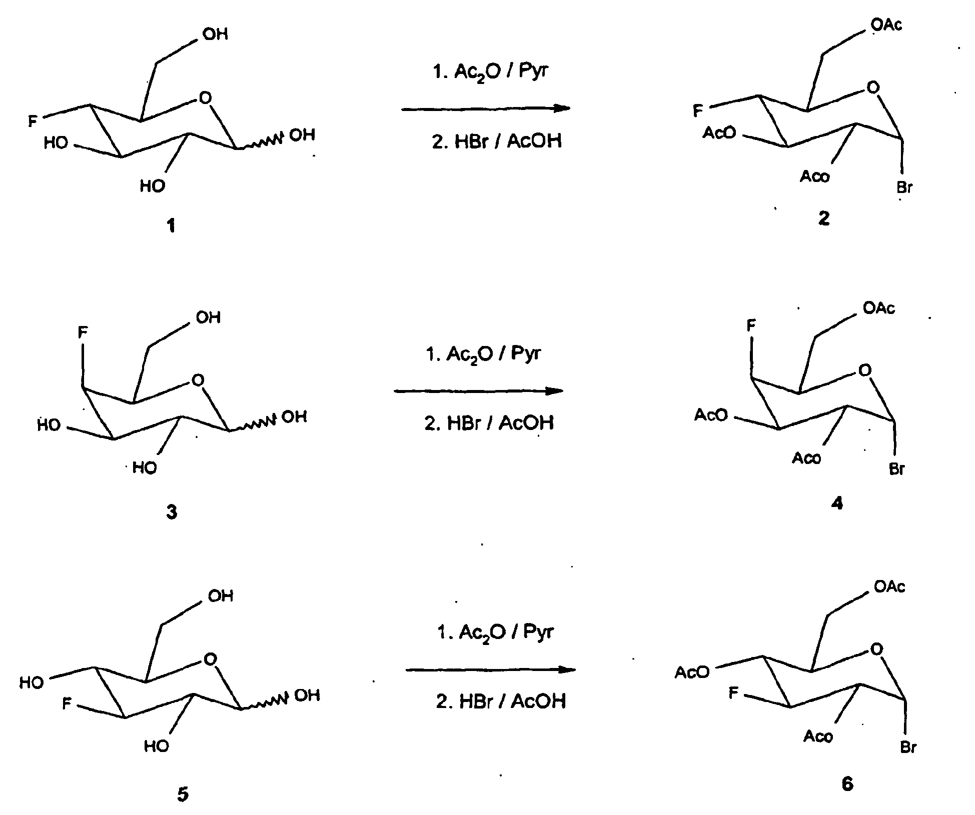

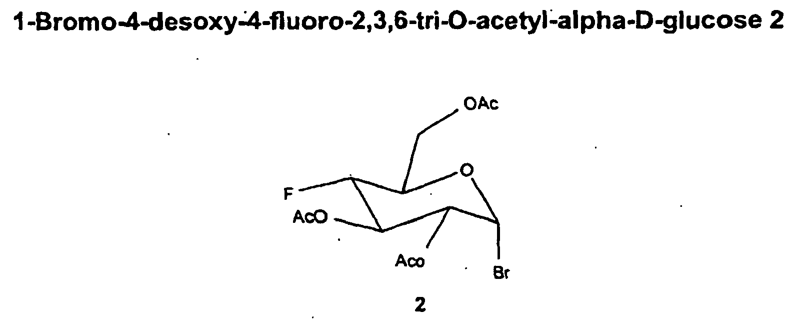

5 g (27.5mmol) 4-Deoxy-4-fluoro-D-glucopyranose **1** (Apollo) werden in 50 ml Pyridin und 50 ml Essigsäureanhydrid suspendiert. Die Reaktionslösung wird 4 Stunden bei 45°C gerührt. Dabei erhält man eine klare Reaktionslösung, die dann eingeengt wird. Man erhält 12 g Rohprodukt. Dieses Rohprodukt wird in 160 ml 33 % iger HBr in Eisessig gelöst und 2 Stunden bei Raumtemperatur stehen gelassen. Die Reaktionslösung wird dann auf eine Mischung von 300 g Eis und 300 ml Ethylacetat gegossen. Die organische Phase wird noch zweimal mit wässriger NaCl-Lösung gewaschen, über wenig Kieselgel filtriert und eingeengt. Der Rückstand wird durch Chromatographie an Kieselgel (Ethylacetat/Heptan = 1/1) getrennt. Man erhält 8.19 g (80 % über 2 Stufen) **2** als hellgelben Feststoff.

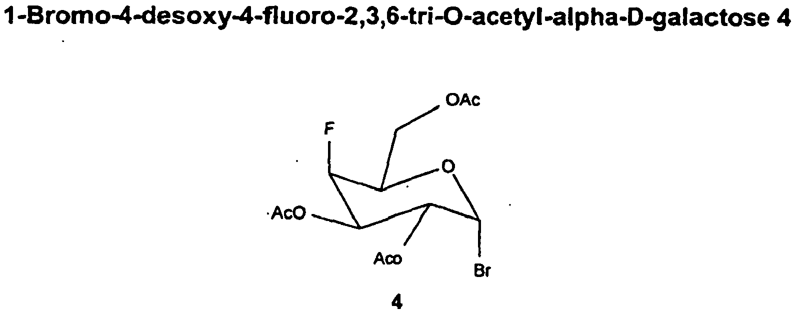

100 mg (0.55 mmol) **3** mit 3.5 ml Pyridin und 3.5 ml Essigsäureanhydrid werden analog der Herstellung von Verbindung **2** umgesetzt. Man erhält 89 mg (44 %) **4** als amorphen Feststoff.

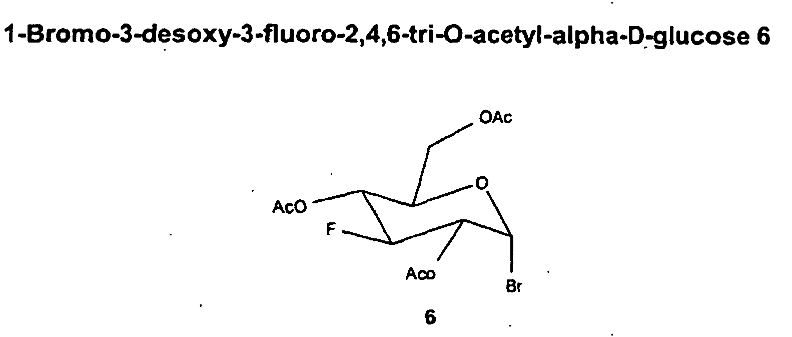

335 mg (1.84 mmol) **5** mit 10 ml Pyridin und 10 ml Essigsäureanhydrid werden analog der Herstellung von Verbindung **2** umgesetzt. Man erhält 628 mg (92 %) **6** als amorphen Feststoff.

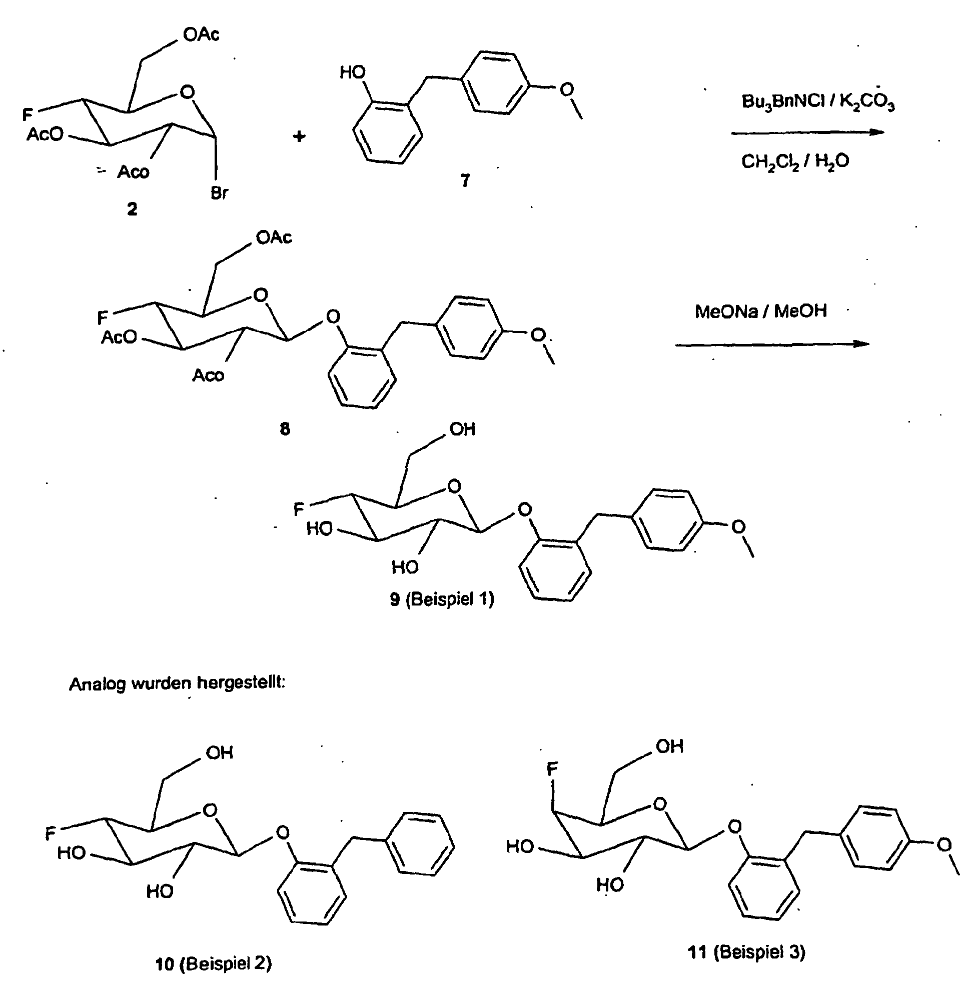

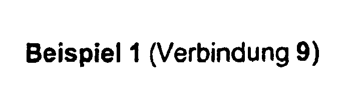

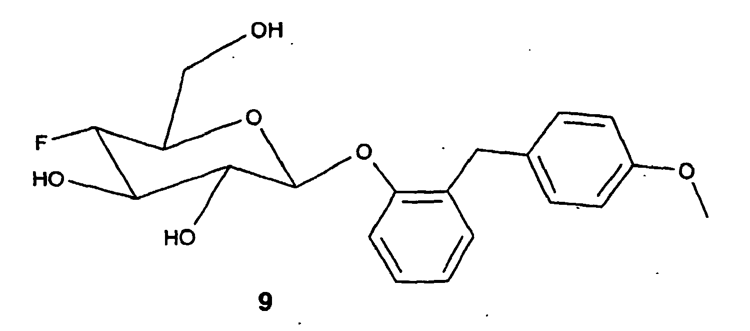

100 mg (0.47 mmol) 2-(4-Methoxy-benzyl)-Phenol **7** und 370 mg (1.17 mmol) Bromid **2** werden in 6 ml Metylenchlorid gelöst. Zu dieser Lösung werden nacheinander 160 mg Bu₃BnNCl (PTK = Phasentransferkatalysator), 320 mg K₂CO₃ und 0.4 ml Wasser zugegeben und anschließend 20 Stunden bei Raumtemperatur gerührt. Die Reaktionslösung wird mit 20 ml Ethylacetat verdünnt und über Kieselgel filtriert. Das Filtrat wird eingeengt und der Rückstand wird durch Chromatographie an Kieselgel (Ethylacetat/Heptan = 1/1) getrennt. Man erhält 72 mg **8** als farblosen Feststoff. Die erhaltenen 72 mg **8** werden in 4 ml Methanol aufgenommen und mit 1 ml 1 N NaOMe/MeOH versetzt. Nach einer Stunde wird mit methanolischer HCl neutralisiert, eingeengt und der Rückstand wird durch Chromatographie an Kieselgel (Methylenchlorid/Methanol/konz.Amoniak. 30/5/1) getrennt. Man erhält 29 mg **9** als farblosen Feststoff. C₂₀H₂₃FO₆ (378.40) MS(ESI⁻) 423.22 (M + CHO₂⁻).

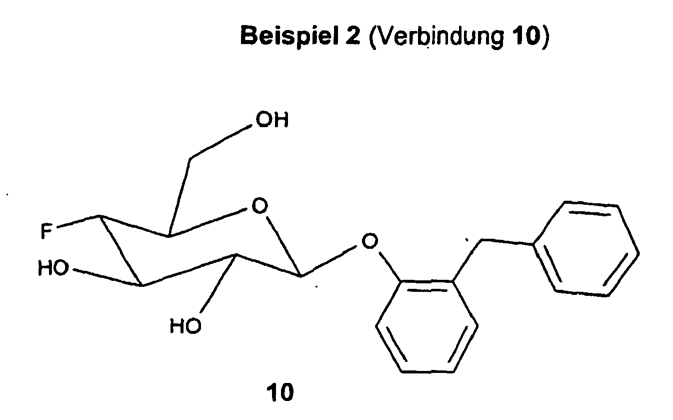

100 mg (0.47 mmol) 2-Benzyl-phenol und 370 mg (1.17 mmol) Bromid **2** werden analog der Synthese von Verbindung **9** umgesetzt und man erhält 31 mg **10** als farblosen Feststoff. C₁₉H₂₁FO₅ (348.37) MS(ESI⁻) 393.15 (M + CHO₂⁻).

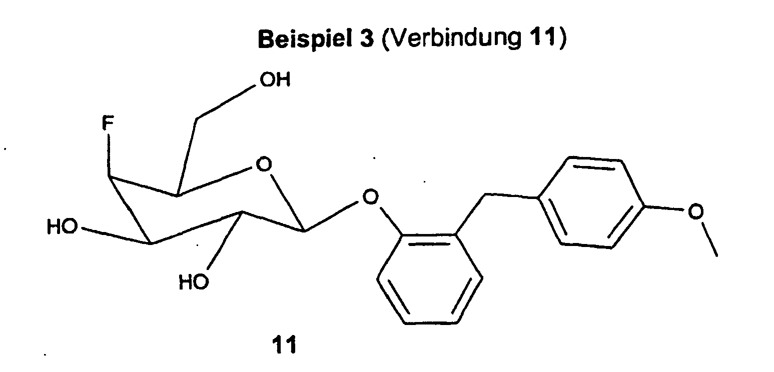

200 mg (0.94 mmol) 2-(4-Methoxy-benzyl)-Phenol **7** und 200 mg (0.63 mmol) Bromid **4** werden analog der Synthese von Verbindung **9** umgesetzt und man erhält 110 mg **11** als farblosen Feststoff. C₂₀H₂₃FO₆ (378.40) MS(ESI⁻) 423.22 (M + CHO₂⁻).

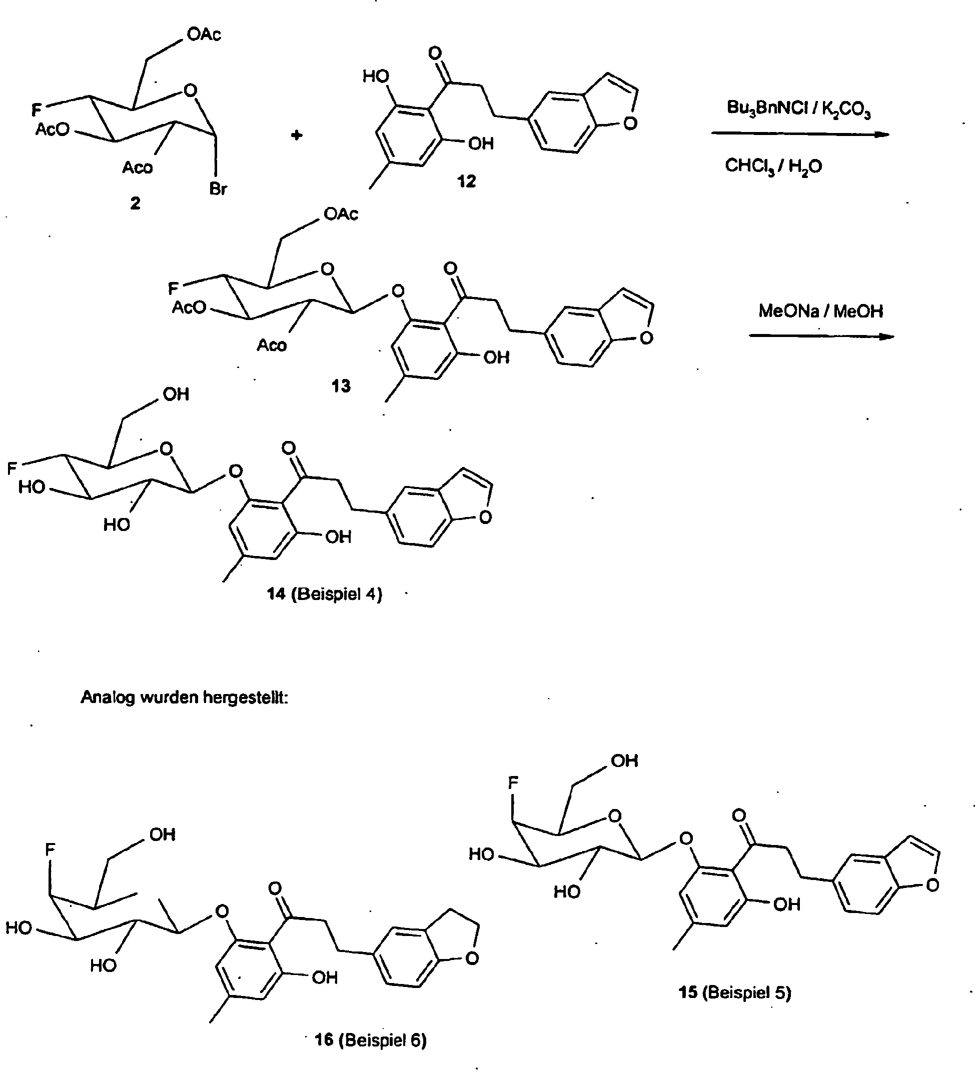

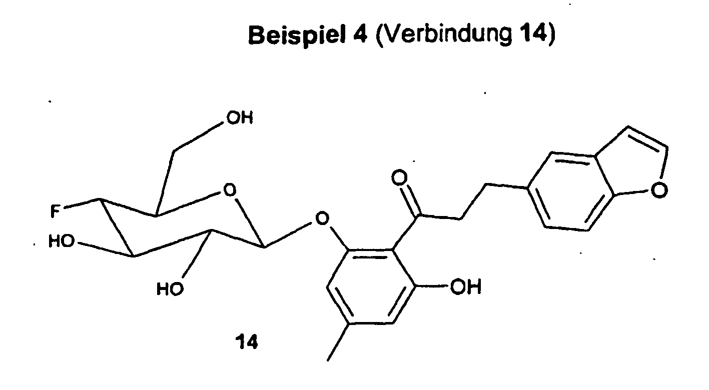

90 mg (0.30 mmol) 3-Benzofuran-5-yl-1-(2,6-dihydroxy-4-methyl-phenyl)-propan-1-on **12** und 280 mg (0.76 mmol) Bromid **2** werden analog der Synthese von Verbindung **8** umgesetzt und man erhält 400 mg **13** als Rohprodukt weiches direkt mit NaOMe/MeOH analog der Synthese von Glucosid **9** entschützt wird. Man erhält 75 mg **14** (54 % über 2 Stufen) als farblosen Feststoff. C₂₄H₂₅FO₈ (460.46) MS(ESI⁻) 459.03 (M - H⁺).

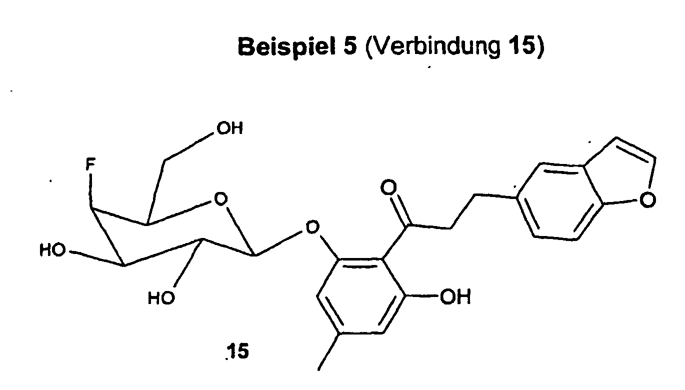

100 mg (0.33 mmol) 3-Benzofuran-5-yl-1-(2,6-dihydroxy-4-methyl-phenyl)-propan-1-on **12** und 150 mg (0.40 mmol) Bromid **4** werden analog der Synthese von Verbindung **14** umgesetzt und man erhält 75 mg **15** als farblosen Feststoff. C₂₄H₂₅FO₈ (460.46) MS(ESI⁻) 459.03 (M - H⁺).

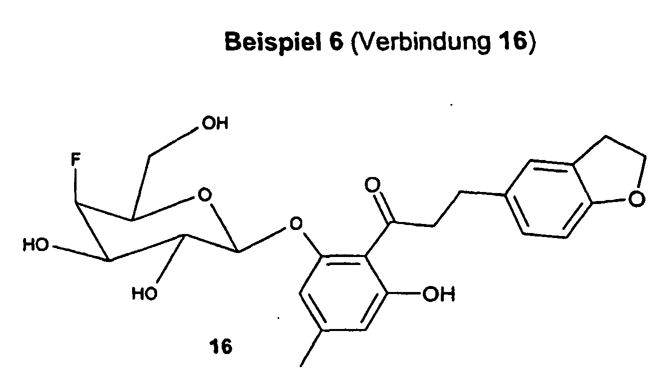

150 mg (0.5 mmol) 3-(2,3-Dihydroxy-benzofuran-5-yl-1-(2,6-dihydroxy-4-methylphenyl)-propan-1-on und 150 mg (0.40 mmol) Bromid **4** werden analog der Synthese von Verbindung **14** umgesetzt und man erhält 75 mg **16** als farblosen Feststoff. C₂₄H₂₇FO₈ (462.46) MS(ESI⁻) 461.03 (M - H⁺).

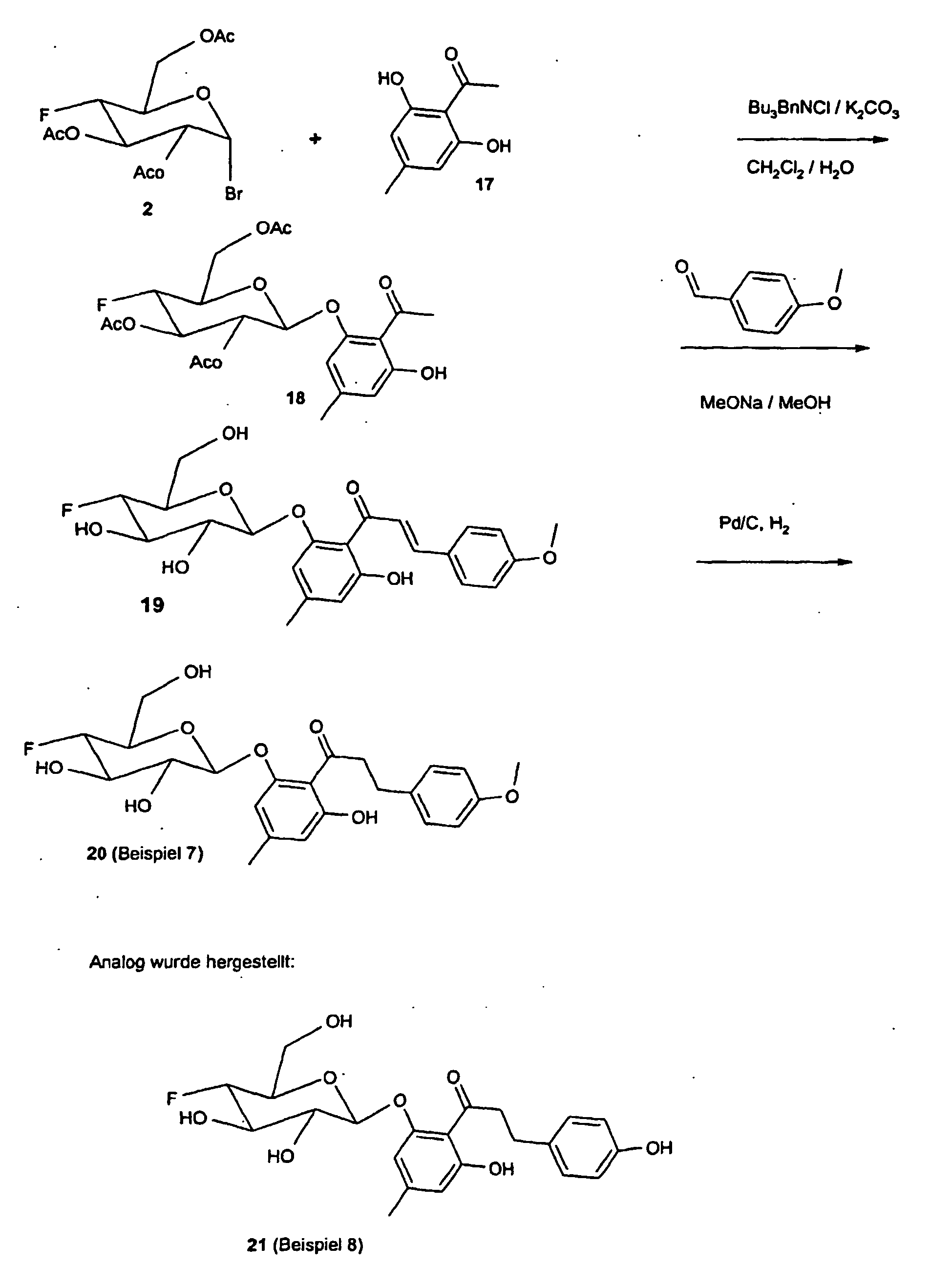

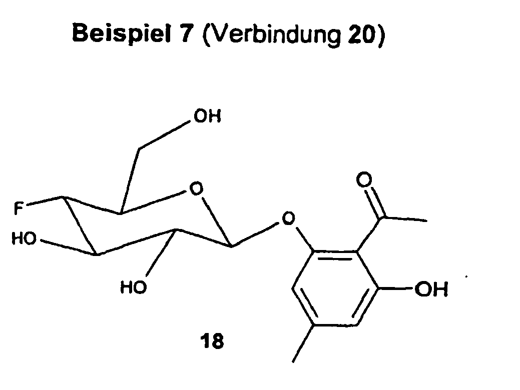

1.0 g (6.0 mmol) 1-(2,6-Dihydroxy-4-methyl-phenyl)ethanon **17** und 1.0 g (2.7 mmol) Bromid 2 werden in 30 ml Methylenchlorid gelöst. Zu dieser Lösung gibt man unter kräftigem Rühren nacheinander 800 mg Benzyl-tributylammonium-chlorid (PTK), 1.6 g Kaliumcarbonat und 1.5 ml Wasser zu. Diese Suspension wird 18 Stunden unter Lichtschutz (Aluminiumfolie) gerührt und dann mit 150 ml Essigester und 150 ml n-Heptan verdünnt. Die festen Bestandteile werden über wenig Kieselgel filtriert und eingeengt. Der Rückstand wird durch Chromatographie an Kieselgel (Ethylacetat/Heptan = 1/2) getrennt. Man erhält 430 mg **18** (schlecht von einem gleich laufenden Nebenprodukt abtrennbar, deshalb nur ungefähr 50 %ige Reinheit. Das Nebenprodukt kann auf der nächsten Stufe leicht abgetrennt werden) als hellgelber Feststoff. C₂₁H₂₅O₁₀F (456.43) MS(ESI⁻): 455.25 (M - H⁺).

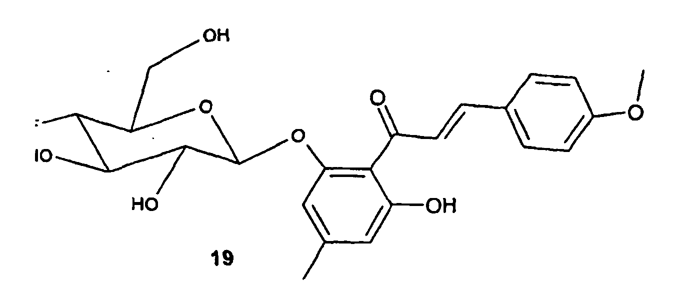

200 mg Verbindung **18** (rund 50 % rein) und 225 mg Anisaldehyd (Fluka) werden in 10 ml Methanol gelöst. Nach Zugabe von 5 ml 1 N NaOMe/MeOH-Lösung läst man die Reaktionslösung 12 Stunden am Rückfluss kochen. Die Reaktionslösung wird mit methanolischer HCl neutralisiert, eingeengt und der Rückstand wird durch Chromatographie an Kieselgel (Methylenchlorid/Methanol/konz.Amoniak, 30/5/1) getrennt. Man erhält 60 mg **19** als gelben Feststoff.

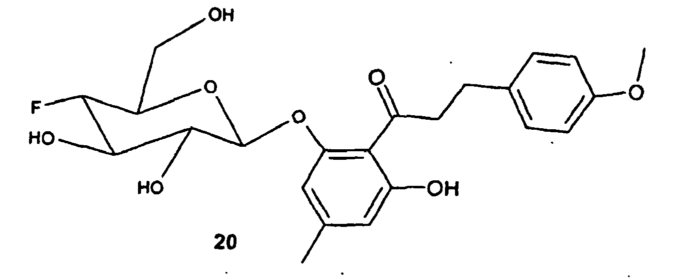

60 mg (0.13 mmol) Chalcon **19** und 50 mg Pd/C (10% Pd) werden in 15 ml Methanol suspendiert und unter einer 5 bar Wasserstoffatmosphäre 5 h bei Raumtemperatur hydriert. Die Reaktionslösung wird eingeengt und der Rückstand mit Flashchromatographie (Methylenchlorid/Methanol/konz.Amoniak, 30/5/1) gereinigt. Ausbeute 25 mg (42%) **20** als weißer, amorpher Feststoff. C₂₃H₂₇FO₈ (424.47) MS(ESI⁻): 449.17 (M - H⁺).

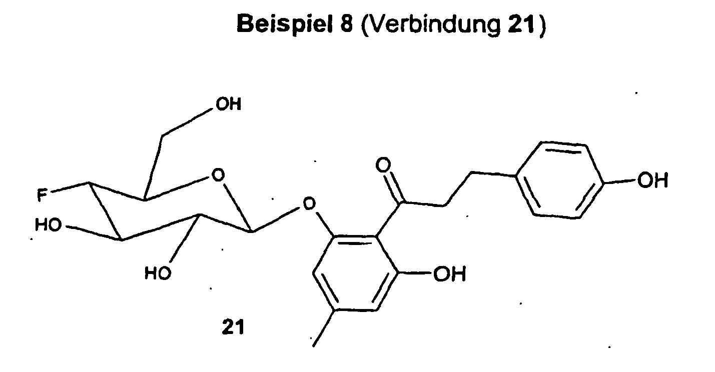

200 mg Verbindung **18** (rund 50 % rein) und 350 mg p-Benzyloxy-benzaldehyd (Fluka) werden analog der Synthese von Verbindung **20** umgesetzt. Man erhält 36 mg **21** als farblosen Feststoff. C₂₂H₂₅FO₈ (436.44) MS(ESI⁻) 481.08 (M + CHO₂⁻).

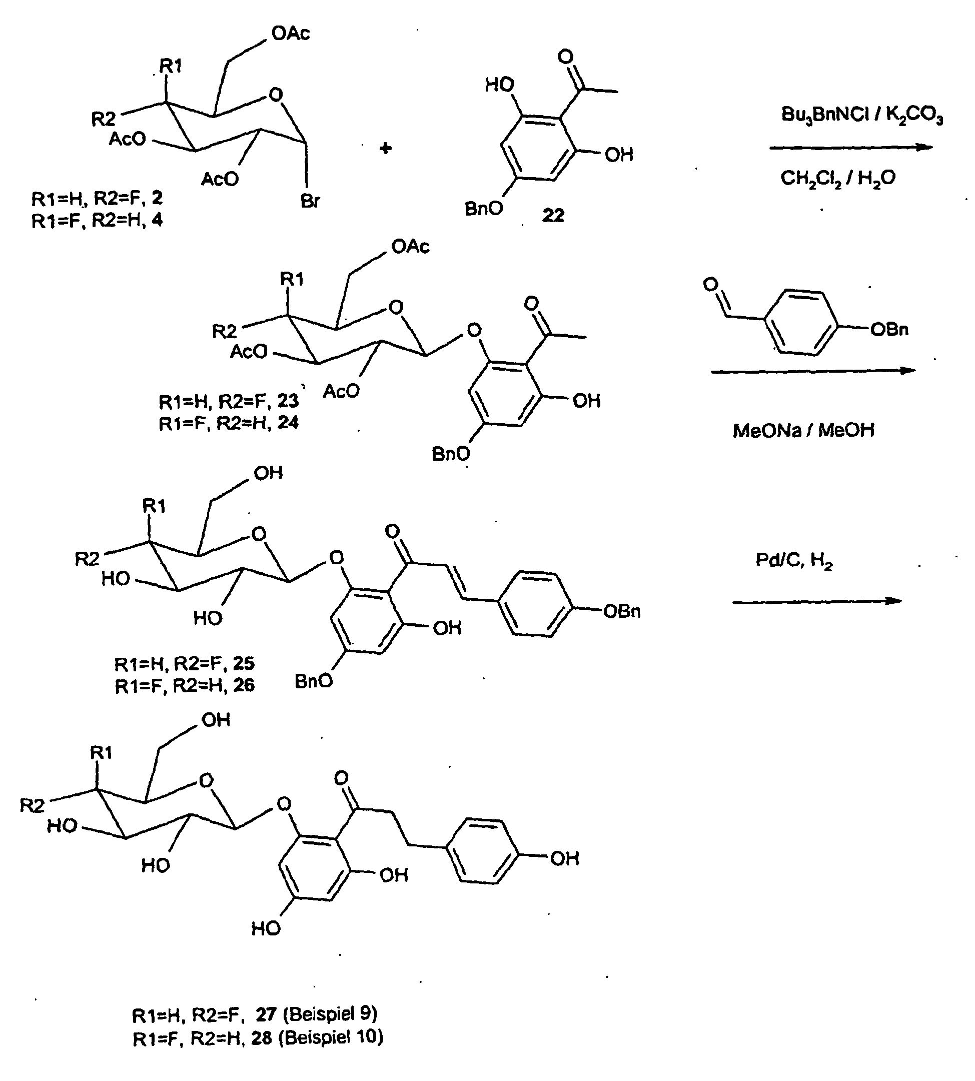

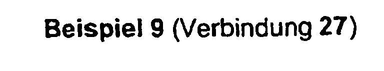

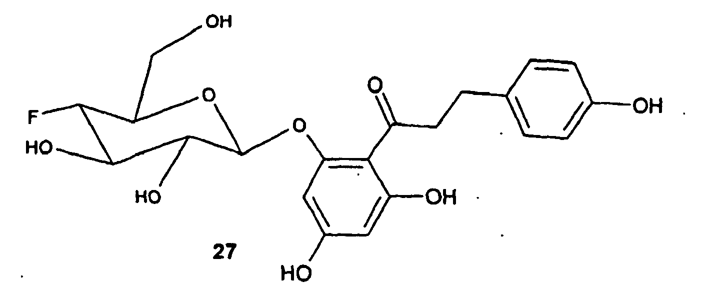

350 mg Bromid 2, 100 mg Phenol **22** und 350 mg p-Benzyloxy-benzaldehyd (Fluka) werden analog der Synthese von Verbindung **21** umgesetzt. Man erhält 40 mg **27** als farblosen Feststoff. C₂₁H₂₃FO₉ (438.41) MS(ESI⁻) 483.15 (M + CHO₂⁻ ).

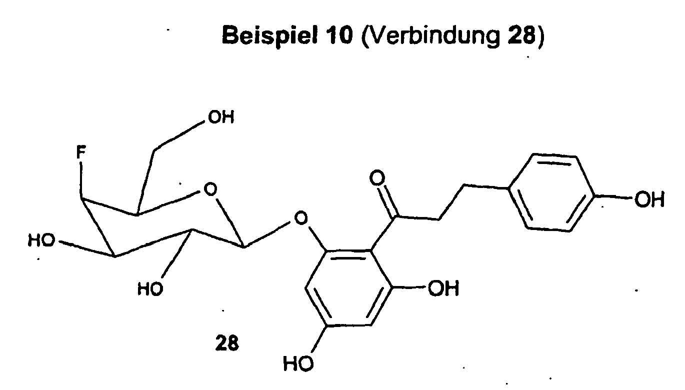

110 mg Bromid **4** 80 mg Phenol **22** und 350 mg p-Benzyloxy-benzaldehyd (Fluka) werden analog der Synthese von Verbindung **21** umgesetzt. Man erhält 50 mg **28** als farblosen Feststoff. C₂₁H₂₃FO₉ (438.41) MS(ESI⁻) 483.15 (M + CHO₂⁻).

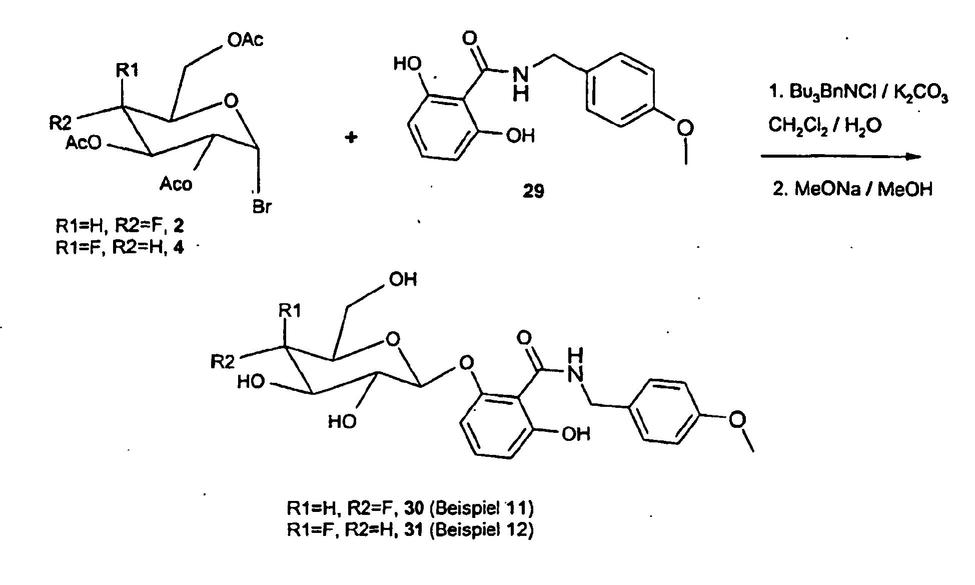

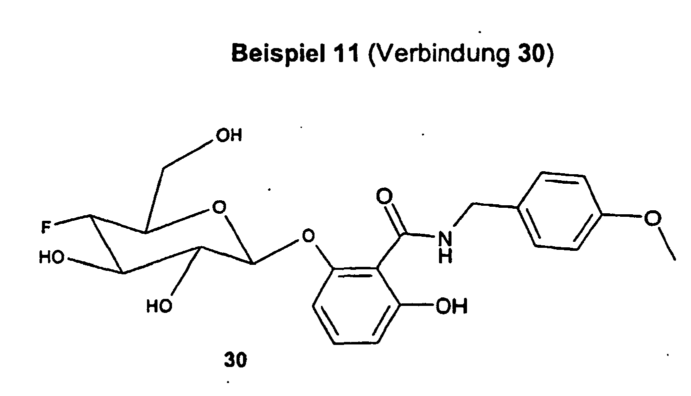

200 mg Bromid **2** und 300 mg Phenol **29** werden analog der Synthese von Verbindung **14** umgesetzt. Man erhält 40 mg **30** als farblosen Feststoff. C₂₁H₂₄FNO₈ (437.43) MS(ESI⁻) 482.15 (M + CHO₂⁻).

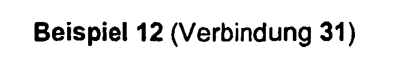

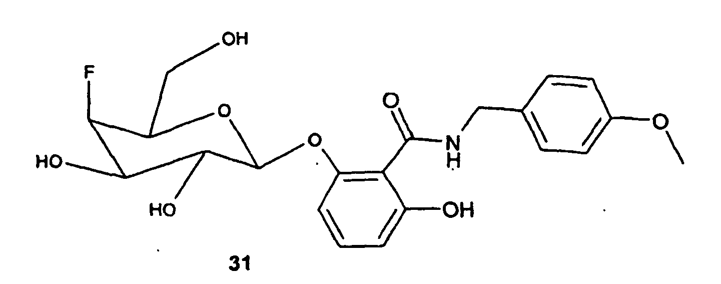

200 mg Bromid **4** und 300 mg Phenol **29** werden analog der Synthese von Verbindung **14** umgesetzt. Man erhält 115 mg **31** als farblosen Feststoff. C₂₁H₂₄FNO₈ (437.43) MS(ESI⁻) 482.15 (M + CHO₂⁻).

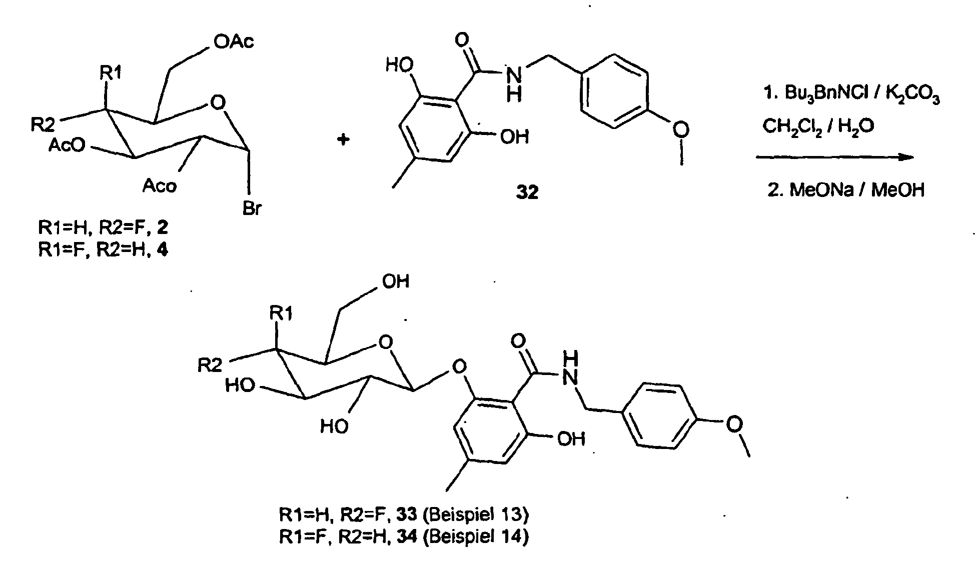

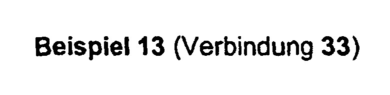

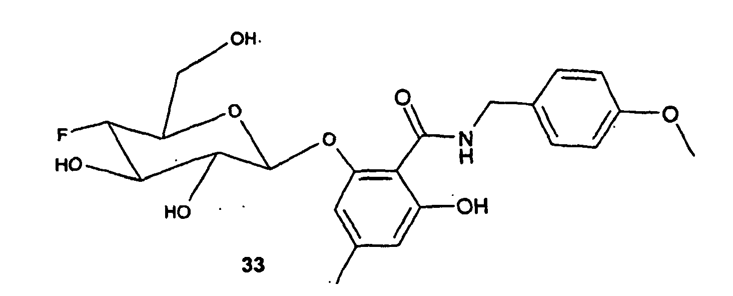

200 mg Bromid **2** und 300 mg Phenol **32** werden analog der Synthese von Verbindung **14** umgesetzt. Man erhält 80 mg **33** als farblosen Feststoff. C₂₂H₂₆FNO₈ (451.45) MS(ESI⁻) 496.17 (M + CHO₂⁻).

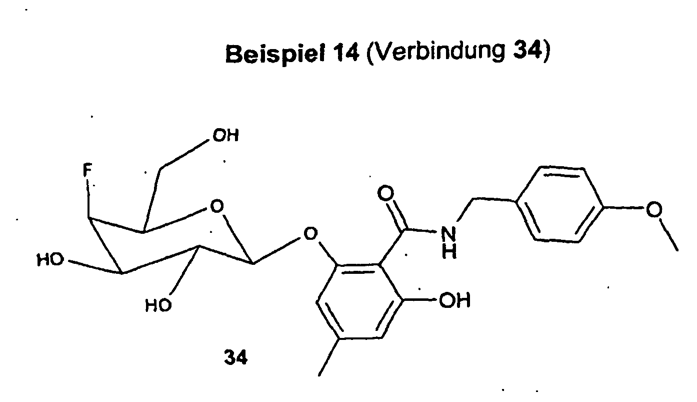

200 mg Bromid **4** und 300 mg Phenol **32** werden analog der Synthese von Verbindung **14** umgesetzt. Man erhält 130 mg **34** als farblosen Feststoff. C₂₁H₂₄FNO₈ (451.45) MS(ESI⁻) 496.15 (M + CHO₂⁻).

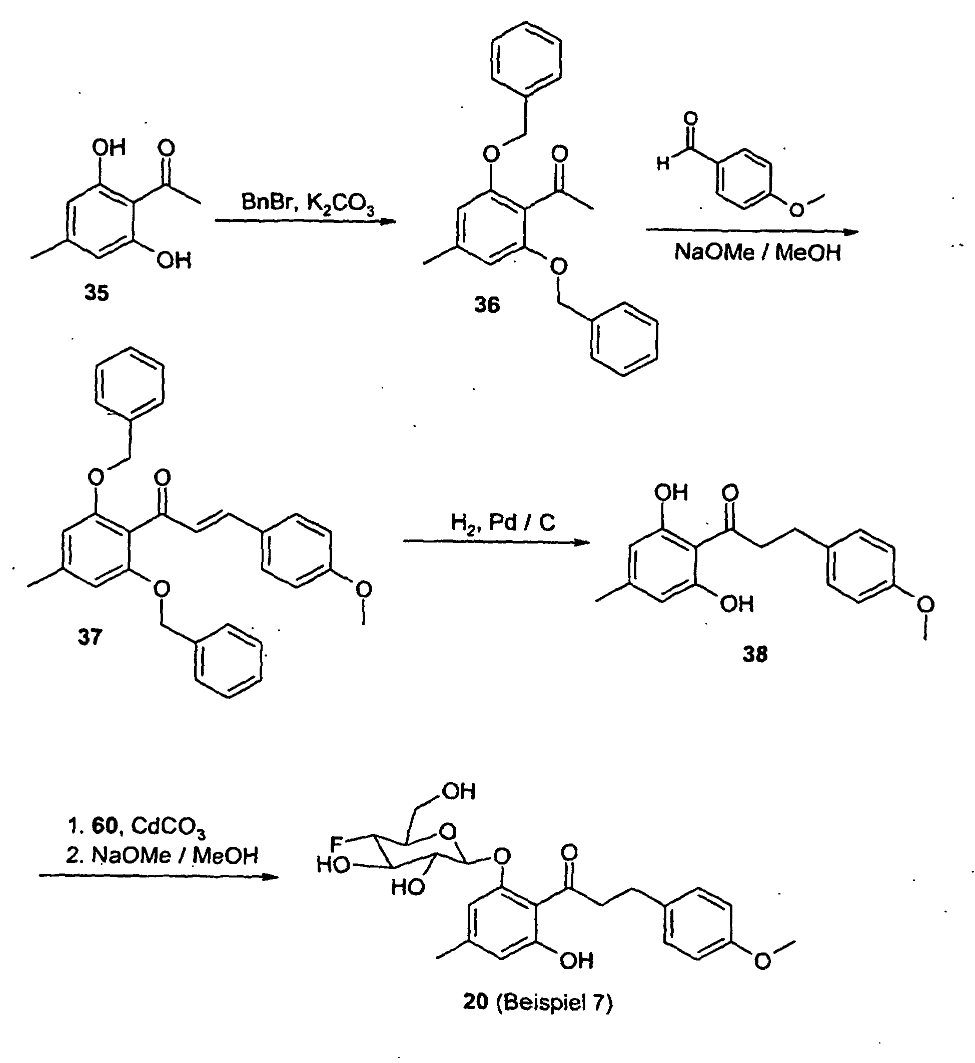

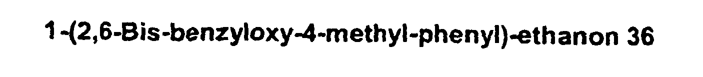

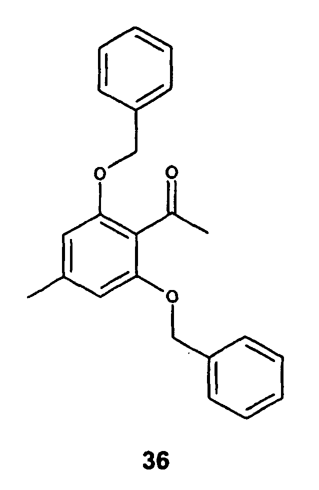

1,62 g (9,75 mmol) 1-(2,6-Dihydroxy-4-methyl-phenyl)-ethanon (**35**) werden in 30 ml Dimethylformamid gelöst und mit 4,0 ml (33,7 mmol) Benzylbromid und 13,8 g (100 mmol) Kaliumcarbonat versetzt. Das Reaktionsgemisch wird 3 Stunden bei Raumtemperatur gerührt. Danach wird Wasser zugegeben und zweimal mit Essigsäureethylester extrahiert. Die vereinigten organischen Phasen werden mit gesättigter Natriumchloridlösung gewaschen, über Natriumsulfat getrocknet und am Rotationsverdampfer eingeengt. Man erhält 1,35 g (40 %) der Verbindung **36** als farbloses kristallines Produkt. C₂₃H₂₂O₃ (346,2) MS (ESI⁺): 347,15 (M+H⁺). 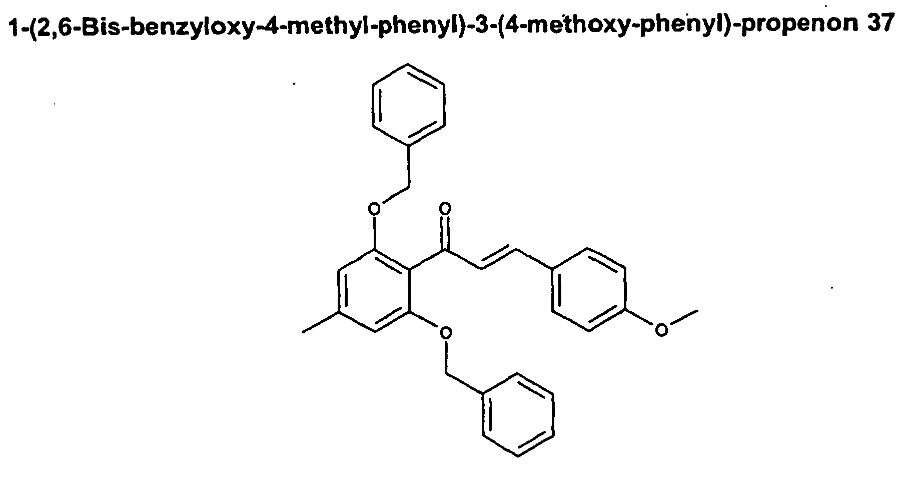 3,46 g (10 mmol) 1-(2,6-Bis-benzyloxy-4-methyl-phenyl)-ethanon (**36**) werden in 150 ml Ethanol gelöst und 1,34 ml p-Anisaldehyd zugegeben. Danach werden 7 ml wässriger Kaliumhydroxyd-Lösung zugetropft. Die Reaktion rührt 12 Stunden bei Raumtemperatur.
Am Rotationsverdampfer wird die Hälfte des Lösungsmittels abgezogen. Unter Eiskühlung wird mit 2 M Salzsäure neutralisiert und danach die Mischung mit Wasser und Essigsäureethylester dreimal ausgeschüttelt. Die organischen Phasen werden vereinigt, mit gesättigter Natriumchlorid-Lösung gewaschen, über Natriumsulfat getrocknet und einrotiert. Das isolierte Öl kristallisiert aus. Die Kristalle werden in Diethylether verrührt, abgesaugt und getrocknet. Man erhält 4,3 g (92%) der Verbindung 37 als farblosen Feststoff. g/mol C₃₁H₂₈O₄ (464,2) MS (ESI⁺): 465,10 (M+H⁺).

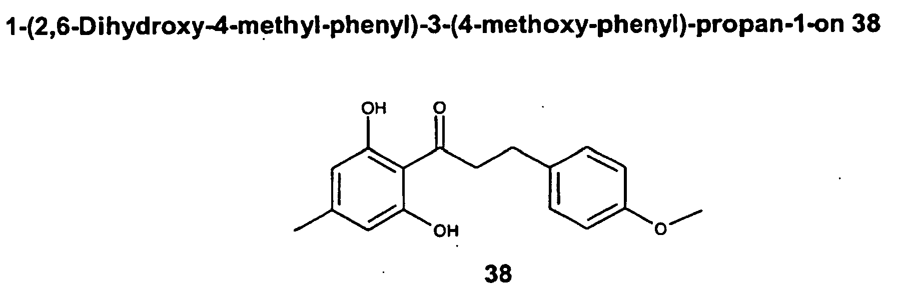

1,50 g (3,23 mmol) 1-(2,6-Bis-benzyloxy-4-methyl-phenyl)-ethanon (**37**) werden in 40 ml Essigsäureethylester gelöst und in Argonatmosphäre 400 mg Palladium auf Aktivkohle, 10% zugegeben Im Hydrierautoklaven wird bei 3 bar und Raumtemperatur 2 Stunden hydriert. Der Katalysator wird danach abfiltriert, mit Essigsäureethylester gewaschen und die erhaltene Lösung wird am Rotationsverdampfer eingeengt. Das Rohprodukt wird durch Säulenchromatgraphie (SiO₂. Ethylacetat/ n-Heptan 1:3) gereinigt.
Man isoliert 600 mg des Produktes **38** (65%) als farblosen Feststoff. C₁₇H₁₈O₄ 286,3 MS (ESI⁺): 287.10 (M+H⁺).

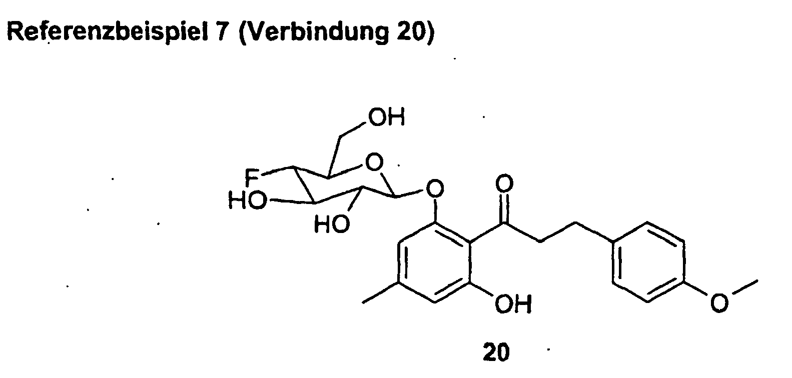

174,4 mg (0,61 mmol) der Verbindung **38** werden in 50 ml Toluol gelöst und mit 340 mg (0,61 mmol) des Bromids **60** und 421 mg Cadmiumcarbonat (2,44 mmol) versetzt. Das Reaktionsgemisch wird 1 h am Wasserabscheider refluxiert. Cadmiumcarbonat wird abfiltriert und die erhaltene klare Lösung wird am Rotationsverdampfer eingeengt. Das Rohprodukt wird in 25 ml Methanol suspendiert und mit 5,0 ml einer 0,5 M methanolischen NaOMe-Lösung versetzt und 12 h bei Raumtemperatur gerührt. Die Reaktionslösung wird durch Zugabe von methanolischer HCl neutralisiert und flashchromatographisch gereinigt (SiO2, EtOAc / Heptan 1:4 → 1:1) gereinigt. Man erhält 78,8 mg (29%) der Verbindung **20** als farblosen Feststoff. C₂₃H₂₇FO₈ 450,5 MS (ESI⁺): 473,15 (M+Na⁺).

Auf analoge Weise werden die Verbindungen **40** (Beispiel 15), **41** (Beispiel 16), **42** (Beispiel 17) und **43** (Beispiel 18) dargestellt.

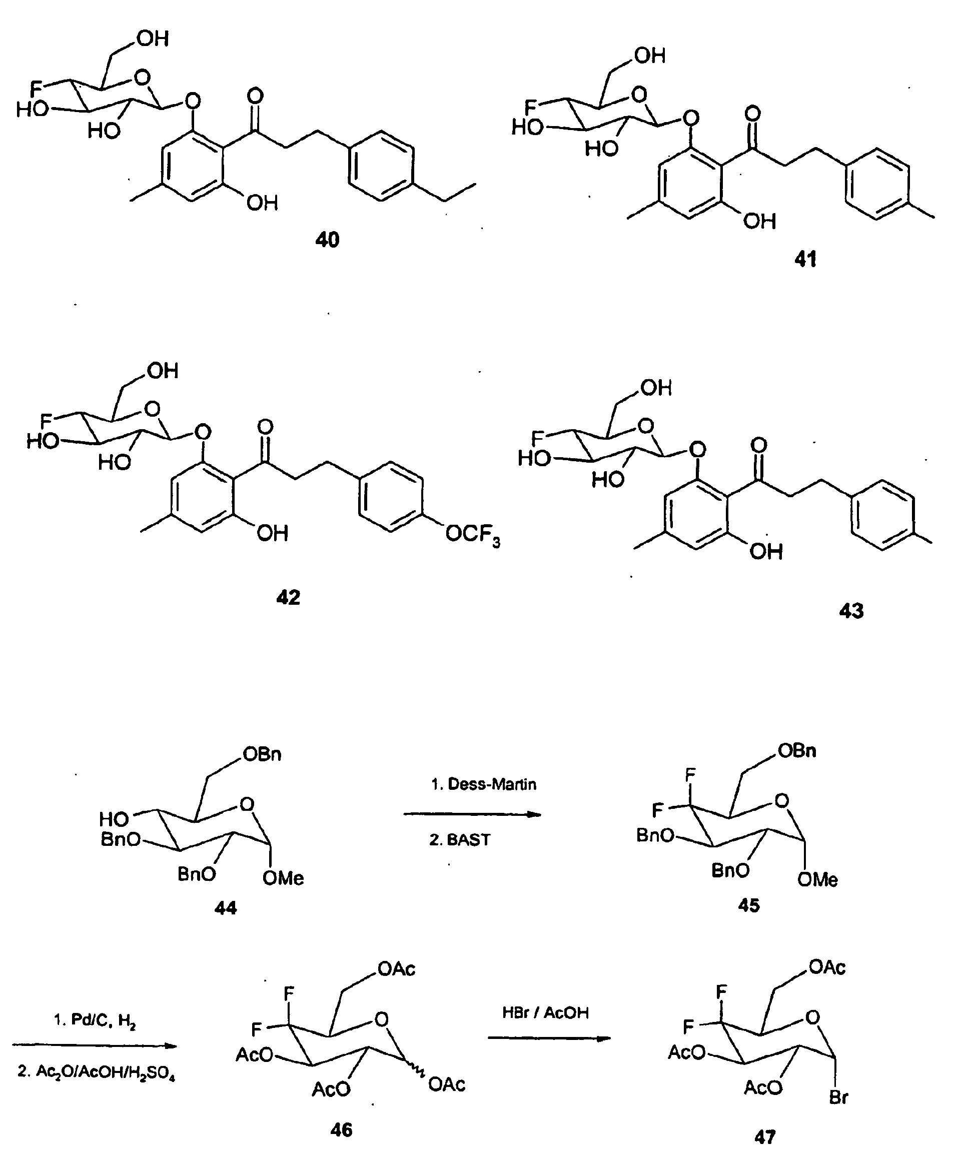

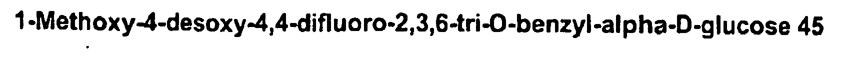

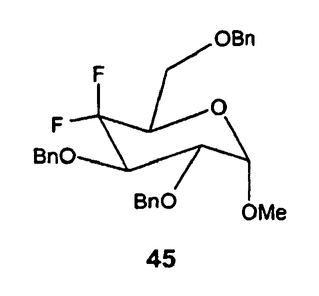

3.69 g (7.9 mmol) 1-Methoxy-2,3,6-tri-O-benzyl-α-D-glucose **44** (Tetrahedron Asymmetry 11 (2000) 385-387) werden in 110 ml Methylenchlorid gelöst und unter einer Argonatmosphere werden 3.6 g (8.5 mmol) Dess-Martin-Reagenz (Aldrich) zugetropft. Nach 3 Stunden bei Raumtemperatur wird mit 300 ml Essigester/n-Heptan (1:1) verdünnt und 1 x mit NaHCO₃- und 1 x mit Na₂S₂O₃-Lösung gewaschen. Die organische Phase wird über Kieselgel filtriert und eingeengt. Der Rückstand wird durch Chromatographie an Kieselgel (Ethylacetat/n-Heptan 1:1) getrennt. Man erhält 2.9 g (79 %) Keton. Dieses wird in 30 ml Methylenchlorid gelöst und unter einer Argonatmosphere werden 4 ml BAST (Aldrich) zugetroft. Nach 20 Stunden bei Raumtemperatur wird mit 200 ml Essigester verdünnt und mit kalter NaHCO₃-Lösung vorsichtig (sprudelt stark) gewaschen. Die organische Phase wird über Kieselgel filtriert und eingeengt. Der Rückstand wird durch Chromatographie an Kieselgel (Ethylacetat/n-Heptan 1:1) getrennt. Man erhält 2.6 g (85 %) **45** als farbloses Öl.

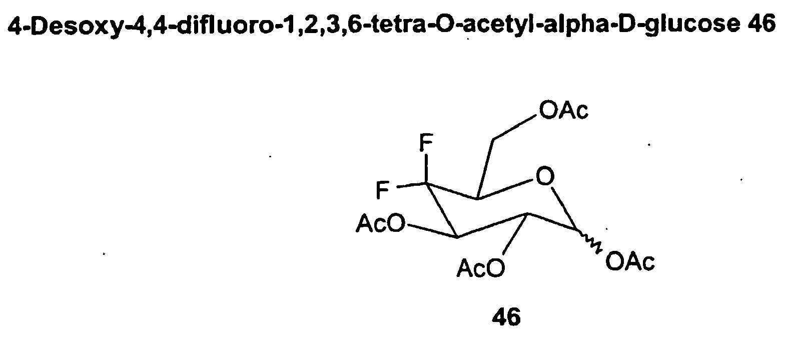

2.3 g (4.7 mmol) **45** und 2 g Pd/C (10 % Pd) werden in 150 ml Methanol und 10 ml Essigsäure gelöst und unter einer 5 bar Wasserstoffatmosphäre 16 h bei Raumtemperatur hydriert. Die Reaktionslösung wird eingeengt und der Rückstand mit Flashchromatographie (Methylenchlorid/Methanol/konz.Amoniak, 30/5/1) gereinigt.
Ausbeute 850 mg (83%) 1-Methoxy-4-desoxy-4,4-difluoro-alpha-D-glucose als weißen, amorpher Feststoff. C₇H₁₂F₂O₅ (214.17) MS(DCI): 215.4 (M + H⁺).
Davon werden 700 mg (3.3 mmol) in 3.5 ml Essigsäure und 6.3 ml Essigsäureanhydrid gelöst. Nach Zugabe von 0.2 ml konz. H₂SO₄ wird 5 h bei 60 °C gerührt.. Die Reaktionslösung wird dann auf eine Mischung von 30 g Eis und 30 ml Ethylacetat gegossen. Die organische Phase wird noch zweimal mit wässriger NaCl-Lösung gewaschen, über wenig Kieselgel filtriert und eingeengt. Der Rückstand wird durch Chromatographie an Kieselgel (Ethylacetat/n-Heptan 1:1) getrennt. Man erhält 300 mg (25 %) **46** als Anomerengemisch. C₁₄H₁₈F₂O₉ (368.29) MS(DCI): 369.3 (M + H⁺).

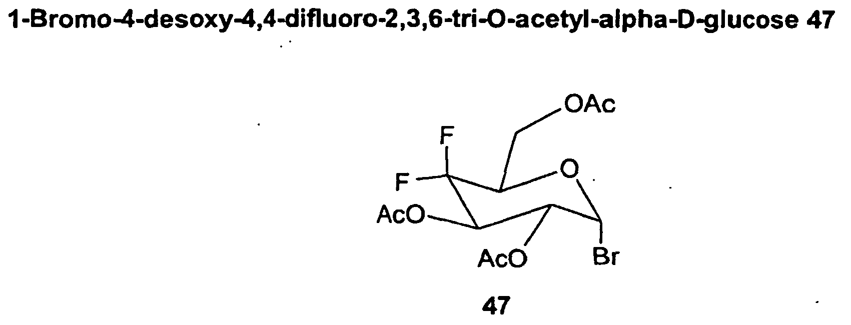

300 mg (0.8 mmol) Tetraacetat **46** werden in 13 ml 33 %-iger HBr in Eisessig gelöst und 6 Stunden bei Raumtemperatur stehen gelassen. Die Reaktionslösung wird dann auf eine Mischung von 10 g Eis und 10 ml Ethylacetat gegossen. Die organische Phase wird noch zweimal mit wässriger NaCl-Lösung gewaschen, über wenig Kieselgel filtriert und eingeengt. Der Rückstand wird durch Chromatographie an Kieselgel (Ethylacetat/Heptan 1:1) getrennt. Man erhält 112 mg (35 %) **47** als farblosen Feststoff. C₁₂H₁₅BrF₂O₇(389.15) MS(DCI): 389.2 (M + H⁺).

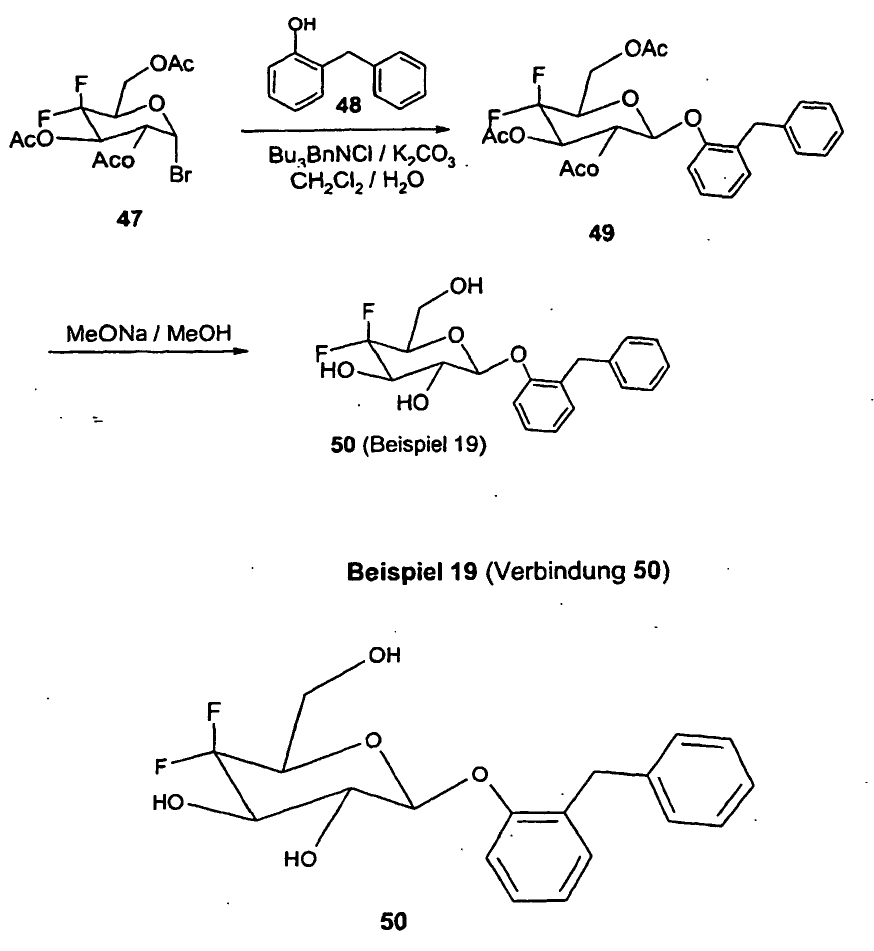

100 mg (0.47 mmol) 2-Benzyl-phenol (Aldrich) und 40 mg (0.10 mmol) Difluor-bromid **47** werden analog der Synthese von Verbindung **9** umgesetzt und man erhält 21 mg **50** als farblosen Feststoff. C₁₉H₂₀F₂O₅ (366.37) MS(ESI⁻) 411.15 (M + CHO₂⁻).

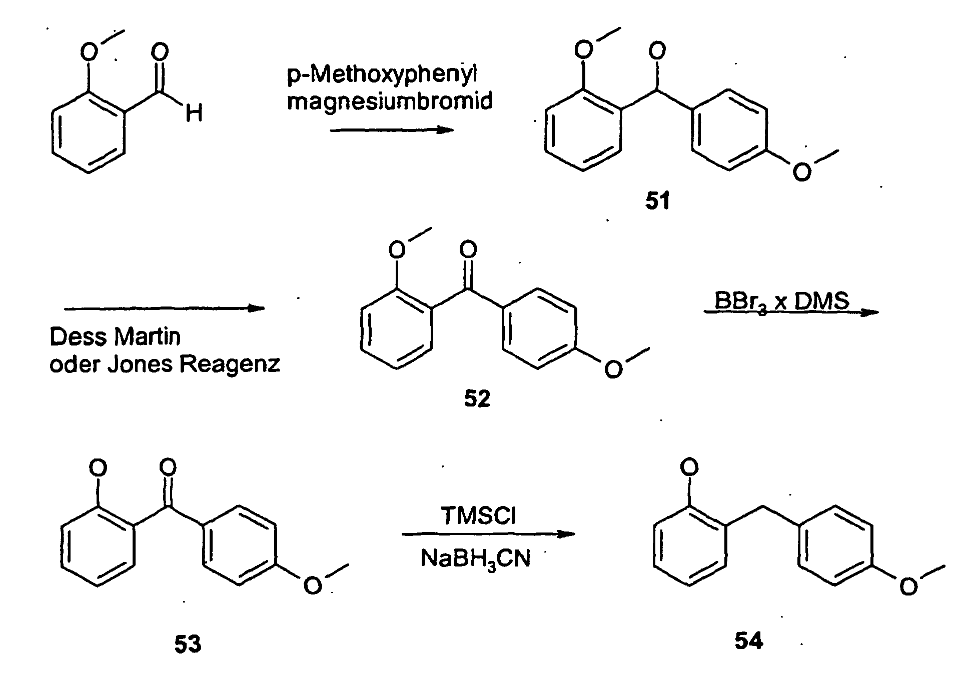

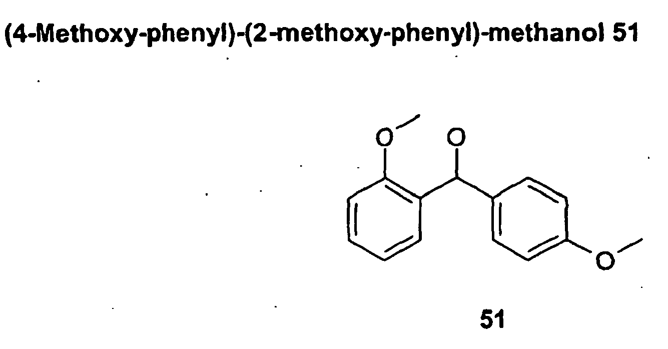

1,5g o-Anisaldehyd werden in THF gelöst und auf 0°C gekühlt. 24,2 ml 4-Methoxyphenylmagnesiumbromid (0.5M in THF) werden zum Ansatz gegeben. Die Reaktionslösung wird über Nacht bei Raumtemperatur gerührt, anschließend in 20% NH₄Cl-Lösung eingegossen und mit Ethylacetat extrahiert. Man erhält 2,63 g des Produktes, das ohne weitere Aufreinigung eingesetzt werden kann. C₁₅H₁₆O₃ (244.29) MS (ESI⁺) 227.05 (M-OH)⁺

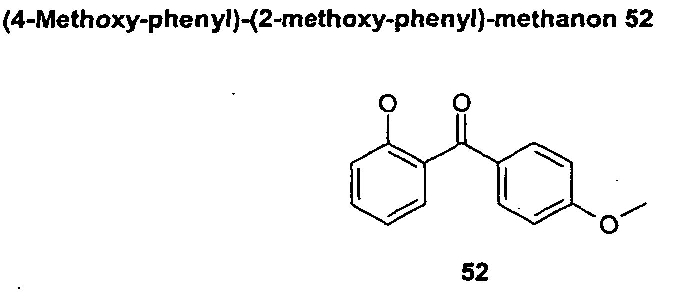

2,63g (4-Methoxy-phenyl)-(2-methoxy-phenyl)-methanol **51** werden in Dichlormethan gelöst und 5,03 g Dess Martin Reagenz zugegeben. Der Ansatz wird für 2 h bei Raumtemperatur gerührt. Dann werden 20% Na₂SO₃- und NaHCO₃-Lsg. zugegeben und das Gemisch mit Diethylether extrahiert. Die organische Phase wird mit ges. NaCl-Lsg. extrahiert und über Natriumsulfat getrocknet. Die Lösung wird im Vakuum eingeengt und durch Säulenfiltration gereinigt. Man erhält 2,61g **52**. C₁₅H₁₄O₃ (242.28) MS (ESI⁺) 243.04 (M+H⁺)
Alternativ dazu kann Oxidation mit Jones-Reagenz erfolgen:

155 mg (4-Methoxy-phenyl)-(2-methoxy-phenyl)-methanol **51** werden in 10ml Aceton gelöst und 2ml Jones-Reagenz werden tropfenweise zugegeben. Nach 2h bei Raumtemperatur werden 50ml MTB-Ether und 30ml Wasser zum Ansatz gegeben. Die organische Phase wird mehrfach mit Wasser gewaschen, die organische Phase mit gesättigter NaCl-Lsg. extrahiert, über Natriumsulfat getrocknet und zur Trockne eingeengt. Das so erhaltene Produkt (126 mg) besitzt eine für die weitere Umsetzung ausreichende Reinheit.

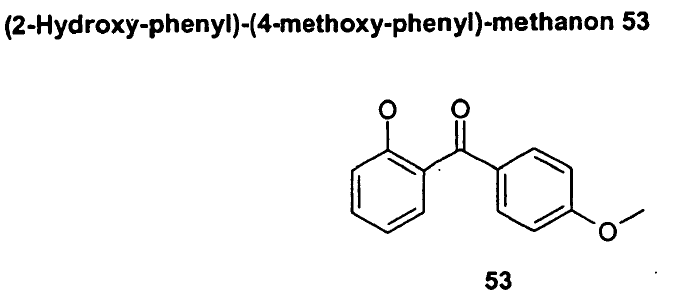

2,61 g (4-Methoxy-phenyl)-(2-methoxy-phenyl)-methanon **52** werden in Dichlormethan gelöst. Der Ansatz wird im Eisbad gekühlt und 3,71 g Bortribromid-Dimethylsulfid Komplex werden zugegeben.Der Ansatz wird auf Raumtemperatur erwärmt und für 3 h rühren lassen. Anschliessend wird die Reaktion durch Eingiessen in Eiswasser abgebrochen, die Dichlormethanphase wird abgetrennt und die wässrige Phase wird mehrfach mit Ethylacetat extrahiert. Die vereinigte organische Phase wird mit Wasser und Natriumchlorid-Lösung. gewaschen, über Natriumsulfat getrocknet und eingeengt. Das Rohprodukt wird mit Ethylacetat / Heptan über Kieselgel chromatographiert. Man erhält 1,26 g des Produktes. C₁₄H₁₂O₃ (228.25) MS (DCI) 229.2 (M+H⁺)

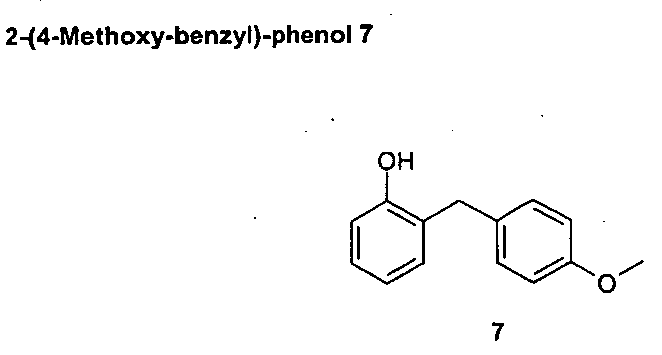

0,78g (2-Hydroxy-phenyl)-(4-methoxy-phenyl)-methanon werden in Acetonitril gelöst und auf 0°C gekühlt. 2ml TMSCI werden zum Ansatz getropft und dann 1g Natriumcyanoborhydrid zugeben. Der Ansatz wird für 3 h bei Raumtemperatur gerührt. Die Reaktionslösung wird mit Dichlormethan verdünnt und über Celite filtriert. Die organische Phase wird mit Wasser und ges. Natriumchloridlösung extrahiert, über Natriumsulfat getrocknet und im Vakuum eingeengt. Das Rohprodukt wird mit Ethylacetat / Heptan (1/2) über Kieselgel chromatographiert. Man erhält 0,72 g des gewünschten Produktes. C₁₄H₁₄O₂ (214.27) MS (ESI⁺):232.20 (M+NH₄⁺)⁺.

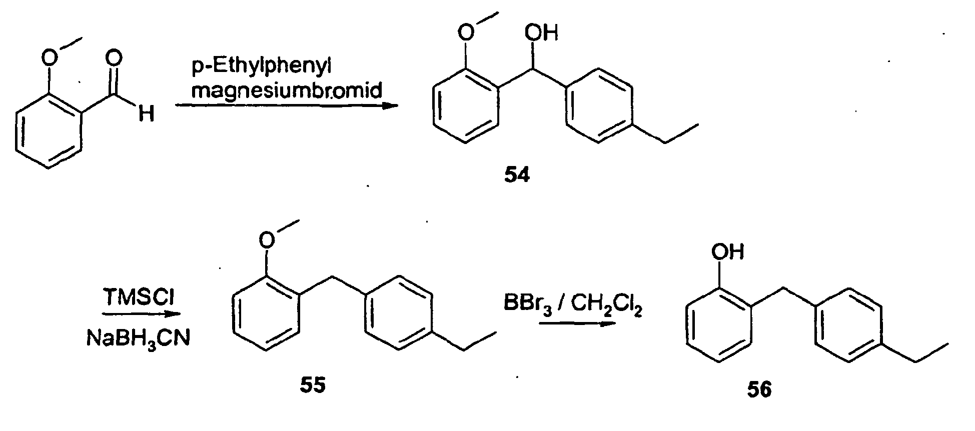

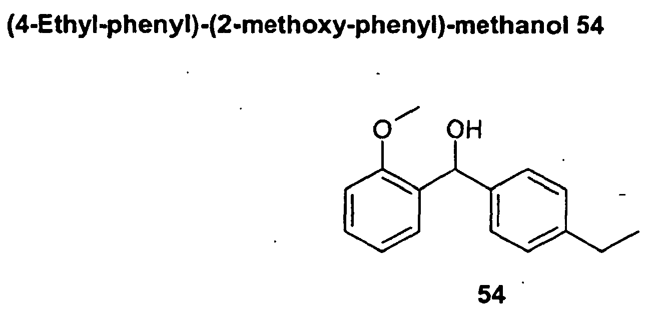

1,01 g o-Anisaldehyd werden in THF gelöst und auf 0°C gekühlt. 16,29 ml 4-Ethylphenylmagnesiumbromid (0.5M in THF) werden zum Ansatz gegeben. Die Reaktionslösung wird über Nacht bei Raumtemperatur gerührt, anschließend in 20% NH₄Cl-Lösung eingegossen und mit Ethylacetat extrahiert. Man erhält 1,92 g des Produktes, das ohne weitere Aufreinigung eingesetzt werden kann. C₁₆H₁₈O₂ (242.32) MS (ESI⁺) 225.15 (M-OH)⁺

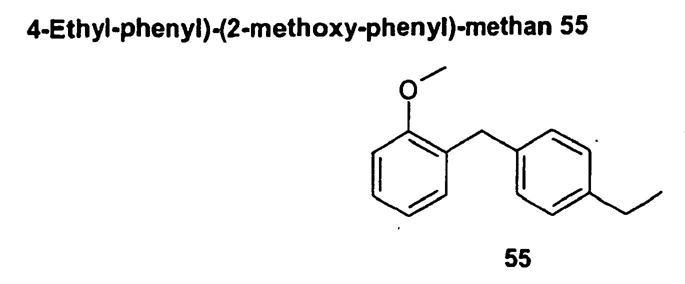

1,34 g (4-Ethyl-phenyl)-(2-methoxy-phenyl)-methanol werden in Acetonitril gelöst und auf 0°C gekühlt. 1,50g Natriumcyanoborhydrid werden zum Ansatz gegeben anschliessend werden 3,00 ml Trimethylsilylchlorid zugegeben. Der Ansatz wird über Nacht bei Raumtemperatur gerührt. Die Reaktionslösung wird über Celite filtriert und mit ges. NaCl -Lösung extrahiert. Die organische Phase wird über Natriumsulfat getrocknet und eingeengt. Das Rohprodukt wird mit Ethylacetat / Heptan (1/12) über Kieselgel chromatographiert. Man erhält 0,83g des Produktes. C₁₆H₁₈O (226.32) MS (DCI) 227.4 (M+H⁺)

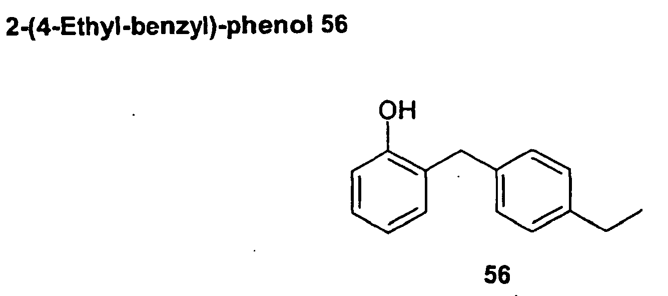

0,83g (4-Ethyl-phenyl)-(2-methoxy-phenyl)-methan 55 werden in Dichlormethan gelöst. 11,0 ml Bortribromid (1M in CH₂Cl₂) werden zum Ansatz getropft. Der Ansatz wird 5 Stunden bei Raumtemperatur.gerührt, mit Wasser versetzt und die DichlormethanPhase abgetrennt. Die wässrige Phase wird mit Ethylacetat extrahiert. Die vereinigten organischen Phasen werden mit Wasser und NaCl-Lsg. gewaschen, über Natriumsulfat getrocknet und eingeengt. Man erhält 0,77g als Rohprodukt, das chromatographisch gereinigt werden kann. C₁₅H₁₆O (212.29) MS (ESI): 235.20 (M+Na⁺)

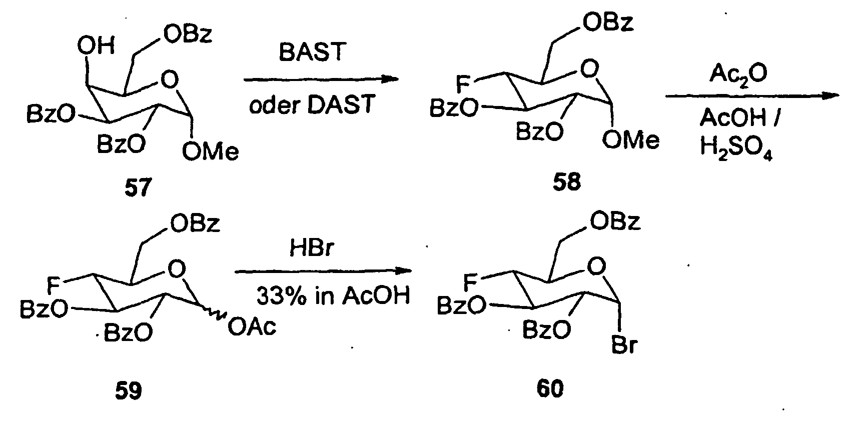

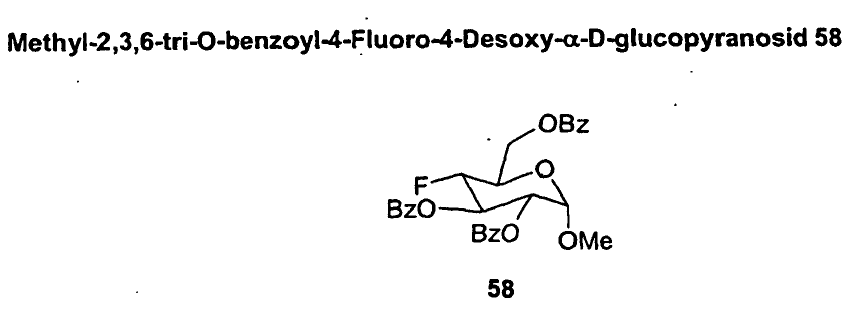

3g Methyl 2,3,6-Tri-O-benzoyl-α-D-galactopyranosid **57** (Reist et al., J.Org.Chem 1965, 30, 231.2) werden in Dichlormethan vorgelegt und auf -30°C gekühlt. Dann werden 3,06 ml [Bis(2-Methoxyethyl)Amino]Sulfurtrifluorid (BAST) zugetropft. Die Reaktionslösung wird auf Raumtemperatur erwärmt und über Nacht gerührt. Der Ansatz wird mit Dichlormethan verdünnt und die organische Phase mit H₂O, NaHCO₃₋Lsg. und gesättigter NaCl-Lsg. extrahiert. Die organische Phase wird über Na₂SO₄ getrocknet und eingeengt. Das Rohprodukt wird aus Ethylacetat und Heptan kristallisiert. Man erhält 1,95g 58 als farblosen Feststoff. C₂₈H₂₅FO₈ (508.51) MS (ESI⁺) 526.18 (M+NH₄⁺). Alternativ kann die Reaktion auch unter Verwendung von 2,8 eq. Diethylaminosulfurtrifluorid (DAST) durchgeführt werden; hierbei wird die Reaktionslösung nach erfolgter Zugabe für 18h refluxiert. Die Aufarbeitung erfolgt analog zu der oben beschriebenen.

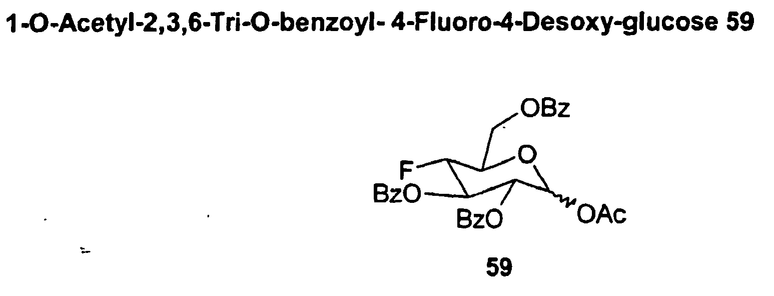

12 g Methyl-2,3,6-tri-O-benzoyl-4-Fluoro-4-Desoxy-α-D-glucopyranosid **58** werden in 150 ml Essigsäureanhydrid suspendiert. 8,4ml konz. Schwefelsäure werden mit 150 ml Eisessig gemischt und unter Eiskühlung zum Ansatz gegeben. Der Ansatz rührt bei Raumtemperatur für 60 h. Das Reaktionsgemisch wird in NaHCO₃-Lsg. gegossen und diese Lösung mit Dichlormethan extrahiert. Die organische Phase wird mit NaCl-Lsg. extrahiert, mit Na₂SO₄ getrocknet und eingeengt. Der Rückstand wird aus Ethylacetat/Heptan umkristallisiert. Man erhält 5,97 g des Produkts als farblosen Feststoff. C₂₉H₂₅FO₉ (536.52) MS (ESI⁺) 554.15 (M+NH₄⁺)

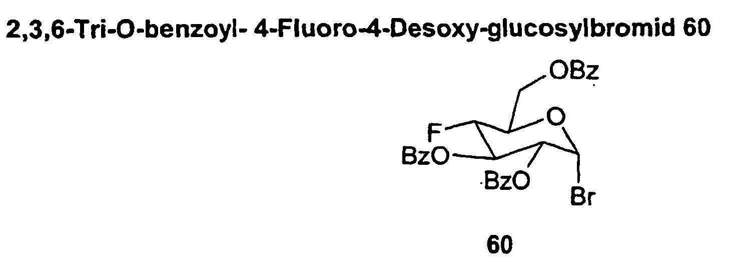

1,44g 1-O-Acetyl, 2,3,6-Tri-O-benzoyl- 4-Fluoro-4-Desoxy-glucose werden in 20ml Bromwasserstoffsäure in Eisessig (33%) gelöst und bei Raumtemperatur gerührt. Nach 5 Stunden wird der Ansatz auf Eiswasser gegeben, die wässrige Phase wird dreimal mit Dichlormethan extrahiert. Die gesammelte organische Phase wird mit gesättigter Natriumchloridlösung extrahiert, über Natriumsulfat getrocknet und zur Trockne eingeengt. Das Rohprodukt wird mit Ethylacetat/Heptan 70:30 über eine Kieselgelsäule filtriert. Man erhält 1,40g des Produkts als Feststoff. C₂₇H₂₂BrFO₇ (557.37) MS (ESI⁺) 574.05/576.05 (M+NH₄⁺)

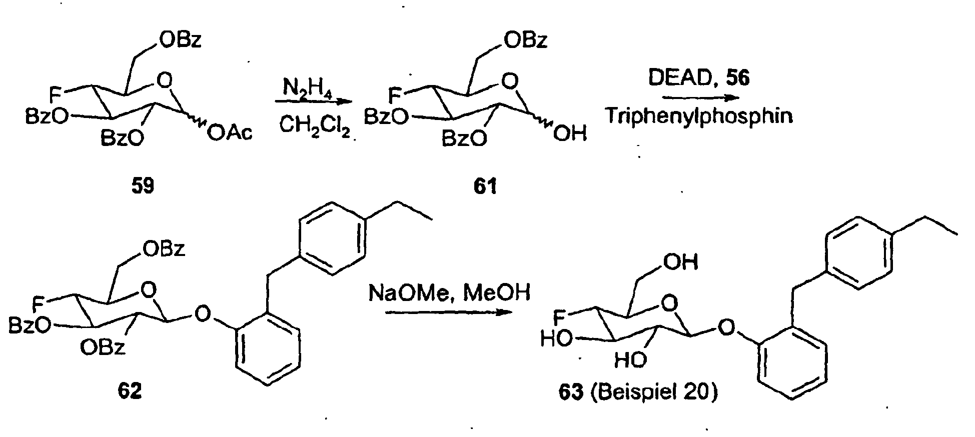

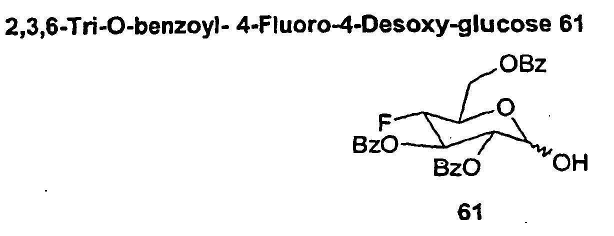

1,60 g 1-O-Acetyl-2,3,6-Tri-O-benzoyl- 4-Fluoro-4-Desoxy-glucose werden in Dichlormethan gelöst. Zu dieser Lösung werden 173µl Hydrazin Hydrat gegeben. Nach 16h wird die Reaktionslösung zwischen Dichlormethan und H₂O verteilt. Die organische Phase wird mit NaCl-Lsg. extrahiert, über Natriumsulfat getrocknet und zur Trockne eingeengt. Das Rohprodukt wird durch Säulenfiltration gereinigt. Man erhält 1,22g des gewünschten Produktes. C₂₇H₂₃FO₈ (494.48) MS (ESI⁺): 512.15 (M+NH₄⁺).

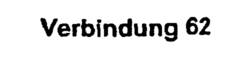

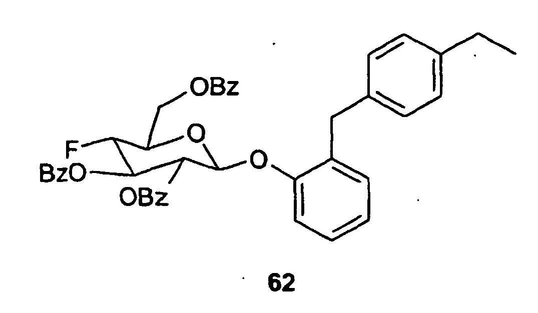

248 mg 2-(4-Ethyl-benzyl)-phenol (**56**), 550 mg 2,3,6-Tri-O-benzoyl- 4-Fluoro-4-Desoxy-glucose (**61**) und 335 mg Triphenylphosphin werden in 2 mL trockenem Dichlormethan unter Argon auf 0°C gekühlt. Langsam werden 0,193 mL Diethylazodicarboxylat zugetropft. Die Lösung wird auf Raumtemeratur gebracht und rührt über Nacht. Die Lösung wird dann mit Dichlormethan verdünnt und mit Wasser, 0,5M NaOH und ges. NaCl-Lösung extrahiert. Die organische Phase wird über Natriumsulfat getrocknet und im Vakuum eingeengt. Der Rückstand wird chromatographisch gereinigt (Heptan:Ethylacetat 3:1). Man erhält 200 mg des gewünschten Produkts. C₄₂H₃₇FO₈ (688,76) MS (ESI): 706,30 (M+NH₄)⁺.

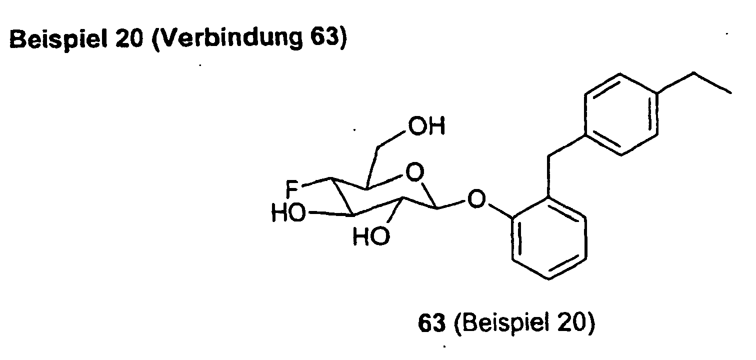

200 mg **62** werden in 10 mL abs. Methanol aufgenommen und mit 1 mL Natriummethanolat-Lösung (10 mg Natriummethanolat pro mL Methanol) versetzt. Die Lösung rührt für 8 h. Natrium wird durch Zugabe von Amberlyst-15 (H⁺-Form) entfernt, der lonentauscher wird abfiltriert und der Rückstand gründlich gewaschen. Das erhaltene Produkt wird durch Kieselgelfiltration (Dichlormethan:Methanol 96:4) gereinigt. Man erhält 56 mg des gewünschten Produktes. C₂₁H₂₅FO₅ (376,43) MS (ESI): 394,25 (M+NH₄⁺)

Folgende Beispiele werden auf analoge Weise wie Beispiel 20 unter Verwendung der entsprechenden Aglycone hergestellt:

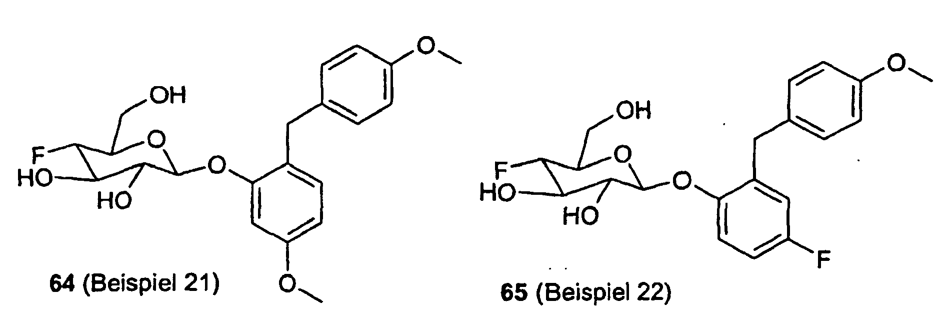

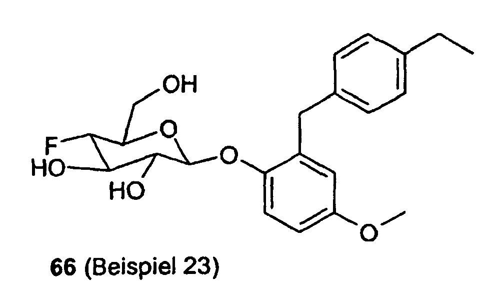

Die entsprechenden Aglycone können beispielsweise nach den für Verbindung **7** oder **56** beschriebenen Verfahren erhalten werden.

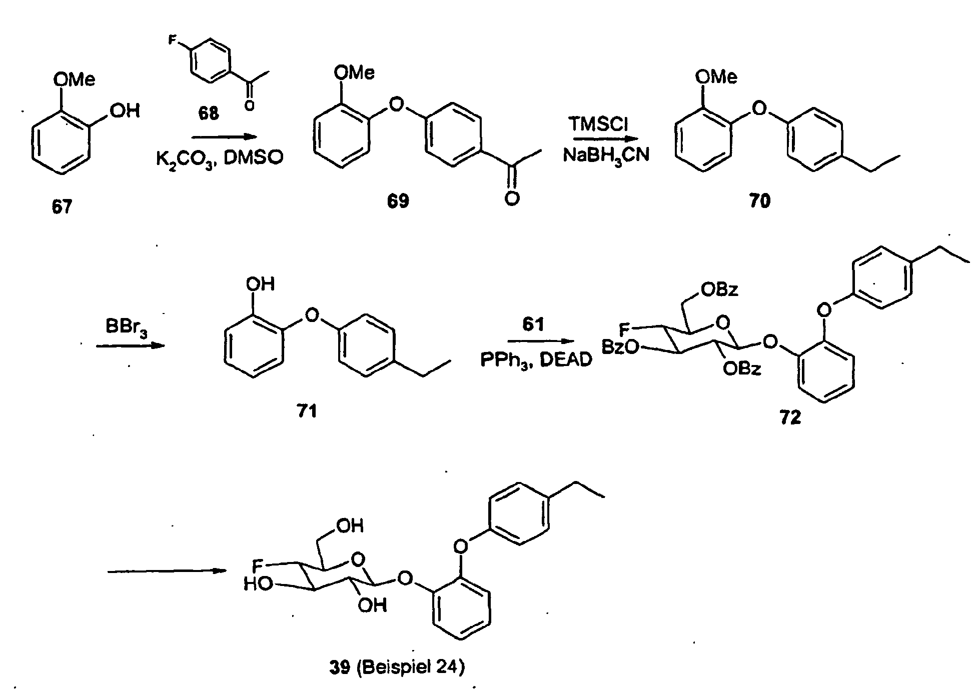

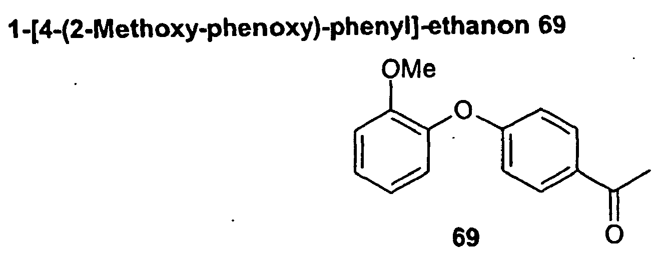

0,15 mL Guaiacol **67** , 167 mg 4-Fluoracetophenon **68**, 335 mg Kaliumcarbonat werden in 5 mL Dimethylsulfoxid in einer Mikrowelle für 10 min. auf 170°C erhitzt. Die Reaktionslösung wird in Wasser eingegossen und die Emulsion wird dreimal mit Methyl tert. Butylether extrahiert. Die kombinierte organische Phse wird zweimal mit 1 N NaOH und einmal mit ges. NaCl-Lösung extrahiert, getrocknet und im Vakuum eingeengt. Man erhält 240 mg des gewünschten Produktes. C₁₅H₁₄O₃ (242,28) MS (ESI): 215,10 (M+H⁺).

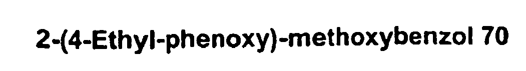

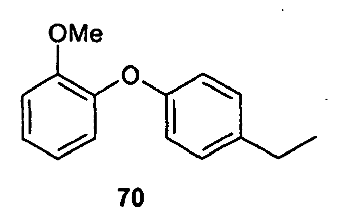

960 mg 1-[4-(2-Methoxy-phenoxy)-phenyl]-ethanon **69** werden in 20 mL Acetonitril gelöst, im Eisbad gekühlt und mit 1,05 g Natriumcyanoborhydrid und 2,01 mL Trimethylsilylchlorid versetzt. Nach 1 h wird mit Dichlormethan verdünnt, über Celite abfiltriert und die organische Phase wird mit Natriumchloridlösung extrahiert, über Natriumsulfat getrocknet und eingeengt. Der Rückstand wird chromatographisch gereinigt (Heptan:Ethylacetat 7:1). Man erhält 710 mg des gewünschten Produkts. C₁₅H₁₆O₂ (228,29) MS (ESI): 246,20 (M+NH₄⁺).

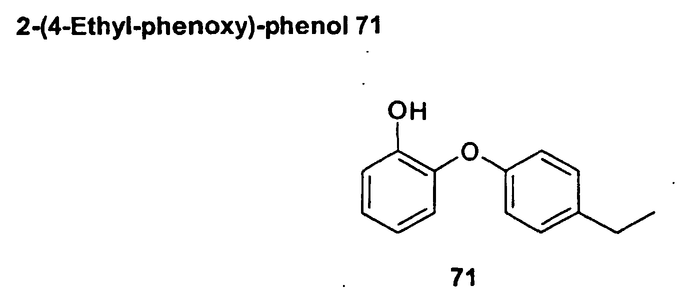

710 mg 2-(4-Ethyl-phenoxy)-methoxybenzol 70 werden in 5 mL abs. Dichlormethan gelöst. 0,6 mL Bortribromid (1M in Dichlormethan) wird zugetropft und die Lösung rührt für 6h. Es wird weiter BBr₃ zugegeben und gerührt, bis nach LCMS die Umsetzung nahezu vollständig ist. Die Lösung wird in Eiswasser gegeben, die organische Phase abgetrennt und die wäßrige Phase wird dreimal mit Dichlormethan extrahiert. Die kombinierte organische Phase wird getrocknet, zur Trockne eingeengt und chromatographisch gereinigt. Man erhält 450 mg des gewünschten Produkts. C₁₄H₁₄O₂ (214,27) MS (ESI): 215,10 (M+H⁺).

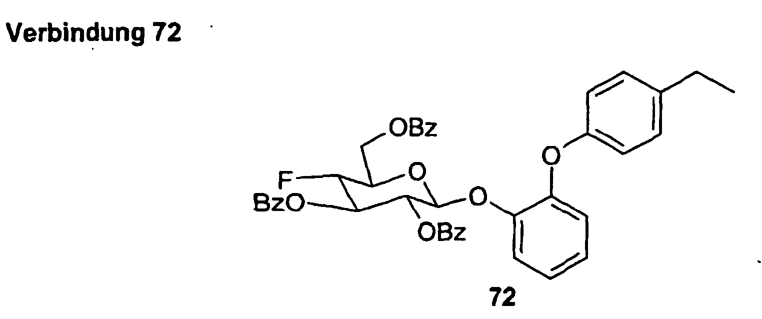

Verbindung **61** (466 mg) und Phenol **71** (242 mg) werden analog zur Synthese von Verbindung **62** zur Reaktion gebracht. Das erhaltene Produkt kann säulenchromatographisch gereinigt werden (Heptan:Ethylacetat 4:1). Man erhält 240 mg des gewünschten Produkts. C₄₁H₃₅FO₉ (690,73) MS (ESI): 708,25 (M+NH₄⁺).

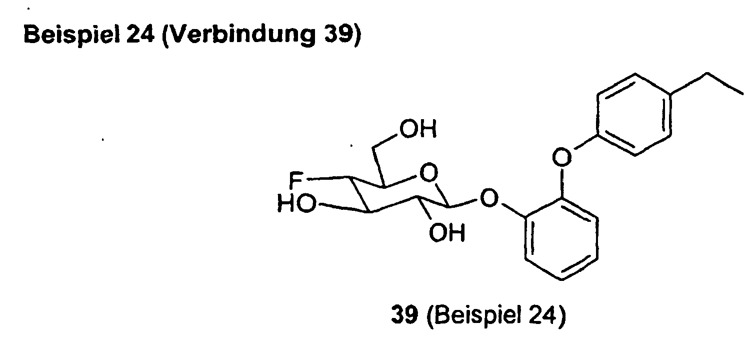

230 mg von Verbindung **72** werden analog zur Freisetzung von Beispiel 20 mit Natriummethanolat zur Reaktion gebracht. Die Verbindung kann durch Kieslgelchromatographie (Dichlormethan:Methanol 96:4) gereinigt werden. Man erhält 119 mg des gewünschten Produkts. C₂₀H₂₃FO₆ (378,40) MS (ESI): 396,15 (M+NH₄⁺).

## Patentansprüche

1. Verbindungen der Formel I, 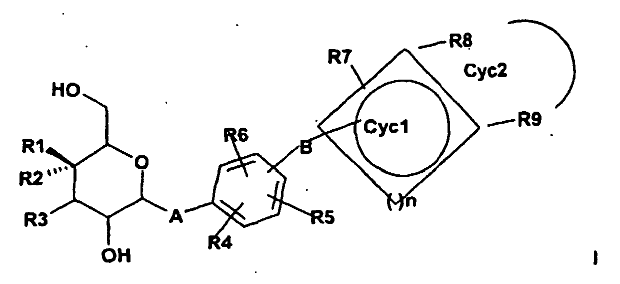 worin bedeuten
R1, R2 OH, F oder H oder R1 und R2 = F, wobei die drei Kombinationen R1=F, R2=OH und R1=OH, R2=F und R1, R2=OH ausgenommen sind;
R3 OH oder F, wobei mindestens einer der Reste R1, R2, R3 F sein muss;
A O, NH, CH₂, S oder eine Bindung;
R4,R5,R6 Wasserstoff, F, Cl, Br, J, OH, NO₂, CN, COOH, CO(C₁-C₆)-Alkyl, COO(C₁-C₆)-Alkyl, CONH₂, CONH(C₁-C₆)-Alkyl, CON[(C₁-C₆)-Alkyl]₂, (C₁₋C₆)-Alkyl, (C₂-C₆)-Alkenyl, (C₂-C₆)-Alkinyl, (C₁-C₆)-Alkoxy, HO-(C₁-C₆)-Alkyl, (C₁-C₆)-Alkyl-O-(C₁-C₆)-alkyl, Phenyl, Benzyl, wobei in den Alkyl-, Alkoxy-, Alkenyl- und Alkinylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂ , S-(C₁-C₆)-Alkyl, S-(CH₂)ₒ-Phenyl, SO-(C₁-C₆)-Alkyl, SO-(CH₂)ₒ-Phenyl, SO₂-(C₁-C₆)-Alkyl, SO₂-(CH₂)ₒ-Phenyl, wobei o = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₒ-Phenyl, wobei o = 0 - 6 sein kann, wobei der Phenylring ein bis 3-fach substituiert sein kann mit F, Cl, Br, J, OH, CF₃, NO₂, CN, OCF₃, O-(C₁-C₆-Alkyl, (C₁-C₆)-Alkyl, NH₂, NH(C₁-C₆)-Alkyl, N((C₁-C₆)-Alkyl)₂, SO₂₋CH₃, COOH, COO-(C₁-C₆)-Alkyl, CONH₂;
B (C₀-C₁₅)-Alkandiyl, wobei ein oder mehrere C-Atome des Alkandiyl-Rests unabhängig voneinander durch -O-, -(C=O)-, -CH=CH-, -C≡C-, -S-, -CH(OH)-, -CHF-, -CF₂-, -(S=O)-, -(SO₂)-, -N((C₁-C₆)-Alkyl)-, -N((C₁-C₆)-Alkyl-Phenyl)- oder -NH- ersetzt sein können;
n eine Zahl von 0 bis 4;
Cyc1 ein 3 bis 7 gliedriger gesättigter, teilweise gesättigter oder ungesättigter Ring, wobei 1 C-Atom durch O, N oder S ersetzt sein kann;
R7, R8, R9 Wasserstoff, F, Cl, Br, J, OH, CF₃, NO₂, CN, COOH, COO(C₁-C₆)-Alkyl, CO(C₁-C₄)-Alkyl, CONH₂, CONH(C₁-C₆)-Alkyl, CON[(C₁-C₆)-Alkyl]_{2,} (C₁₋C₆)-Alkyl, (C₂-C₆)-Alkenyl, (C₂-C₆)-Alkinyl, (C₁-C₈)-Alkoxy, (C₁-C₆)-Alkyl-OH, (C₁-C₆)-Alkyl-O-(C₁-C₆)-alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂, S-(C₁-C₆)-Alkyl, S-(CH₂)ₒ-Phenyl, SO-(C₁-C₆)-Alkyl, SO-(CH₂)ₒ-Phenyl, SO₂-(C₁-C₆)-Alkyl, SO₂-(CH₂)ₒ-Phenyl, wobei o = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₒ-Phenyl, wobei o = 0 - 6 sein kann, wobei der Phenylring ein bis 3-fach substituiert sein kann mit F, Cl, Br, J, OH, CF₃, NO₂, CN, OCF₃, (C₁₋C₈)-Alkoxy, (C₁-C₆)-Alkyl, NH₂, NH(C₁-C₆)-Alkyl, N((C₁-C₆)-Alkyl)₂, SO₂₋CH₃, COOH, COO-(C₁-C₆)-Alkyl, CONH₂;
oder
R8 und R9 gemeinsam mit den sie tragenden C-Atomen ein 5 bis 7 gliedrigen, gesättigter, teilweise oder vollständig ungesättigter Ring Cyc2, wobei 1 oder 2 C-Atom(e) des Ringes auch durch N, O oder S ersetzt sein können und Cyc2 gegebenenfalls durch (C₁-C₆)-Alkyl, (C₂-C₅)-Alkenyl, (C₂-C₅)-Alkinyl, wobei jeweils eine CH₂-Gruppe durch O ersetzt sein kann, oder durch H, F, Cl, OH, CF₃, NO₂, CN, COO(C₁-C₄)-Alkyl, CONH₂, CONH(C₁₋C₄)-Alkyl, OCF₃ substituiert sein kann;
sowie deren pharmazeutisch verträglichen Salze.

2. Verbindungen der Formel I gemäß Anspruch 1, worin bedeuten
R1, R2 OH, F oder H oder R1 und R2 = F, wobei einer der Reste R1 oder R2 F sein muss und die drei Kombinationen R1=F, R2=OH und R1=OH, R2=F und R1,R2=OH ausgenommen sind;
R3 OH;
A O oder NH;
R4,R5,R6 Wasserstoff, F, Cl, Br, J, OH, CF₃, NO₂, CN, COOH, CO(C₁-C₆)-Alkyl, COO(C₁-C₆)-Alkyl, CONH₂, CONH(C₁-C₆)-Alkyl, CON[(C₁-C₆)-Alkyl]₂, (C₁₋C₆)-Alkyl, (C₂-C₆)-Alkenyl, (C₂-C₆)-Alkinyl, (C₁-C₆)-Alkoxy, HO-(C₁-C₆)-Alkyl, (C₁-C₆)-Alkyl-O-(C₁-C₆)-alkyl, Phenyl, Benzyl, SO-(C₁-C₆)-Alkyl, wobei in den Alkyl-, Alkoxy-, Alkenyl- und Alkinylresten ein, mehrere, oder alle Wasserstoff(e) durch Fluor ersetzt sein können;
B (C₀-C₁₅)-Alkandiyl, wobei ein oder mehrere C-Atom(e) des Alkandiyl-Rests unabhängig voneinander durch -O-, -(C=O)-, -CH=CH-, -C≡C-, -S-, -CH(OH)-, -CHF-, -CF₂-, -(S=O)-, -(SO₂)-, -N((C₁-C₆)-Alkyl)-, -N((C₁-C₆)-Alkyl-Phenyl)- oder -NH- ersetzt sein können;
n eine Zahl 0 bis 4;
Cyc1 ein 3 bis 7 gliedriger gesättigter, teilweise gesättigter oder ungesättigter Ring, wobei 1 C-Atom durch O, N oder S ersetzt sein kann;
R7, R8, R9 Wasserstoff, F, Cl, Br, J, OH, CF₃, NO₂, CN, COOH, COO(C₁-C₆)-Alkyl, CO(C₁-C₄)-Alkyl, CONH₂, CONH(C₁-C₆)-Alkyl, CON[(C₁-C₆)-Alkyl]₂, (C₁₋C₆)-Alkyl, (C₂-C₆)-Alkenyl, (C₂-C₆)-Alkinyl, (C₁-C₈)-Alkoxy, (C₁-C₆)-Alkyl-OH, (C₁-C₆)-Alkyl-O-(C₁-C₆)-alkyl, SO-(C₁-C₆)-Alkyl, wobei in den Alkylresten ein, mehrere, oder alle Wasserstoff(e) durch Fluor ersetzt sein können;
oder
R8 und R9 gemeinsam mit den sie tragenden C-Atomen ein 5 bis 7 gliedrigen, gesättigter, teilweise oder vollständig ungesättigter Ring Cyc2, wobei 1 oder 2 C-Atom(e) des Ringes auch durch N, O oder S ersetzt sein können und Cyc2 gegebenenfalls durch (C₁-C₆)-Alkyl, (C₂-C₅)-Alkenyl, (C₂-C₅)-Alkinyl, wobei jeweils eine CH₂-Gruppe durch O ersetzt sein kann, oder durch H, F, Cl, OH, CF₃, NO₂, CN, COO(C₁-C₄)-Alkyl, CONH₂, CONH(C₁₋C₄)-Alkyl, OCF₃ substituiert sein kann.

3. Verbindungen der Formel 1, gemäß den Ansprüchen 1 oder 2, in denen die Zucker-Reste beta(β)-verknüpft sind oder solche, in denen der B-Substituent am Phenylring in ortho-Position zu dem A-Substituenten angeordnet ist.

4. Verbindungen der Formel I gemäß den Ansprüchen 1 bis 3, worin
R1, R2 OH, F oder H oder R1 und R2 = F, wobei einer der Reste R1 oder R2 F sein muss und die drei Kombinationen R1=F, R2=OH und R1=OH, R2=F und R1,R2=OH ausgenommen sind ;
R3 OH;
A O;
R4, R5, R6 Wasserstoff, OH, (C₁-C₆)-Alkyl, (C₁-C₄)-Alkoxy, HO-(C₁-C₄)-Alkyl, (C₁₋C₄)-Alkyl-O-(C₁-C₄)-alkyl, F, Cl, Br, J, CF₃, OCF₃, OCH₂CF₃ (C₁-C₄)-Alkyl-CF₂-, Phenyl, Benzyl, (C₂-C₄)-Alkenyl, (C₂-C₄)-Alkinyl, COO(C₁-C₄)-Alkyl;
B (C₁-C₄-Alkandiyl, wobei eine CH₂-Gruppe auch ersetzt sein kann durch -(C=O)-, -CH(OH)-, -CO-NH-, -CO-N(C₁-C₆)-Alkyl-, -CHF-, -CF₂-, -O-, -NH-;
n eine Zahl 2 oder 3;
Cyc1 ungesättigter 5- oder 6-gliedriger Ring, wobei 1 C-Atom durch O, N oder S ersetzt sein kann;
R7,R8,R9 Wasserstoff, (C₁-C₆)-Alkyl, (C₁-C₈)-Alkoxy, OCF₃, OCH₂CF₃ , OH , (C₁₋C₄)-Alkyl-OH, (C₁-C₄)-Alkyl-O-(C₁-C₄)-alkyl, F, Cl, Br oder
R8 und R9 gemeinsam -CH=CH-O-, -CH₂-CH₂-O-,-CH=CH-S-, -CH=CH-CH=CH-, -O-(CH₂)ₚ-O-, mit p = 1 oder 2 und
R7 Methyl, Ethyl, OMe, F, Cl, Br oder Wasserstoff bedeuten.

5. Verbindungen der Formel I gemäß den Ansprüchen 1 bis 4, worin
R1 Fund R2 H oder
R1 H und R2 F oder
R1 F und R2 F;
R3 OH;
A O;
R4, R5, R6 Wasserstoff, OH, (C₁-C₄)-Alkoxy , CF₃, (C₁-C₄)-Alkyl, F, Cl, Br, J,
B -CH₂-, -C₂H₄-, -C₃H₆, -CH(OH)-, -(C=O)-, -CO-NH-CH₂- oder -CO-CH₂₋CH₂-, -O-, -NH-;
n eine Zahl 2 oder 3;
Cyc1 ungesättigter 6-gliedriger Ring, wobei 1 C-Atom durch N ersetzt sein kann oder ungesättigter 5-gliedriger Ring, wobei 1 C-Atom durch S ersetzt sein kann;
R7,R8,R9 Wasserstoff, OH, (C₁-C₄)-Alkyl, (C₁-C₇)-Alkoxy, OCF₃, Halogen oder
R8 und R9 gemeinsam -CH=CH-O-, -CH₂CH₂-O-, -CH=CH-CH=CH- -O-(CH₂)ₚ-O-, mit p = 1 oder 2, und
R7 Methyl, Ethyl, OMe, F, Cl, Br, Wasserstoff bedeuten.

6. Verbindungen der Formel la 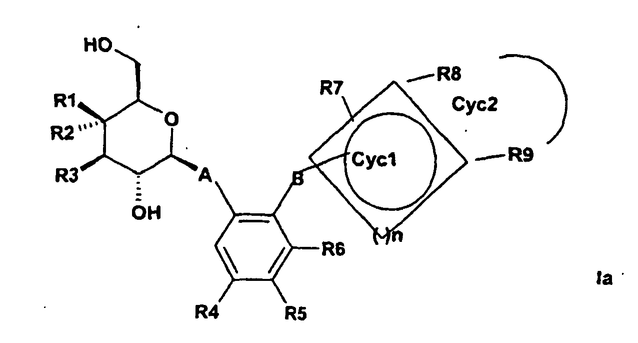 gemäßen Ansprüchen 1 bis 5, worin
R1 F und R2 H oder
R1 H und R2 F oder
R1 F und R2 F;
R3 OH;
A O;
R4 Wasserstoff, (C₁-C₄)-Alkyl, (C₁-C₄)-Alkoxy oder OH;
R5 Wasserstoff, F, Methoxy oder Ethoxy;
R6 Wasserstoff oder OH;
B -CH₂-, -CO-NH-CH₂-; -O- oder -CO-CH₂-CH₂-;
Cyc1 Phenyl oder Thiophen;
R7,R8,R9 Wasserstoff, OH, Cl, OCF₃, (C₁-C₄)-Alkyl oder(C₁-C₄)-Alkoxy; oder
R8 und R9 gemeinsam -CH=CH-O-, -CH=CH-CH=CH- oder -CH₂-CH₂-O- und
R7 Wasserstoff bedeuten.

7. Verbindungen der Formel Ib 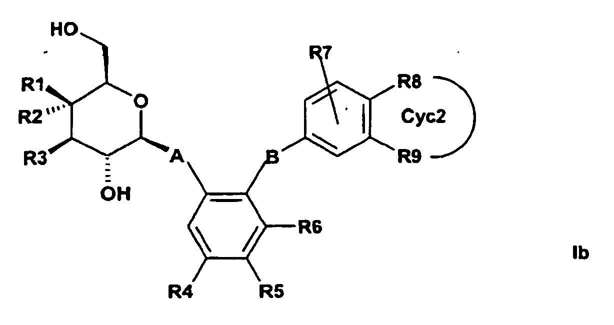 gemäß den Ansprüchen 1 bis 6, worin
R1 F und R2 H oder
R1 H und R2 F oder
R1 F und R2 F;
R3 OH;
A O;
R4 Wasserstoff, Methyl, Methoxy oder OH;
R5 Wasserstoff, F oder Methoxy;
R6 Wasserstoff oder OH;
B -CH₂-, -CO-NH-CH₂-; -O- oder -CO-CH₂-CH₂-;
Cyc1 Phenyl;
R7 Wasserstoff;
R8 Wasserstoff, OH, Ethyl, Cl, OCF₃ oder Methoxy;
R9 Wasserstoff; oder
R8 und R9 gemeinsam -CH=CH-O- oder -CH₂-CH₂-O- bedeuten.

8. Arzneimittel enthaltend eine oder mehrere der Verbindungen gemäß einem oder mehreren der Ansprüche 1 bis 7.

9. Arzneimittel enthaltend eine oder mehrere der Verbindungen gemäß einem oder mehreren der Ansprüche 1 bis 7 und einen oder mehrere Blutzucker senkende Wirkstoffe.

10. Verwendung der Verbindungen gemäß einem oder mehreren der Ansprüche 1 bis 7 zur Herstellung eines Medikamentes zur Behandlung des Typ 1 und Typ 2 Diabetes.

11. Verwendung der Verbindungen gemäß einem oder mehreren der Ansprüche 1 bis 7 zur Herstellung eines Medikamentes zur Blutzuckersenkung.

12. Verwendung der Verbindungen gemäß einem oder mehreren der Ansprüche 1 bis 7 in Kombination mit mindestens einem weiteren Blutzucker senkenden Wirkstoff zur Herstellung eines Medikamentes zur Behandlung des Typ 1 und Typ 2 Diabetes.

13. Verwendung der Verbindungen gemäß einem oder mehreren der Ansprüche 1 bis 7 in Kombination mit mindestens einem weiteren Blutzucker senkenden Wirkstoff zur Herstellung eines Medikamentes zur Blutzuckersenkung.

14. Verfahren zur Herstellung eines Arzneimittels enthaltend eine oder mehrere der Verbindungen gemäß einem oder mehreren der Ansprüche 1 bis 7, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I 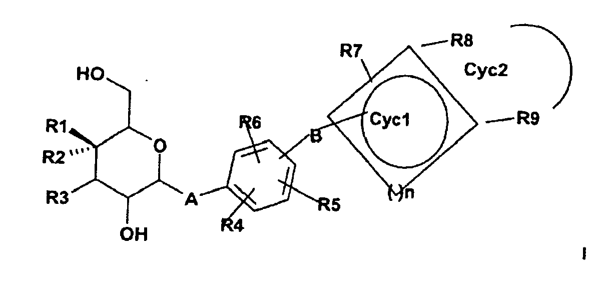 in which the meanings are
R1, R2 OH, F or H or R1 and R2 = F, excluding the three combinations R1 = F, R2 = OH and R1 = OH, R2 = F and R1, R2 =OH;
R3 OH or F, where at least one of the R1, R2, R3 radicals must be F;
A O, NH, CH₂, S or a bond;
R4, R5, R6 hydrogen, F, Cl, Br, I, OH, NO₂, CN, COOH, CO(C₁-C₆)-alkyl, COO(C₁-C₆)-alkyl, CONH₂, CONH(C₁-C₆) -alkyl, CON [(C₁-C₆)-alkyl]₂, (C₁-C₆)-alkyl, (C₂-C₆) -alkenyl, (C₂-C₆)-alkynyl, (C₁-C₆)-alkoxy, HO(C₁-C₆)-alkyl, (C₁-C₆) -alkyl-O-(C₁-C₆)-alkyl, phenyl, benzyl, it being possible for one, more than one or all hydrogen(s) in the alkyl, alkoxy, alkenyl and alkynyl radicals to be replaced by fluorine;
SO₂-NH₂, SO₂NH (C₁-C₆) -alkyl, SO₂N [(C₁-C₆)-alkyl]₂, S- (C₁-C₆) -alkyl, S-(CH₂)ₒ-phenyl, SO-(C₁-C₆)-alkyl, SO-(CH₂)ₒ-Phenyl, SO₂- (C₁-C₆) -alkyl, SO₂- (CH₂)ₒ-phenyl, where o can be 0-6, and the phenyl radical may be substituted up to twice by F, Cl, Br, OH, CF₃, NO₂, CN, OCF₃, O-(C₁-C₆)-alkyl, (C₁-C₆)-alkyl, NH₂;
NH₂, NH- (C₁-C₆) -alkyl, N((C₁-C₆) -alkyl)₂, NH(C₁-C₇)-acyl, phenyl, O-(CH₂)ₒ-phenyl, where o can be 0-6, where the phenyl ring may be substituted one to 3 times by F, Cl, Br, I, OH, CF₃, NO₂, CN, OCF₃, O-(C₁-C₆)-alkyl, (C₁-C₆) -alkyl, NH₂, NH(C₁-C₆)-alkyl, N(C₁-C₆) -alkyl)₂, SO₂-CH₃, COOH, COO-(C₁-C₆)-alkyl, CONH₂;
B (C₀-C₁₅)-alkanediyl, it being possible for one or more C atoms in the alkanediyl radical to be replaced independently of one another by -O-, -(C=O)-, -CH=CH-, -C≡C-, -S-, -CH(OH)-, -CHF-, -CF₂-, - (S=O) -, - (SO₂) -, -N( (C₁-C₆) -alkyl) -, -N( (C₁-C₆) -alkyl-phenyl)- or -NH-;
n a number from 0 to 4;
Cyc1 a 3 to 7 membered saturated, partially saturated or unsaturated ring, where 1 C atom may be replaced by O, N or S;
R7, R8, R9 hydrogen, F, Cl, Br, I, OH, CF₃, NO₂, CN, COOH, COO(C₁-C₆)-alkyl, CO(C₁-C₄)-alkyl, CONH₂, CONH (C₁-C₆) -alkyl, CON [(C₁-C₆)-alkyl]₂, (C₁-C₆)-alkyl, (C₂-C₆) -alkenyl, (C₂-C₆)-alkynyl, (C₁-C₈)-alkoxy, (C₁-C₆)-alkyl-OH, (C₁-C₆)-alkyl-O-(C₁-C₆)-alkyl, it being possible for one, more than one or all hydrogen(s) in the alkyl radicals to be replaced by fluorine;
SO₂-NH₂, SO₂NH (C₁-C₆) -alkyl, SO₂N [(C₁-C₆)-alkyl]₂, S- (C₁-C₆) -alkyl, S-(CH₂)ₒ-phenyl, SO- (C₁-C₆) -alkyl, SO- (CH₂)ₒ-phenyl, SO₂- (C₁-C₆) -alkyl, SO₂- (CH₂)ₒ-phenyl, where o can be 0-6, and the phenyl radical may be substituted up to twice by F, Cl, Br, OH, CF₃, NO₂, CN, OCF₃, O- (C₁-C₆) -alkyl, (C₁-C₆) -alkyl, NH₂;
NH₂, NH-(C₁-C₆-alkyl, N((C₁-C₆)-alkyl)₂, NH(C₁-C₇)-acyl, phenyl, O-(CH₂)ₒ-phenyl, where o can be 0-6, where the phenyl ring may be substituted one to 3 times by F, Cl, Br, I, OH, CF₃, NO₂, CN, OCF₃, (C₁-C₈)-alkoxy, (C₁-C₆)-alkyl, NH₂, NH(C₁-C₆) -alkyl, N ((C₁-C₆) -alkyl)₂, SO₂-CH₃, COOH, COO-(C₁-C₆)-alkyl, CONH₂;
or
R8 and R9 together with the C atoms carrying them a 5 to 7 membered, saturated, partially or completely unsaturated ring Cyc2, it being possible for 1 or 2 C atom(s) in the ring also to be replaced by N, O or S, and Cyc2 may optionally be substituted by (C₁-C₆)-alkyl, (C₂-C₅)-alkenyl, (C₂-C₅)-alkynyl, where in each case one CH₂ group may be replaced by O, or substituted by H, F, Cl, OH, CF₃, NO₂, CN, COO-(C₁-C₄)-alkyl, CONH₂, CONH(C₁-C₄)-alkyl, OCF₃;
and the pharmaceutically acceptable salts thereof.

2. A compound of the formula I as claimed in claim 1, in which the meanings are
R1, R2 OH, F or H or R1 and R2 = F, where one of the radicals R1 or R2 must be F, excluding the three combinations R1 = F, R2 =OH and R1 = OH, R2 = F and R1, R2 = OH;
R3 OH;
A O or NH;
R4, R5, R6 hydrogen, F, Cl, Br, I, OH, CF₃, NO₂, CN, COOH, CO(C₁-C₆)-alkyl, COO(C₁-C₆)-alkyl, CONH₂, CONH (C₁-C₆) -alkyl, CON[(C₁-C₆)-alkyl]₂, (C₁-C₆) -alkyl, (C₂-C₆) -alkenyl, (C₂-C₆) -alkynyl, (C₁-C₆) -alkoxy, HO (C₁-C₆) -alkyl, (C₁-C₆) -alkyl-O- (C₁-C₆)-alkyl, phenyl, benzyl, SO-(C₁-C₆)-alkyl, it being possible for one, more than one or all hydrogen(s) in the alkyl, alkoxy, alkenyl and alkynyl radicals to be replaced by fluorine;
B (C₀-C₁₅) -alkanediyl, where one or more C atom(s) in the alkanediyl radical may be replaced independently of one another by -O-, -(C=O)-, -CH=CH-, -C≡C-, -S-, -CH(OH)-, -CHF-, -CF₂-, - (S=O)-, -(SO₂)-, -N((C₁-C₆) -alkyl) -, -N((C₁-C₆) -alkyl-phenyl)- or -NH-;
n a number 0 to 4;
Cyc1 a 3 to 7 membered saturated, partially saturated or unsaturated ring, where 1 C atom may be replaced by O, N or S;
R7, R8, R9 hydrogen, F, Cl, Br, I, OH, CF₃, NO₂, CN, COOH, COO(C₁-C₆)-alkyl, CO (C₁-C₄)-alkyl, CONH₂, CONH (C₁-C₆) -alkyl, CON [(C₁- C₆)-alkyl]₂, (C₁-C₆)-alkyl, (C₂-C₆)-alkenyl, (C₂-C₆)-alkynyl, (C₁-C₈)-alkoxy, (C₁-C₆)-alkyl-OH, (C₁-C₆)-alkyl-O-(C₁-C₆) -alkyl, SO-(C₁-C₆)-alkyl, it being possible for one, more than one or all hydrogen(s) in the alkyl radicals to be replaced by fluorine;
or
R8 and R9 together with the C atoms carrying them a 5 to 7 membered, saturated, partially or completely unsaturated ring Cyc2, where 1 or 2 C atom(s) in the ring may also be replaced by N, O or S, and Cyc2 may optionally be substituted by (C₁-C₆)-alkyl, (C₂-C₅)-alkenyl, (C₂-C₅)-alkynyl, where in each case one CH₂ group may be replaced by O, or substituted by H, F, Cl, OH, CF₃, NO₂, CN, COO (C₁-C₄) -alkyl, CONH₂. CONH(C₁-C₄) -alkyl, OCF₃.

3. A compound of the formula I as claimed in claims 1 or 2, in which the sugar residues are beta(β)-linked, or one in which the B substituent on the phenyl ring is disposed in the position ortho to the A substituent.

4. A compound of the formula I as claimed in claims 1 to 3, in which
R1, R2 are OH, F or H or R1 and R2 = F, where one of the radicals R1 or R2 must be F, excluding the three combinations R1 = F, R2 = OH and R1 = OH, R2 = F and R1, R2 = OH;
R3 is OH;
A is O;
R4, R5, R6 are hydrogen, OH, (C₁-C₆)-alkyl, (C₁-C₄)-alkoxy, HO- (C₁-C₄) -alkyl, (C₁-C₄) -alkyl-O-(C₁-C₄)-alkyl, F, Cl, Br, I, CF₃, OCF₃, OCH₂CF₃, (C₁-C₄)-alkyl-CF₂-, phenyl, benzyl, (C₂-C₄)-alkenyl, (C₂-C₄)-alkynyl, COO(C₁-C₄)-alkyl;
B is (C₁-C₄) -alkanediyl, where one CH₂ group may also be replaced by - (C=O)-, -CH(OH)-, -CO-NH-, -CO-N (C₁-C₆) -alkyl-, -CHF-, -CF₂-, -O-, -NH-;
n is a number 2 or 3;
Cyc1 is unsaturated 5- or 6-membered ring, where 1 C atom may be replaced by O, N or S;
R7, R8, R9 are hydrogen, (C₁-C₆)-alkyl, (C₁-C₈)-alkoxy, OCF₃, OCH₂CF₃, OH, (C₁-C₄)-alkyl-OH, (C₁-C₄) -alkyl-O- (C₁-C₄) -alkyl, F, Cl, Br or
R8 and R9 together are -CH=CH-O-, -CH₂-CH₂-O-, -CH=CH-S-, -CH=CH-CH=CH-, -O-(CH₂)ₚ-O-, with p = 1 or 2, and
R7 is methyl, ethyl, OMe, F, Cl, Br or hydrogen.

5. A compound of the formula I as claimed in claims 1 to 4, in which
R1 is F and R2 is H or
R1 is H and R2 is F or
R1 is F and R2 is F;
R3 is OH;
A is O;
R4, R5, R6 are hydrogen, OH, (C₁-C₄)-alkoxy, CF₃, (C₁-C₄)-alkyl, F, Cl, Br, I,
B is -CH₂-, -C₂H₄-, -C₃H₆-, -CH(OH)-, (C=O)-, -CO-NH-CH₂- or -CO-CH₂-CH₂-, -O-, -NH-;
n is a number 2 or 3;
Cyc1 is unsaturated 6-membered ring, where 1 C atom may be replaced by N, or unsaturated 5-membered ring, where 1 C atom may be replaced by S;
R7, R8, R9 are hydrogen, OH, (C₁-C₄) -alkyl, (C₁-C₇)-alkoxy, OCF₃, halogen or
R8 and R9 together are -CH=CH-O-, -CH₂-CH₂-O-, -CH=CH-CH=CH-, -O-(CH₂)ₚ-O-, with p = 1 or 2, and
R7 is methyl, ethyl, OMe, F, Cl, Br, hydrogen.

6. A compound of the formula Ia 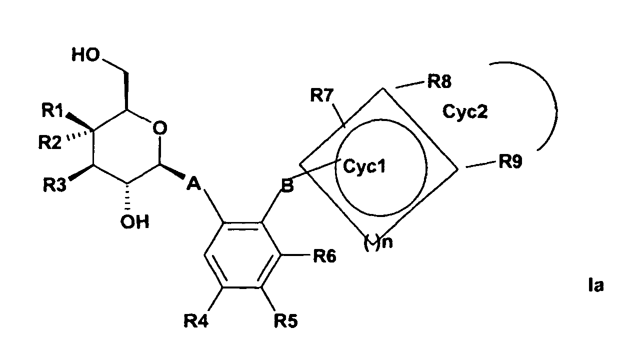 as claimed in claims 1 to 5, in which
R1 is F and R2 is H or
R1 is H and R2 is F or
R1 is F and R2 is F;
R3 is OH;
A is O;
R4 is hydrogen, (C₁-C₄)-alkyl, (C₁-C₄) -alkoxy or OH;
R5 is hydrogen, F, methoxy or ethoxy;
R6 is hydrogen or OH;
B is -CH₂-, -CO-NH-CH₂-, -O- or -CO-CH₂-CH₂-;
Cyc1 is phenyl or thiophene;
R7, R8, R9 are hydrogen, OH, Cl, OCF₃, (C₁-C₄) -alkyl or (C₁-C₄)-alkoxy; or
R8 and R9 together are -CH=CH-O-, -CH=CH-CH=CH- or -CH₂-CH₂-O- and
R7 is hydrogen.

7. A compound of the formula Ib 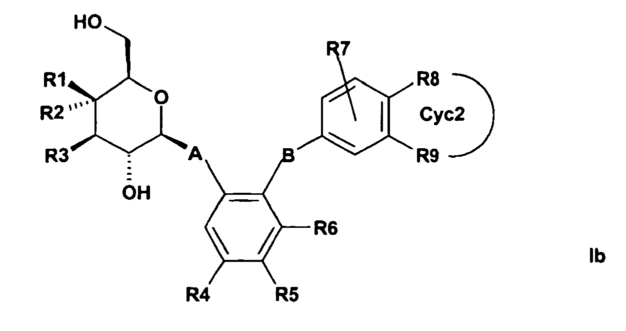 as claimed in claims 1 to 6, in which
R1 is F and R2 is H or
R1 is H and R2 is F or
R1 is F and R2 is F;
R3 is OH;
A is O;
R4 is hydrogen, methyl, methoxy or OH;
R5 is hydrogen, F or methoxy;
R6 is hydrogen or OH;
B is -CH₂-, -CO-NH-CH₂-, -O- or -CO-CH₂-CH₂-;
Cyc1 is phenyl;
R7 is hydrogen;
R8 is hydrogen, OH, ethyl, Cl, OCF₃ or methoxy;
R9 is hydrogen; or
R8 and R9 together are -CH=CH-O- or -CH₂-CH₂-O-

8. A medicament comprising one or more of the compounds as claimed in one or more of claims 1 to 7.

9. A medicament comprising one or more of the compounds as claimed in one or more of claims 1 to 7 and one or more blood glucose-lowering active ingredients.

10. The use of the compounds as claimed in one or more of claims 1 to 7 for producing a medicament for the treatment of type 1 and type 2 diabetes.

11. The use of the compounds as claimed in one or more of claims 1 to 7 for producing a medicament for lowering blood glucose.

12. The use of the compounds as claimed in one or more of claims 1 to 7 in combination with at least one other blood glucose-lowering active ingredient for producing a medicament for the treatment of type 1 and type 2 diabetes.

13. The use of the compounds as claimed in one or more of claims 1 to 7 in combination with at least one other blood glucose-lowering active ingredient for producing a medicament for lowering blood glucose.

14. A process for producing a medicament comprising one or more of the compounds as claimed in one or more of claims 1 to 7, which comprises mixing the active ingredient with a pharmaceutically suitable carrier and converting this mixture into a form suitable for administration.

## Revendications

1. Composé de formule I 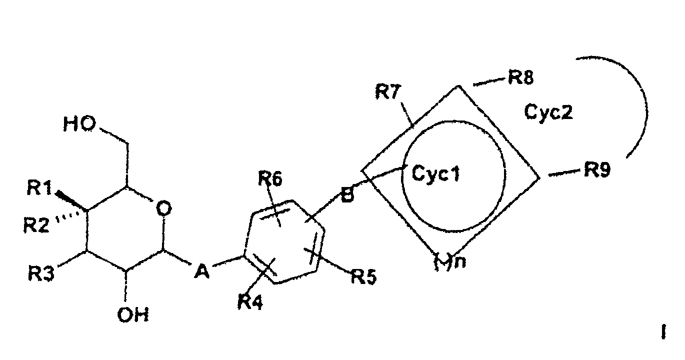 dans laquelle les significations sont
R1, R2 : OH, F ou H ou R1 et R2 = F, à l'exception des trois combinaisons R1 = F, R2 = OH et R1 = OH, R2 = F et R1, R2=OH ;
R3 : OH ou F, où au moins l'un des radicaux R1, R2, R3 doit être F ;
A : O, NH, CH₂, S ou une liaison ;
R4, R5, R6 : un hydrogène, F, Cl, Br, I, OH, NO₂, CN, COOH, CO(alkyle en C₁-C₆), COO(alkyle en C₁-C₆), CONH₂, CONH(alkyle en C₁-C₆), CON[(alkyle en C₁-C₆)]₂, un alkyle en C₁-C₆, un alcényle en C₂-C₆, un alcynyle en C₂-C₆, un alcoxy en C₁-C₆, HO(alkyle en C₁-C₆), (alkyle en C₁-C₆)-O-(alkyle en C₁-C₆), un phényle, un benzyle, un, plusieurs ou tous les hydrogène(s) dans les radicaux alkyle, alcoxy, alcényle et alcynyle pouvant être remplacé(s) par un fluor ;
SO₂-NH₂, SO₂NH(alkyle en C₁-C₆), SO₂N[(alkyle en C₁-C₆)]₂, S-(alkyle en C₁-C₆), S-(CH₂)ₒ-phényle, SO-(alkyle en C₁-C₆), SO-(CH₂)ₒ-phényle, SO₂-(alkyle en C₁-C₆), SO₂-(CH₂)ₒ-phényle, où o peut être de 0 à 6, et le radical phényle peut être substitué jusqu'à deux fois par F, Cl, Br, OH, CF₃, NO₂, CN, OCF₃, O-(alkyle en C₁-C₆), un alkyle en C₁-C₆, NH₂ ;
NH₂, NH-(alkyle en C₁-C₆), N((alkyle en C₁-C₆))₂, NH(acyle en C₁-C₇), un phényle, O-(CH₂)ₒ-phényle, où o peut être de 0 à 6, où le cycle phényle peut être substitué une à trois fois par F, Cl, Br, I, OH, CF₃, NO₂, CN, OCF₃, O-(alkyle en C₁-C₆), un alkyle en C₁-C₆, NH₂, NH(alkyle en C₁-C₆), N((alkyle en C₁-C₆))₂, SO₂₋CH₃, COOH, COO-(alkyle en C₁-C₆), CONH₂;
B : un alcanediyle en C₀-C₁₅, un ou plusieurs atomes de C dans le radical alcanediyle pouvant être remplacés indépendamment les uns des autres par -O-, -(C=O)-, -CH=CH-, -C ≡C-, -S-, -CH(OH)-, -CHF-, -CF₂-, -(S=O)-, -(SO₂)-, -N(alkyle en C₁-C₆)-, -N((alkyle en C₁-C₆)-phényle)- ou -NH- ;
n : un nombre de 0 à 4 ;
Cyc1 : un cycle ayant de 3 à 7 chaînons saturé, partiellement saturé ou insaturé, où 1 atome de C peut être remplacé par O, N ou S ;
R7, R8, R9 : un hydrogène, F, Cl, Br, I, OH, CF₃, NO₂, CN, COOH, COO(alkyle en C₁-C₆), CO(alkyle en C₁-C₄), CONH₂, CONH(alkyle en C₁-C₆), CON[(alkyle en C₁-C₆)]₂, un alkyle en C₁-C₆, un alcényle en C₂-C₆, un alcynyle en C₂-C₆, un alcoxy en C₁-C₈, (alkyle en C₁-C₆)-OH, (alkyle en C₁-C₆)-O-(alkyle en C₁-C₆), un, plusieurs ou tous les hydrogène(s) dans les radicaux alkyle pouvant être remplacé(s) par un fluor ;
SO₂-NH₂, SO₂NH(alkyle en C₁-C₆), SO₂N[(alkyle en C₁-C₆)]₂, S-(alkyle en C₁-C₆), S-(CH₂)ₒ-phényle, SO-(alkyle en C₁-C₆), SO-(CH₂)ₒ-phényle, SO₂-(alkyle en C₁-C₆), SO₂-(CH₂)ₒ-phényle, où o peut être de 0 à 6, et le radical phényle peut être substitué jusqu'à deux fois par F, CI, Br, OH, CF₃, NO₂, CN, OCF₃, O-(alkyle en C₁-C₆), un alkyle en C₁-C₆, NH₂ ;
NH₂, NH-(alkyle en C₁-C₆), N((alkyle en C₁-C₆))₂, NH(acyle en C₁-C₇), un phényle, O-(CH₂)ₒ-phényle, où o peut être de 0 à 6, où le cycle phényle peut être substitué une à trois fois par F, Cl, Br, l, OH, CF₃, NO₂, CN, OCF₃, un alcoxy en C₁-C₈, un alkyle en C₁-C₆, NH₂, NH(alkyle en C₁-C₆), N((alkyle en C₁-C₆))₂, SO₂₋CH₃, COOH, COO-(alkyle en C₁-C₆), CONH₂ ;
ou
R8 et R9 forment avec les atomes de C qui les portent un cycle Cyc2 ayant de 5 à 7 chaînons saturé, partiellement ou complètement insaturé, 1 ou 2 atome(s) de C dans le cycle pouvant également être remplacé(s) par N, O ou S, et Cyc2 peut éventuellement être substitué par un alkyle en C₁-C₆, un alcényle en C₂-C₅, un alcynyle en C₂-C₅, où dans chaque cas un groupe CH₂ peut être remplacé par O, ou substitué par H, F, CI, OH, CF₃, NO₂, CN, COO-(alkyle en C₁-C₄), CONH₂, CONH(alkyle en C₁-C₄), OCF₃ ;
et les sels pharmaceutiquement acceptables de celui-ci.

2. Composé de formule l selon la revendication 1, dans laquelle les significations sont
R1, R2 : OH, F ou H ou R1 et R2 = F, où l'un des radicaux R1 ou R2 doit être F, à l'exception des trois combinaisons R1 = F, R2 = OH et R1 = OH, R2 = F et R1, R2=OH;
R3: OH;
A : O ou NH;
R4, R5, R6 : un hydrogène, F, CI, Br, l, OH, CF₃, NO₂, CN, COOH, CO(alkyle en C₁-C₆), COO(alkyle en C₁-C₆), CONH₂, CONH(alkyle en C₁-C₆), CON[(alkyle en C₁-C₆)]₂, un alkyle en C₁-C₆, un alcényle en C₂-C₆, un alcynyle en C₂-C₆, un alcoxy en C₁-C₆, HO(alkyle en C₁-C₆), (alkyle en C₁-C₆)-O-(alkyle en C₁-C₆), un phényle, un benzyle, SO-(alkyle en C₁-C₆), un, plusieurs ou tous les hydrogène(s) dans les radicaux alkyle, alcoxy, alcényle et alcynyle pouvant être remplacé(s) par un fluor ;
B : un alcanediyle en C₀-C₁₅, un ou plusieurs atome(s) de C dans le radical alcanediyle pouvant être remplacé(s) indépendamment les uns des autres par -O-, -(C=O)-, -CH=CH-, -C≡C-, -S-, -CH(OH)-, -CHF-, -CF₂-, -(S=O)-, -(SO₂)-, -N(alkyle en C₁-C₆)-, N((alkyle en C₁-C₆)-phényle)- ou -NH- ;
n: un nombre de 0 à 4 ;
Cyc1 : un cycle ayant de 3 à 7 chaînons saturé, partiellement saturé ou insaturé, où 1 atome de C peut être remplacé par O, N ou S ;
R7, R8, R9 : un hydrogène, F, Cl, Br, l, OH, CF₃, NO₂, CN, COOH, COO(alkyle en C₁-C₆), CO(alkyle en C₁-C₄), CONH₂, CONH(alkyle en C₁-C₆), CON[(alkyle en C₁-C₆)]₂, un alkyle en C₁-C₆, un alcényle en C₂-C₆, un alcynyle en C₂-C₆, un alcoxy en C₁-C₈, (alkyle en C₁-C₆)-OH, (alkyle en C₁-C₆)-O-(alkyle en C₁-C₆), SO-(alkyle en C₁-C₆), un, plusieurs ou tous les hydrogène(s) dans les radicaux alkyle pouvant être remplacé(s) par un fluor ;
ou
R8 et R9 forment avec les atomes de C qui les portent un cycle Cyc2 ayant de 5 à 7 chaînons saturé,
partiellement ou complètement insaturé, où 1 ou 2 atome(s) de C dans le cycle peuvent également être remplacé(s) par N, O ou S, et Cyc2 peut éventuellement être substitué par un alkyle en C₁-C₆, un alcényle en C₂-C₅, un alcynyle en C₂-C₅, où dans chaque cas un groupe CH₂ peut être remplacé par O, ou substitué par H, F, Cl, OH, CF₃, NO₂, CN, COO(alkyle en C₁-C₄), CONH₂, CONH(alkyle en C₁-C₄), OCF₃.

3. Composé de formule l selon les revendications 1 ou 2, dans lequel les résidus de sucre sont liés en position bêta (β), ou composé dans lequel le substituant B sur le cycle phényle est situé en position ortho par rapport au substituant A.

4. Composé de formule I selon les revendications 1 à 3, dans laquelle
R1, R2 sont OH, F ou H ou R1 et R2 = F, où l'un des radicaux R1 ou R2 doit être F, à l'exception des trois combinaisons R1 = F, R2 = OH et R1 = OH, R2 = F et R1, R2 = OH ;
R3 est OH ;
A est O ;
R4, R5, R6 sont un hydrogène, OH, un alkyle en C₁-C₆, un alcoxy en C₁-C₄, HO-(alkyle en C₁-C₄), (alkyle en C₁-C₄)-O-(alkyle en C₁-C₄), F, Cl, Br, I, CF₃, OCF₃, OCH₂CFg, (alkyle en C₁-C₄)-CF₂-, un phényle, un benzyle, un alcényle en C₂-C₄, un alcynyle en C₂-C₄, COO(alkyle en C₁-C₄) ;
B est un alcanediyle en C₁-C₄, où un groupe CH₂ peut également être remplacé par -(C=O)-, -CH(OH)-, -CO-NH-, -CO-N(alkyle en C₁-C₆)-, -CHF-, -CF₂-, -O-, -NH-;
n est un nombre valant 2 ou 3 ;
Cyc1 est un cycle insaturé ayant de 5 à 6 chaînons, où 1 atome de C peut être remplacé par O, N ou S ;
R7, R8, R9 sont un hydrogène, un alkyle en C₁-C₆, un alcoxy en C₁-C₈, OCF₃, OCH₂CF₃, OH, (alkyle en C₁-C₄)-OH, (alkyle en C₁-C₄)-O-(alkyle en C₁-C₄), F, Cl, Br ou
R8 et R9 forment ensemble -CH=CH-O-, -CH₂-CH₂-O-, -CH=CH-S-, -CH=CH-CH=CH-, -O-(CH₂)ₚ-O-, avec p = 1 ou 2, et
R7 est un méthyle, un éthyle, OMe, F, Cl, Br ou un hydrogène.

5. Composé de formule l selon les revendications 1 à 4, dans laquelle
R1 est F et R2 est H ou
R1 est H et R2 est F ou
R1 est F et R2 est F ;
R3 est OH ;
A est O ;
R4, R5, R6 sont un hydrogène, OH, un alcoxy en C₁-C₄, CF₃, un alkyle en C₁-C₄, F, Cl, Br, I ;
B est -CH₂-, -C₂H₄-, -C₃H₆-, -CH(OH)-, -(C=O)-. -CO-NH-CH₂- ou -CO-CH₂-CH₂-, -O-, -NH- ;
n est un nombre valant 2 ou 3 ;
Cyc1 est un cycle insaturé à 6 chaînons, où 1 atome de C peut être remplacé par N, ou un cycle insaturé à 5 chaînons, où 1 atome de C peut être remplacé par S ;
R7, R8, R9 sont un hydrogène, OH, un alkyle en C₁-C₄, un alcoxy en C₁-C₇, OCF₃, un halogène ou
R8 et R9 forment ensemble -CH=CH-O-, -CH₂-CH₂-O-, -CH=CH-CH=CH-, -O-(CH₂)ₚ-O-, avec p = 1 ou 2, et
R7 est un méthyle, un éthyle, OMe, F, Cl, Br ou un hydrogène.

6. Composé de formule la 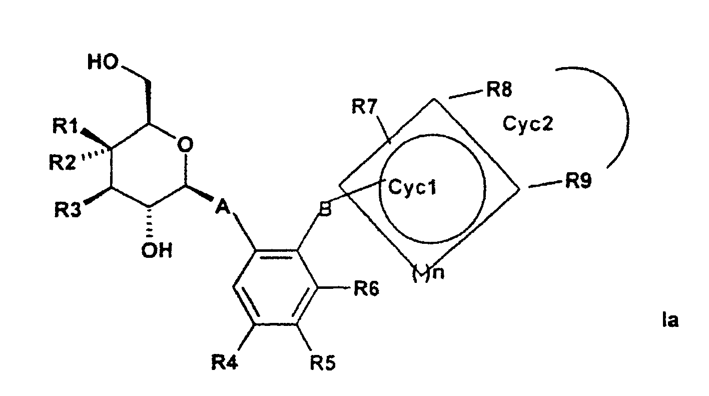 selon les revendications 1 à 5, dans laquelle
R1 est F et R2 est H ou
R1 est H et R2 est F ou
R1 est F et R2 est F ;
R3 est OH ;
A est O ;
R4 est un hydrogène, un alkyle en C₁-C₄, un alcoxy en C₁₋C₄ ou OH ;
R5 est un hydrogène, F, un méthoxy ou un éthoxy ;
R6 est un hydrogène ou OH ;
B est -CH₂-, -CO-NH-CH₂-, -O- ou -CO-CH₂-CH₂-;
Cyc1 est un phényle ou un thiophène ;
R7, R8, R9 sont un hydrogène, OH, Cl, OCF₃, un alkyle en C₁-C₄ ou un alcoxy en C₁-C₄; ou
R8 et R9 forment ensemble -CH=CH-O-, -CH=CH-CH=CH- ou -CH₂-CH₂-O- et
R7 est un hydrogène.

7. Composé de formule Ib 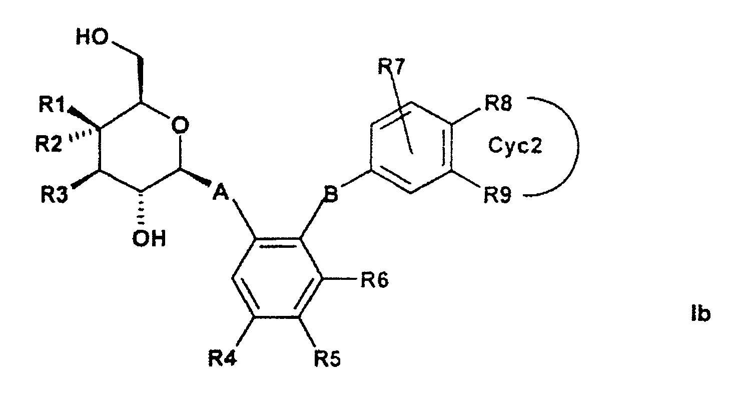 selon les revendications 1 à 6, dans laquelle
R1 est F et R2 est H ou
R1 est H et R2 est F ou
R1 est F et R2 est F;
R3 est OH ;
A est O;
R4 est un hydrogène, un méthyle, un méthoxy ou OH ;
R5 est un hydrogène, F ou un méthoxy ;
R6 est un hydrogène ou OH ;
B est -CH₂-, -CO-NH-CH₂-, -O- ou -CO-CH₂-CH₂- ;
Cyc1 est un phényle ;
R7 est un hydrogène ;
R8 est un hydrogène, OH, un éthyle, CI, OCF₃ ou un méthoxy ;
R9 est un hydrogène; ou
R8 et R9 forment ensemble -CH=CH-O- ou -CH₂-CH₂-O-.

8. Médicament comprenant un ou plusieurs des composés selon l'une ou plusieurs des revendications 1 à 7.

9. Médicament comprenant un ou plusieurs des composés selon l'une ou plusieurs des revendications 1 à 7 et une ou plusieurs substances actives hypoglycémiantes.

10. Utilisation des composés selon l'une ou plusieurs des revendications 1 à 7 pour produire un médicament destiné au traitement des diabètes de type 1 et 2.

11. Utilisation des composés selon l'une ou plusieurs des revendications 1 à 7 pour produire un médicament hypoglycémiant.

12. Utilisation des composés selon l'une ou plusieurs des revendications 1 à 7 en combinaison avec au moins une autre substance active hypoglycémiante pour produire un médicament destiné au traitement des diabètes de type 1 et 2.

13. Utilisation des composés selon l'une ou plusieurs des revendications 1 à 7 en combinaison avec au moins une autre substance active hypoglycémiante pour produire un médicament hypoglycémiant.

14. Procédé de production d'un médicament comprenant un ou plusieurs des composés selon l'une ou plusieurs des revendications 1 à 7, qui consiste à mélanger la substance active avec un support pharmaceutiquement approprié et à convertir ce mélange en une forme appropriée pour être administrée.
